(19) Europäisches Patentamt
European Patent Office
Office européen des brevets

(11) Veröffentlichungsnummer: **0 534 341 A1**

# (12) EUROPÄISCHE PATENTANMELDUNG

(21) Anmeldenummer: **92116108.9**

(22) Anmeldetag: **21.09.92**

(51) Int. Cl.5: **C07D 239/60**, C07D 239/52, C07D 401/12, C07D 239/34, C07D 239/70, C07D 239/90, C07D 405/12, C07D 409/12, A01N 43/54

(30) Priorität: **25.09.91 DE 4131924**

(43) Veröffentlichungstag der Anmeldung: **31.03.93 Patentblatt 93/13**

(84) Benannte Vertragsstaaten: **PT**

(71) Anmelder: **HOECHST AKTIENGESELLSCHAFT**
**Postfach 80 03 20**
**W-6230 Frankfurt am Main 80(DE)**

(72) Erfinder: **Preuss, Rainer, Dr.**
**Am Sonnenhang 7**
**W-6238 Hofheim/Ts(DE)**
Erfinder: **Salbeck, Gerhard, Dr.**
**Königsberger Strasse 52**
**W-6239 Kriftel/Ts(DE)**
Erfinder: **Schaper, Wolfgang, Dr.**
**Kapellenweg 5c**
**W-8901 Diedorf(DE)**
Erfinder: **Braun, Peter, Dr.**
**Pfarrer-Dorn-Strasse 13**
**W-6500 Mainz(DE)**
Erfinder: **Knauf, Werner, Dr.**
**Im Kirschgarten 24**
**W-6239 Eppstein/Ts(DE)**
Erfinder: **Sachse, Burkhard, Dr.**
**An der Ziegelei 30**
**W-6233 Kelkheim/Ts(DE)**
Erfinder: **Waltersdorfer, Anna, Dr.**
**Rauenthaler Weg 28**
**W-6000 Frankfurt/Main 71(DE)**
Erfinder: **Kern, Manfred, Dr.**
**Im Traminerweg 8**
**W-6501 Lörzweiler(DE)**
Erfinder: **Lümmen, Peter, Dr.**
**Rautenweg 1**
**W-6272 Niedernhausen(DE)**

(54) **Substituierte 4-Alkoxypyrimidine, Verfahren zu ihrer Herstellung, diese enthaltende Mittel, und ihre Verwendung als Schädlingsbekämpfungsmittel.**

(57) Die Erfindung betrifft substituierte 4-Alkoxypyrimidine der Formel

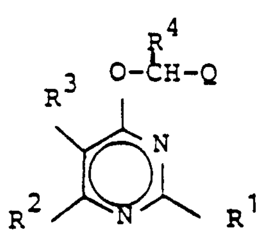

worin $R^1$ Wasserstoff, Halogen, Alkyl oder Cycloalkyl, $R^2$ Wasserstoff, ggf. substituiertes Alkyl, ggf. substituiertes Alkoxy, ggf. substituiertes Alkylthio, Alkylamino oder Dialkylamino, $R^3$ Wasserstoff, Alkyl, Alkoxy, Halogenalkoxy, Halogen, Alkylthio, Amino, Alkylamino oder Dialkylamino und $R^4$ Wasserstoff, Alkyl, Cycloalkyl oder Halogenalkyl bedeuten, wobei $R^2$ und $R^3$ auch zusammen mit den diese tragenden C-Atomen einen Ring bilden können und Q die in der Beschreibung definierten Bedeutungen hat.
Die Erfindung betrifft weiterhin ein Verfahren zu ihrer Herstellung, diese enthaltende Mittel und ihre Verwendung

EP 0 534 341 A1

als Schädlingsbekämpfungsmittel.

Die Erfindung betrifft neue substituierte 4-Alkoxypyrimidine, Verfahren zur ihrer Herstellung, sowie ihre Verwendung als Schädlingsbekämpfungsmittel, insbesondere als Insektizid, Akarizid, Nematozid und Fungizid.

Es ist bereits bekannt, daß bestimmte substituierte 4-Alkoxychinazoline gute fungizide, akarizide und insektizide Wirkung besitzen (vgl. EP 326 328, EP 326 329). Die biologische Wirkung dieser Verbindungen ist jedoch insbesondere bei niedrigen Aufwandmengen und Konzentrationen nicht in allen Anwendungsbereichen zufriedenstellend.

Gegenstand der Erfindung sind substituierte 4-Alkoxypyrimidine der Formel I

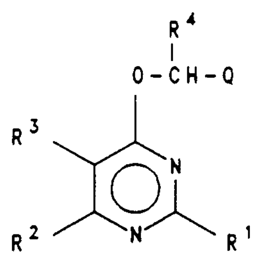

(I)

worin

| | |
|---|---|
| $R^1$ | Wasserstoff, Halogen, $(C_1\text{-}C_4)$Alkyl oder $(C_3\text{-}C_6)$Cycloalkyl bedeutet, |
| $R^2$ | Wasserstoff, $(C_1\text{-}C_4)$Alkyl, Halogen, $(C_1\text{-}C_4)$Halogenalkyl, $(C_1\text{-}C_{10})$Alkoxy, Phenyl-$(C_1\text{-}C_4)$-alkoxy, $(C_1\text{-}C_{10})$Alkoxy-$(C_1\text{-}C_{10})$alkoxy, Benzyloxy-$(C_1\text{-}C_{10})$alkoxy, $(C_1\text{-}C_{10})$Halogenalkoxy, $(C_1\text{-}C_{10})$Alkoxy-$(C_1\text{-}C_{10})$alkyl, $(C_1\text{-}C_{10})$Alkylthio, $(C_1\text{-}C_{10})$Alkylthio-$(C_1\text{-}C_{10})$alkyl, $(C_1\text{-}C_{10})$-Alkylamino oder Di-$(C_1\text{-}C_{10})$alkylamino bedeutet, wobei in den genannten Resten Phenyl gegebenenfalls mit $(C_1\text{-}C_6)$Alkyl, $(C_1\text{-}C_6)$Alkoxy oder Halogen monosubstituiert ist, |
| $R^3$ | Wasserstoff, $(C_1\text{-}C_4)$Alkyl, $(C_1\text{-}C_4)$Alkoxy, $(C_1\text{-}C_4)$Halogenalkoxy, Halogen, $(C_1\text{-}C_4)$-Alkylthio, Amino, $(C_1\text{-}C_4)$Alkylamino, $(C_1\text{-}C_4)$Dialkylamino bedeutet oder |
| $R^2$ und $R^3$ | zusammen mit dem Kohlenstoffatom, an das sie gebunden sind, einen ungesättigten 5-gliedrigen Ring bilden, der ein Sauerstoff oder Schwefelatom enthält und der gegebenenfalls durch Alkyl oder Halogen substituiert ist, oder |
| $R^2$ und $R^3$ | zusammen mit dem Kohlenstoffatom, an das sie gebunden sind, einen gesättigten 5-, 6- oder 7-gliedrigen Ring bilden, der ein Sauerstoff- oder Schwefelatom enthalten kann und der gegebenenfalls durch Alkyl substituiert ist, |
| $R^4$ | Wasserstoff, $(C_1\text{-}C_4)$Alkyl, $(C_3\text{-}C_6)$Cycloalkyl oder $(C_1\text{-}C_4)$-Halogenalkyl, wie $CF_3$, bedeutet, |
| Q | die Bedeutung $Q^1$ hat und |
| $Q^1$ | $(C_1\text{-}C_{15})$Alkyl bedeutet, welches bis zu dreifach ungesättigt sein kann und welches, gegebenenfalls substituiert ist mit einem, zwei oder drei Halogenatomen, einer $(C_3\text{-}C_8)$-Cycloalkylgruppe, einer $(C_1\text{-}C_{15})$Alkoxygruppe, einer $(C_1\text{-}C_{15})$Alkoxy-$(C_1\text{-}C_{15})$-Alkoxygruppe, einer $(C_1\text{-}C_{15})$Alkylthiogruppe, einer $(C_1\text{-}C_{15})$Alkylsulfinylgruppe, einer $(C_1\text{-}C_{15})$Alkylsulfonylgruppe, einer $(C_1\text{-}C_{15})$-Alkanoylgruppe, einer $(C_4\text{-}C_8)$-Cycloalkylalkoxygruppe, einer $(C_4\text{-}C_8)$Cycloalkylalkylthiogruppe, einer Benzyloxy- oder einer Phenoxybenzyloxygruppe, oder |
| Q | die Bedeutung $Q^2$ hat und |
| $Q^2$ | eine Gruppe der allgemeinen Formel IIa-IIj bedeutet |

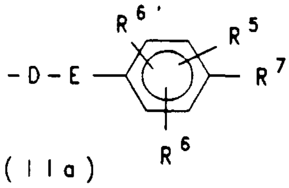

(IIa)

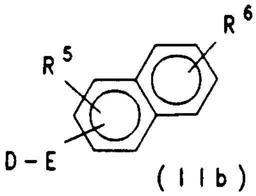

(IIb)

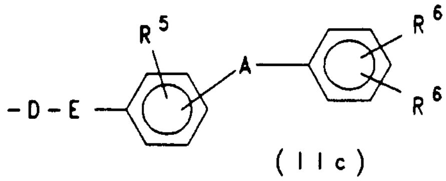

(IIc)

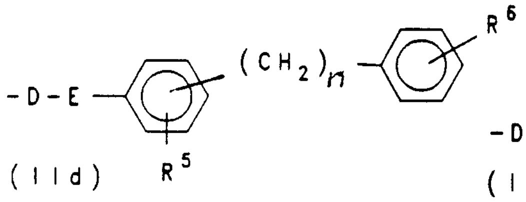

(IId)

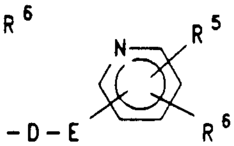

(IIe)

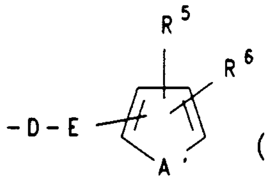

(IIf)

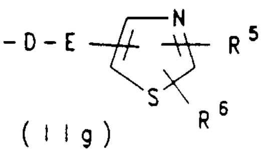

(IIg)

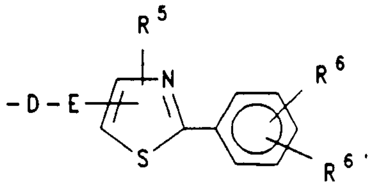

(IIh)

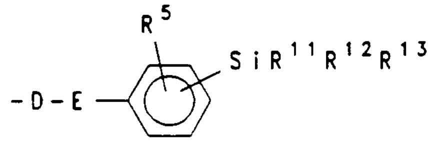

(IIi)

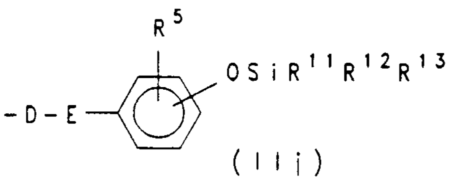

(IIj)

worin

n 0, 1 oder 2 ist,

A Sauerstoff, $-O-CH_2-$, Schwefel, Sulfinyl oder Sulfonyl bedeutet,

A' Sauerstoff oder Schwefel bedeutet,

D eine direkte Bindung oder eine $(C_1-C_6)$Alkylengruppe bedeutet

E eine direkte Bindung, sowie für den Fall, daß D eine Alkylengruppe ist, Sauerstoff oder Imino bedeutet,

$R^5$, $R^6$ und $R^{6'}$ gleich oder verschieden sind und jeweils Wasserstoff, Halogen, $(C_1-C_8)$-Alkyl, $(C_2-C_8)$-Alkenyl, $(C_2-C_8)$-Alkinyl, $(C_3-C_6)$Cycloalkyl, $(C_1-C_8)$-Halogenalkyl, $(C_1-C_8)$Alkoxy, $(C_1-C_8)$Alkylthio, $(C_1-C_8)$Alkylsulfinyl, $(C_1-C_8)$-Alkylsulfonyl, $(C_1-C_4)$Dialkylamino oder Nitro bedeuten, wobei für den Fall, daß $R^5$, $R^6$, $R^{6'}$ Alkylreste bedeuten, diese auch cyclisch verknüpft sein können,

$R^7$ die für $R^5$, $R^6$ und $R^{6'}$ angegebene Bedeutung hat, sowie für den Fall, daß E eine direkte Bindung bedeutet, $(C_1-C_4)$Alkoxy-$(C_1-C_4)$alkyl,
$(C_1-C_4)$Alkoxycarbonyl, $(C_1-C_8)$Alkanoyl oder eine Gruppe der allgemeinen Formel III bedeutet,

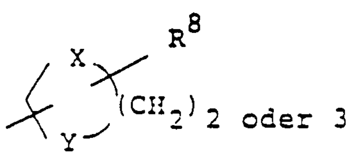

(III),

worin X und Y gleich oder verschieden sind und jeweils Schwefel oder Sauerstoff bedeuten und

$R^8$ Wasserstoff oder $(C_1-C_4)$Alkyl bedeutet, oder

Q die Bedeutung $Q^3$ hat und

$Q^3$ falls von der vorstehenden Formel IId nicht umfaßt, eine Gruppe der allgemeinen Formel IV bedeutet,

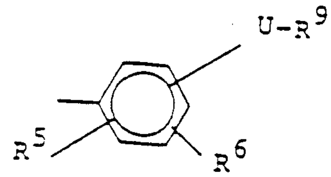

(IV),

worin $R^5$ und $R^6$ die oben angegebenen Bedeutungen haben,

U eine direkte Bindung, Sauerstoff, Schwefel, Sulfinyl, Sulfonyl oder Methylen bedeutet,

$R^9$ Phenyl oder einen Heterocyclen-Rest bedeutet, wobei jeder der beiden vorgenannten Reste unsubstituiert oder mit einem oder zwei Substituenten versehen sein kann und diese Substituenten gleich oder verschieden sind und jeweils Halogen, $(C_1-C_4)$Alkyl, Trifluormethyl, Nitro, $(C_1-C_4)$Alkoxy, $(C_1-C_4)$Alkylthio, $(C_1-C_4)$-Alkylsulfinyl, $(C_1-C_4)$-Alkylsulfonyl, $(C_1-C_4)$Alkoxy-$(C_1-C_4)$alkyl, Phenyl, Phenoxy, $(C_1-C_4)$Alkoxyphenoxy, Halogenphenoxy oder $(C_1-C_4)$Alkylphenoxy bedeuten,

$R^9$ weiterhin, für den Fall, daß U Sauerstoff bedeutet, $(C_1-C_4)$Halogenalkyl oder eine Gruppe der allgemeinen Formel V bedeutet

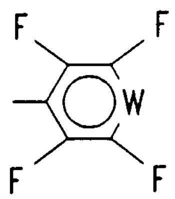

(V),

worin

W Stickstoff oder eine Gruppe $CR^{10}$ bedeutet, worin $R^{10}$ Wasserstoff, Fluor, Cyano, Formyl, Acetyl, Nitro, Methyl, Methoxy oder 1,3-Dioxolan-2-yl bedeutet,

$R^{11}$, $R^{12}$ und $R^{13}$ gleich oder verschieden sind und $(C_1-C_{15})$Alkyl, das gegebenenfalls ein- oder mehrfach halogensubstituiert ist,

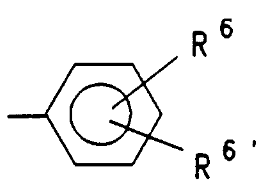

oder

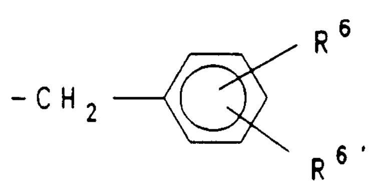

bedeutet, worin $R^6$ und $R^{6'}$ wie oben definiert sind.

Bevorzugt sind solche Verbindungen der Formel I, worin

$R^1$ Wasserstoff, Chlor oder Methyl bedeutet,
$R^2$ $(C_1-C_4)$Alkyl, Chlor, Trifluormethyl, Methoxy, Ethoxy oder Methoxymethyl bedeutet,
$R^3$ Wasserstoff, $(C_1-C_3)$Alkyl, Methoxy, Ethoxy oder Halogen bedeutet oder
$R^2$ und $R^3$ zusammen mit dem Kohlenstoffatom, an das sie gebunden sind, einen ungesättigten 5-gliedrigen Ring bilden, der ein Sauerstoff- oder Schwefelatom enthält oder $R^2$ und $R^3$ zusammen mit dem Kohlenstoffatom, an das sie gebunden sind, einen gesättigten 5- oder 6-gliedrigen Ring bilden, der ein Schwefelatom enthalten kann,
$R^4$ Wasserstoff, Methyl, Ethyl, Trifluormethyl oder Cyclopropyl bedeutet,
Q die Bedeutung $Q^1$, $Q^2$ oder $Q^3$ hat.

Stärker bevorzugt sind solche Verbindungen der Formel I, worin

$R^1$ Wasserstoff oder Methyl bedeutet,
$R^2$ Methyl, Ethyl, Trifluormethyl, Methoxy, Ethoxy oder Methoxymethyl bedeutet,
$R^3$ Methyl, Ethyl, Methoxy, Chlor oder Brom bedeutet oder
$R^2$ und $R^3$ zusammen mit dem Kohlenstoffatom, an das sie gebunden sind einen gesättigten 5- oder 6-gliedrigen Ring bilden,
$R^4$ Wasserstoff, Methyl, Ethyl, Trifluormethyl oder Cyclopropyl bedeutet,
Q die Bedeutung $Q^1$, $Q^2$ oder $Q^3$ hat.

Noch stärker bevorzugt sind solche Verbindungen der Formel I, worin

$R^1$ Wasserstoff bedeutet,
$R^2$ Methyl, Ethyl oder Methoxymethyl bedeutet,
$R^3$ Chlor, Brom oder Methoxy bedeutet oder
$R^2$ und $R^3$ zusammen mit dem Kohlenstoffatom, an das sie gebunden sind, einen gesättigten 6-gliedrigen Ring bilden,
$R^4$ Wasserstoff, Methyl oder Ethyl bedeutet,
Q die Bedeutung $Q^1$, $Q^2$ oder $Q^3$ hat.

Ganz besonders bevorzugt sind solche Verbindungen der Formel I, worin

$R^1$ Wasserstoff bedeutet,
$R^2$ Methoxymethyl und $R^3$ Methoxy oder
$R^2$ Methyl oder Ethyl und $R^3$ Chlor oder Brom bedeutet, oder
$R^2$ und $R^3$ zusammen mit dem Kohlenstoffatom, an das sie gebunden sind, einen gesättigten 6-gliedrigen Ring bilden,
$R^4$ Wasserstoff, Methyl oder Ethyl bedeutet,
Q die Bedeutung $Q^1$, $Q^2$ oder $Q^3$ hat.

Am stärksten bevorzugt sind Verbindungen der Formel I, worin

$R^1$ Wasserstoff bedeutet,
$R^2$ Methoxymethyl und $R^3$ Methoxy oder
$R^2$ Ethyl und $R^3$ Chlor bedeutet, oder
$R^2$ und $R^3$ zusammen mit dem Kohlenstoffatom, an das sie gebunden sind, einen gesättigten 6-gliedrigen Ring bilden,
$R^4$ Wasserstoff oder Methyl bedeutet,
$Q^1$ eine $(C_4-C_{10})$Alkylgruppe oder eine $(C_1-C_3)$Alkylgruppe, die mit einer Cyclohexylgruppe $(C_4-C_8)$Cycloalkylalkoxygruppe oder einer $(C_4-C_8)$Cycloalkylalkylthiogruppe substituiert ist, bedeutet.

Am stärksten bevorzugt sind weiterhin Verbindungen der Formel I, worin

$R^1$ Wasserstoff bedeutet,
$R^2$ Methoxymethyl und $R^3$ Methoxy oder
$R^2$ Ethyl und $R^3$ Chlor bedeutet, oder
$R^2$ und $R^3$ zusammen mit dem Kohlenstoffatom, an das sie gebunden sind, einen gesättigten 6-gliedrigen Ring bilden,
$R^4$ Wasserstoff oder Methyl bedeutet,
$Q^2$ eine Gruppe der allgemeinen Formeln IIa-IIj, vorzugsweise IIa, IIb, IIc, IId, IIf, IIi, IIj, insbesondere IIa, IIi, IIj bedeutet, worin D eine Methylengruppe und E eine direkte Bindung bedeutet, $R^5$, $R^6$ und $R^{6'}$ Wasserstoff, $(C_1-C_8)$Alkyl,$(C_1-C_4)$Alkoxy, Cyclohexyl oder Trifluormethyl bedeuten,
$R^7$ Methoxy- oder Ethoxyethyl bedeutet.

Am stärksten bevorzugt sind weiterhin Verbindungen der Formel I, worin

$R^1$ Wasserstoff bedeutet,
$R^2$ Methoxymethyl und $R^3$ Methoxy oder
$R^2$ Ethyl und $R^3$ Chlor bedeutet oder
$R^2$ und $R^3$ zusammen mit dem Kohlenstoffatom, an das sie gebunden sind, einen gesättigten 6-gliedrigen Ring bilden,
$R^4$ Methyl, Ethyl oder Cyclopropyl bedeutet,
Q die Bedeutung $Q^3$ hat,
$R^5$ und $R^6$ Wasserstoff bedeuten,
U Sauerstoff bedeutet,
$R^9$ Phenyl oder einen Heterocyclus bedeutet, wobei jeder der beiden vorgenannten Reste unsubstituiert oder mit einem oder zwei Substituenten versehen sein kann und diese Substituenten gleich oder verschieden sind und jeweils Halogen, $(C_1-C_4)$Alkyl, Trifluormethyl, Nitro, $(C_1-C_4)$Alkoxy, $(C_1-C_4)$Alkylthio oder $(C_1-C_4)$Alkoxy-$(C_1-C_4)$alkyl bedeuten.

In der obigen Formel I ist unter "Halogen" ein Fluor-, Chlor-, Brom- oder Jodatom vorzugsweise ein Chlor- oder Bromatom zu verstehen,
unter dem Ausdruck "$(C_1-C_4)$Alkyl" ein unverzweigter oder verzweigter Kohlenwasserstoffrest mit 1-4 Kohlenstoffatomen, wie z.B. der Methyl-, Ethyl-, Propyl-, 1-Methylethyl-, 2-Methylpropyl- oder 1,1-Dimethylethylrest,
unter dem Ausdruck $(C_1-C_8)$Alkyl die vorgenannten Alkylreste sowie z.B. der Pentyl-, 2-Methylbutyl- oder der 2,2-Dimethylpropylrest, der Hexyl-, Heptyl- oder Octylrest.
Unter dem Ausdruck "$(C_1-C_{15})$Alkyl" ist zu verstehen ein verzweigter oder unverzweigter Kohlenwasserstoffrest mit 1-15 Kohlenstoffatomen wie z.B. die vorstehend unter "$(C_1-C_4)$Alkyl" genannten Reste oder der Pentyl-, Hexyl-, Heptyl-, Octyl-, 1-Nonyl-, 2-Nonyl-, 1-Decyl-, 2-Decyl-, 1-Undecyl-, 2-Undecyl-, Dodecyl-, Tridecyl-, der Tetradecyl- oder Pentadecylrest,
unter dem Ausdruck "$(C_3-C_6)$Cycloalkyl" die Cyclopropyl-, Cyclobutyl-, Cyclopentyl- oder Cyclohexylgruppe,
unter dem Ausdruck "$(C_1-C_4)$Alkoxy" eine Alkoxygruppe, deren Kohlenwasserstoffrest die unter dem Ausdruck "$(C_1-C_4)$Alkyl" angegebene Bedeutung hat,
unter dem Ausdruck "$(C_1-C_{15})$Alkoxy" eine Alkoxygruppe, deren Alkylgruppen die unter "$(C_1-C_{15})$Alkyl" vorstehend genannte Bedeutung haben,
unter dem Ausdruck "$(C_1-C_4)$Alkylthio" eine Alkylthiogruppe, deren Kohlenwasserstoffrest die unter dem Ausdruck "$(C_1-C_4)$-Alkyl" angebenene Bedeutung hat,
unter dem Ausdruck "$(C_1-C_{15})$Alkylthio" eine Alkylthiogruppe, deren Kohlenwasserstoffrest die unter dem Ausdruck "$(C_1-C_{15})$Alkyl" angegebene Bedeutung hat,
unter dem Ausdruck "$(C_1-C_4)$Alkoxycarbonyl" eine Carbonsäureestergruppe, deren Alkoxyteil die unter dem Ausdruck "$(C_1-C_4)$Alkoxy" angegebene Bedeutung hat,
unter dem Ausdruck "$(C_1-C_4)$Halogenalkyl" eine unter dem Ausdruck "$(C_1-C_4)$Alkyl" genannte Alkylgruppe, in der eines oder mehrere Wasserstoffatome durch die obengenannten Halogenatome, bevorzugt Chlor oder Fluor, ersetzt sind, wie beispielsweise die Trifluormethylgruppe, die 2,2,2-Trifluorethylgruppe, die Chlormethyl- oder Fluormethylgruppe,
unter dem Ausdruck "$(C_1-C_4)$Halogenalkoxy" eine Halogenalkoxygruppe deren Halogen-Kohlenwasserstoffrest die unter dem Ausdruck "$(C_1-C_4)$Halogenalkyl" angegebene Bedeutung hat,
unter dem Ausdruck "$(C_1-C_4)$Alkoxy-$(C_1-C_4)$alkyl" beispielsweise eine 1-Methoxyethylgruppe, eine 2-Methoxyethylgruppe, eine 2-Ethoxyethylgruppe, eine Methoxymethyl- oder Ethoxymethylgruppe, eine 3-Methoxypropylgruppe oder eine 4-Butoxybutylgruppe,
unter dem Ausdruck "$(C_1-C_4)$Alkylthio-$(C_1-C_4)$alkyl" beispielsweise Methylthiomethyl, Ethylthiomethyl, Propylthiomethyl, 2-Methylthioethyl, 2-Ethylthioethyl oder 3-Methylthiopropyl,
unter dem Ausdruck "$(C_1-C_4)$Alkylamino" eine Alkylaminogruppe, deren Kohlenwasserstoffrest die unter dem Ausdruck "$(C_1-C_4)$Alkyl" angegebene Bedeutung hat, bevorzugt die Ethyl- und Methylaminogruppe,
unter dem Ausdruck "$(C_1-C_4)$Dialkylamino" eine Dialkylaminogruppe, deren Kohlenwasserstoffreste die unter dem Ausdruck "$(C_1-C_4)$Alkyl" angegebene Bedeutung hat, bevorzugt die Dimethyl- und Diethylaminogruppe,
unter dem Ausdruck "$(C_1-C_{15})$Alkylsulfinyl" eine Alkylsulfinylgruppe, deren Kohlenwasserstoffrest die unter dem Ausdruck "$(C_1-C_{15})$Alkyl" angegebene Bedeutung hat,
unter dem Ausdruck "$(C_1-C_{15})$Alkylsulfonyl" eine Alkylsulfonylgruppe, deren Kohlenwasserstoffrest die unter dem Ausdruck "$(C_1-C_{15})$Alkyl" angegebene Bedeutung hat,
unter dem Ausdruck "$(C_4-C_8)$Cycloalkylalkoxy" z.B. die Cyclopropylmethoxygruppe, die Cyclopropylethoxygruppe, die Cyclobutylmethoxygruppe, die Cyclopentylmethoxygruppe, die Cyclohexylmethoxygruppe oder

die Cyclohexylethoxygruppe,
unter dem Ausdruck "($C_4$-$C_8$)Cycloalkylalkylthio" z.B. die Cyclopropylmethylthiogruppe, die Cyclopropylethylthiogruppe, die Cyclobutylmethylthiogruppe, die Cyclopentylmethylthiogruppe, die Cyclohexylmethylthiogruppe oder die Cyclohexylethylthiogruppe,
unter dem Ausdruck "($C_1$-$C_6$)Alkylen" eine geradkettige oder verzweigte Alkylenkette mit 1-6 Kohlenstoffatomen wie z.B. die Methylengruppe, die Ethylengruppe, die Trimethylengruppe, die Tetramethylengruppe, $-CH(CH_3)-$,

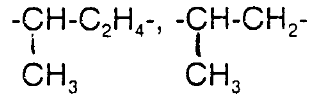

oder

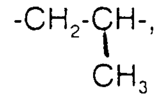

unter dem Ausdruck "($C_1$-$C_8$)Alkanoyl" eine geradkettige oder verzweigte Alkanoylgruppe mit 1-8 Kohlenstoffatomen, wie z.B. die Formylgruppe, die Acetylgruppe, die Propionylgruppe, die 2-Methylpropionylgruppe, die Butyrylgruppe, die Pivaloylgruppe, die Pentanoyl-, Hexanoyl-, Heptanoyl- ode Octanoylgruppe.

Ein "Heterocyclen-Rest" ist vorzugsweise ein Heteroaryl-Rest mit bis zu 10 C-Atomen oder ein davon abgeleiteter teilweise oder vorläufig gesättigter Rest. Unter einem "Heteroaryl-Rest, mit bis zu 10 C-Atomen versteht man vorzugsweise einen mono-, bi- oder tribicyclischen ($C_6$-$C_{14}$)Arylrest, wie Phenyl, Naphthyl oder Anthryl, in welchem mindestens ein CH durch N ersetzt ist und/oder worin mindestens zwei benachbarte CH-Gruppen durch NH, O und/oder S ersetzt sind. Beispiele solcher Rest sind Thienyl, Benzothienyl, Furyl, Benzofuryl, Pyrrolyl, Imidazolyl, Pyryzolyl, Pyridyl, Pyrazinyl, Pyrimidinyl, Pyridazinyl, Indolyl, Oxazolyl, Isoxazolyl, Thiazolyl, Isothiazoyl, Benzoxazolyl, Benzothiazolyl, Indalizonyl, Isoindolyl, Indazolyl, Chinolizinyl, Chinolyl, Isochinolyl, Phthalazonyl, Chinoxalonyl, Chinazolinyl, Cinnolinyl, Carbozolyl, Acridinyl, Phenazinyl, Phenoxazinyl, Phenoxazinyl und Tetrazolyl.

Entsprechendes gilt für hier nicht im einzelnen aufgeführte Gruppen, die in ihrer C-Zahl von den obengenannten Gruppen abweichen bzw. von diesen abgeleitet werden.

Die oben gegebene Erläuterung gilt entsprechend für Homologe.

Die vorliegende Erfindung betrifft die Verbindungen der Formel I in Form der freien Base oder eines Säureadditionssalzes. Säuren, die zur Salzbildung herangezogen werden können, sind anorganische Säuren, wie Salzsäure, Bromwasserstoffsäure, Salpetersäure, Schwefelsäure, Phosphorsäure oder organische Säuren wie Ameisensäure, Essigsäure, Propionsäure, Malonsäure,Oxalsäure, Fumarsäure, Adipinsäure, Stearinsäure, Ölsäure, Methansulfonsäure, Benzolsulfonsäure oder Toluolsulfonsäure.

Die Verbindungen der Formel I weisen zum Teil ein oder mehrere asymmetrische Kohlenstoffatome auf. Es können daher Racemate und Diastereomere auftreten. Die Erfindung umfaßt sowohl die reinen Isomeren als auch deren Gemische. Die Gemische von Diastereomeren können nach gebräuchlichen Methoden, z.B. durch selektive Kristallisation aus geeigneten Lösungsmitteln oder durch Chromatographie in die Komponenten aufgetrennt werden. Racemate können nach üblichen Methoden in die Enantiomeren aufgetrennt werden, so z.B. durch Salzbildung mit einer optisch aktiven Säure, Trennung der diasteromeren Salze und Freisetzung der reinen Enantiomeren mittels einer Base.

Gegenstand der Erfindung ist weiterhin ein Verfahren zur Herstellung von Verbindungen der Formel I, dadurch gekennzeichnet, daß man eine Verbindung der Formel VI

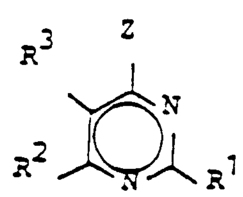

(VI),

worin $R^1$, $R^2$ und $R^3$ die unter Formel I angegebenen Bedeutungen haben und Z eine Abgangsgruppe, beispielsweise Halogen, Alkylthio, Alkansulfonyloxy oder Arylsulfonyloxy, Alkylsulfonyl oder Arylsulfonyl, bedeutet, mit einem Alkohol der Formel VII,

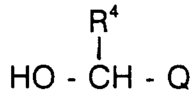

(VII),

worin $R^4$ und Q die unter Formel I angegebenen Bedeutungen haben, umsetzt und die so erhaltenen Verbindungen der Formel I gegebenenfalls am Schwefel einer Thioether-Seitenkette oxidiert oder am $C_5$-Atom des Pyrimidins chloriert oder bromiert, und die so erhaltenen Verbindungen gegebenenfalls in ihre Salze überführt.

Die oben beschriebene Substitutionsreaktion ist im Prinzip bekannt. Die Abgangsgruppe Z ist in weiteren Grenzen variierbar und kann beispielsweise ein Halogenatom wie Fluor, Chlor, Brom oder Jod bedeuten oder Alkylthio wie Methyl- oder Ethylthio, oder Alkansulfonylxoy wie Methan-, Trifluormethan- oder Ethansulfonyloxy oder Arylsulfonyloxy, wie Benzolsulfonyloxy oder Toluolsulfonyloxy oder Alkylsulfonyl wie Methyl- oder Ethylsulfonyl oder Arylsulfonyl wie Phenyl- oder Toluolsulfonyl.

Die vorgenannte Reaktion wird in einem Temperaturbereich von 20-150 °C, zweckmäßig in Anwesenheit einer Base und gegebenenfalls in einem inerten organischem Lösungsmittel wie N,N-Dimethylformamid, N,N-Dimethylacetamid, Dimethylsulfoxid, N-Methylpyrrolidin-2-on, Dioxan, Tetrahydrofuran, 4-Methyl-2-pentanon, Methanol, Ethanol, Butanol, Ethylenglykol, Ethylenglykoldimethylether, Toluol, Chlorbenzol oder Xylol durchgeführt. Es können auch Gemische der genannten Lösungsmittel verwendet werden.

Geeignete Basen sind beispielsweise Alkali- oder Erdalkalimetallcarbonate,-hydrogencarbonate, -amid oder -hydride wie Natriumcarbonat, Natriumhydrogencarbonat, Kaliumcarbonat, Natriumamid oder Natriumhydrid, lithiumorganische Verbindungen, wie n-Butyllithium.

Gegenstand der Erfindung ist weiterhin ein Verfahren zur Herstellung von Verbindungen der Formel I',

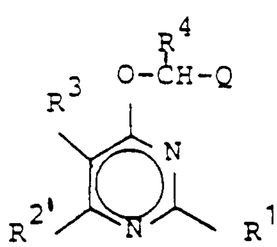

(I'),

worin $R^1$, $R^3$, $R^4$ und Q die unter Formel I angegebenen Bedeutungen haben und $R^{2'}$ $(C_1-C_{10})$Alkoxy, $(C_1-C_{10})$Halogenalkoxy, $(C_1-C_{10})$Alkylthio, $(C_1-C_{10})$Alkylamino oder $(C_1-C_{10})$Dralkylamino bedeutet, dadurch gekennzeichnet, daß man eine Verbindung der Formel VIII

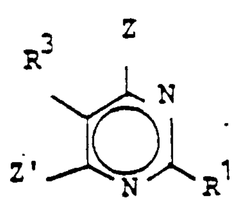

(VIII),

worin Z und Z' gleich oder verschieden sein können und eine Abgangsgruppe, beispielsweise Halogen, Alkylthio, Alkansulfonyloxy, Arylsulfonyloxy, Alkylsulfonyl, oder Arylsulfonyl bedeuten, und $R^1$ und $R^3$ die unter Formel I' angegebenen Bedeutungen haben, mit einer Verbindung der Formel VII

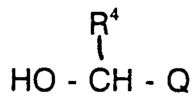 (VII),

worin $R^4$ und Q die unter Formel I' angegebene Bedeutung haben, zu einer Verbindung der Formel IX

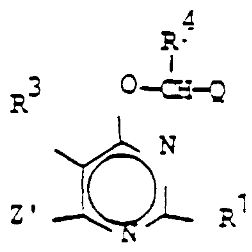 (IX),

worin $R^1$, $R^3$, $R^4$, Q und Z die oben angegebenen Bedeutungen haben, umsetzt und in einem zweiten Reaktionsschritt die Verbindung der Formel VIII mit einer Verbindung der Formel X umsetzt

$HR^{2'}$ (X),

worin $R^{2'}$ die oben angegebene Bedeutung hat, und die so erhaltene Verbindung der Formel I' gegebenenfalls am Schwefel einer Thioether-Seitenkette oxidiert oder gegebenenfalls, falls $R^3$ Wasserstoff bedeutet, chloriert oder bromiert, und die so erhaltenen Verbindungen gegebenenfalls in ihre Salze überführt.

Zur Herstellung der Verbindungen der Formel I' wird dabei völlig analog der oben beschriebenen Herstellung der Verbindungen der Formel I durch Umsetzung der Verbindungen VI mit den Verbindungen VII verfahren.

Die im zweiten Reaktionsschritt benötigte Abgangsgruppe Z' hat die gleiche Bedeutung wie die oben beschriebene Abgangsgruppe Z. Was die Reaktionsbedingungen betrifft, so können im zweiten Reaktionsschritt die gleichen Lösungsmittel, Hilfsbasen und Reaktionstemperaturen zur Anwendung kommen wie bei der oben beschriebenen Herstellung der Verbindungen der Formel I aus den Verbindungen der Formeln VI und VII. Beide Reaktionsschritte können ohne Aufarbeitung nach der ersten Reaktionsstufe als Eintopfreaktion durchgeführt werden.

Gegenstand der Erfindung ist weiterhin ein Verfahren zur Herstellung von Verbindungen der Formel I', dadurch gekennzeichnet, daß man eine Verbindung der obengenannten Formel VIII mit einer Verbindung der Formel X zu einer Verbindung der Formel XI,

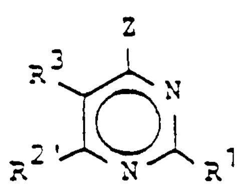 (XI),

worin $R^1$ und $R^3$ die unter Formel I' angegebenen Bedeutungen haben,
$R^{2'}$ $(C_1$-$C_4)$Alkoxy, $(C_1$-$C_4)$Halogenalkoxy, $(C_1$-$C_4)$Alkylthio, $(C_1$-$C_4)$Alkylamino oder $(C_1$-$C_4)$Dialkylamino bedeutet und Z die unter Formel VI angebenene Bedeutung hat, umsetzt, die Verbindung der Formel XI mit einem Alkohol der Formel VII umsetzt und die so erhaltene Verbindung der Formel I' gegebenenfalls am Schwefel einer Thioether-Seitenkette oxidiert, oder, falls $R^3$ Wasserstoff bedeutet, chloriert oder bromiert.

Was die Reaktionsbedingungen betrifft, so können für beide Reaktionsschritte die gleichen Lösungsmittel, Hilfsbasen und Reaktionstemperaturen zur Anwendung kommen wie bei der oben beschriebenen Herstellung der Verbindungen der Formel I aus den Verbindungen der Formel VI und VII.

Beide Reaktionsschritte können auch ohne Aufarbeitung nach der ersten Reaktionsstufe als Eintopfreaktion durchgeführt werden.

Die Ausgangsverbindungen der Formel VI können in Analogie zu bekannten Verfahren hergestellt werden. Als Ausgangsprodukte dienen Acetessigester-Derivate, die über die entsprechenden Hydroxypyrimidine in die Halogenpyrimidine überführt werden:

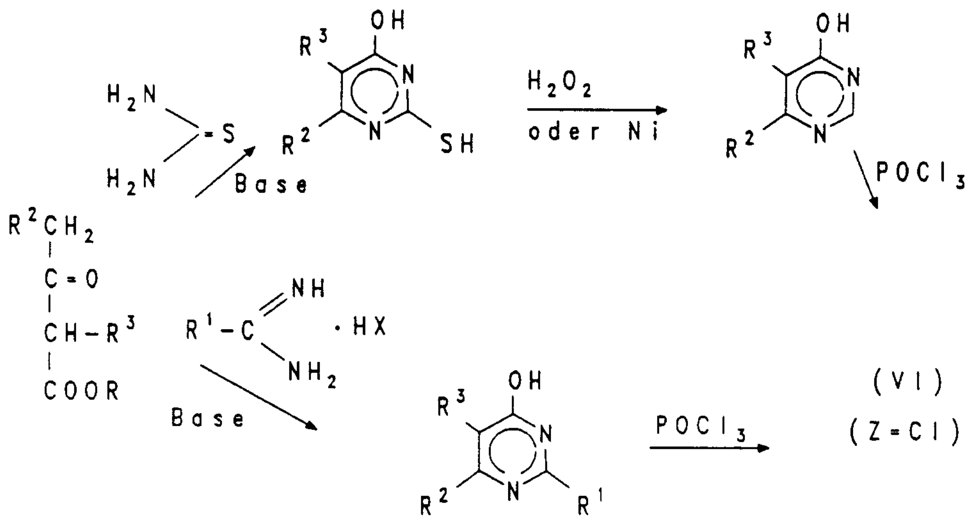


Die Ausgangsverbindungen der Formel VII können in Analogie zu bekannten Verfahren aus Malonester-Derivaten erhalten werden:

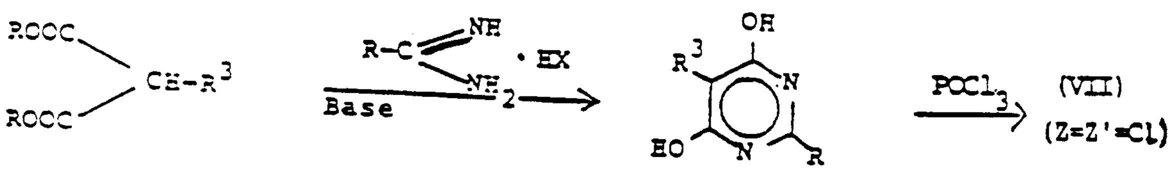


Die als Ausgangsprodukte benötigten Alkohole der Formel VII können nach bekannten Verfahren hergestellt werden.

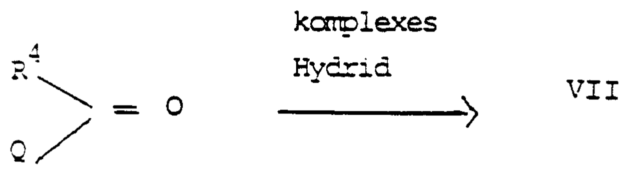


$R^4$ = H, Alkyl, OAlkyl, OH

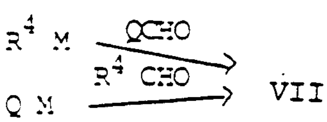


M = MgHal, Li

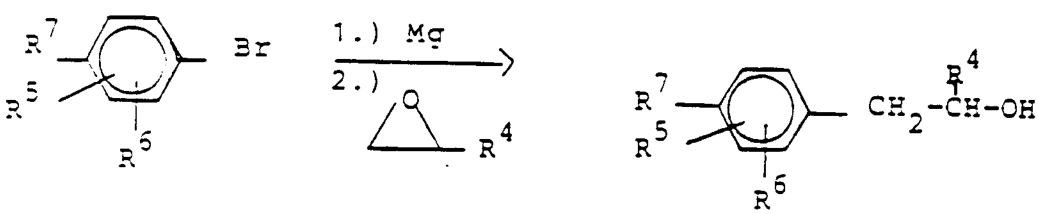

Die Verbindungen der Formel V, für die $R^3$ Halogen bedeutet, können nach bekannten Verfahren halogeniert werden.

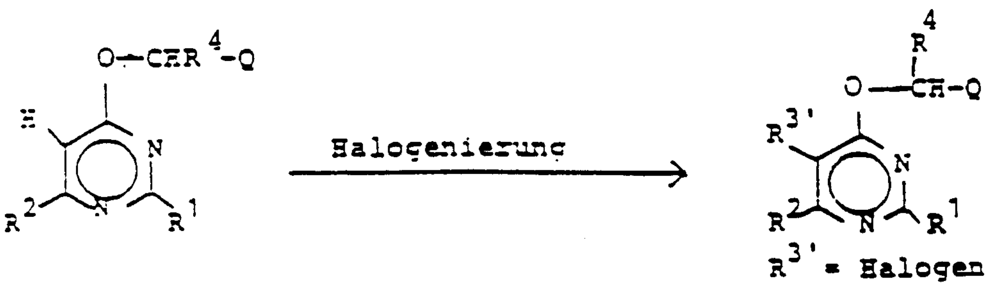


Im Falle der 5-Chlorderivate können z.B. elementares Chlor, Natriumhypochlorit, Sulfurylchlorid oder N-Chlorsuccinimid zum Einsatz kommen, zur Bromierung eignen sich besonders elementares Brom oder N-Bromsuccinimid. Geeignete Lösungsmittel sind z.B. Dichlormethan, Chloroform oder Eisessig.

Die Wirkstoffe eignen sich bei guter Pflanzenverträglichkeit zur Bekämpfung von tierischen Schädlingen, insbesondere Insekten, Spinnentieren, Helminthen und Mollusken, ganz besonders bevorzugt zur Bekämpfung von Insekten und Spinnentieren, die in der Landwirtschaft, bei der Tierzucht, in Forsten, im Vorrats- und Materialschutz sowie auf dem Hygienesektor vorkommen.

Hervorzuheben ist die Eigenschaft der Wirkstoffe von der Pflanze über Stengel und Blätter aufgenommen zu werden und durch basipetalen Transport bis in die Wurzeln transportiert zu werden und damit eine effektive Kontrolle von Nematoden zu ermöglichen.

Die Wirkstoffe sind gegen normal sensible und resistente Arten sowie alle oder einzelne Entwicklungsstadien wirksam. Zu den oben erwähnten Schädlingen gehören:
Aus der Ordnung der Acarina z.B. Acarus siro, Agras spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp., Eotetranychus spp., Oligonychus spp., Eutetranychus spp. Aus der Ordnung der Isopoda, z.B. Oniscus asselus, Armadium vulgare, Porcellio scaber.
Aus der Ordnung der Diplopoda z.B. Blaniulus guttulatus.
Aus der Ordnung der Chilopoda z.B. Geophilus carpophagus, Scutigera spp.
Aus der Ordnung der Symphyla z.B. Scutigerella immaculata.
Aus der Ordung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blatella germanica, Acheta domesticus, Gryllotalpa spp., Locusta migratoria migratorioides, Melanoplus differentialis, Schistocerca gregarai.

Aus der Ordnung des Isoptera z.B. Reticulitermes spp.

Aus der Ordnung der Anoplura z.B. Phylloera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp.,
Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum,, Aphis gossypii, Brevicornyne brassicae, Cryptomyzus ribis, Doralis fabae, Dorolis pomi, Eriosoma lanigerum, Hyalopterus arundinis, Macrosiphium avenae, Myzus spp., Phorodon humuli, Rhopalosiphum padi, Empoasca spp., Euscelus bilobatus, Nephotettix cinoticeps, Lecanium corni, Saissetia oleae, Laodelphax striatellus, Nilaparvata lugens, Aonidiella aurantil, Aspidiotus hederae, Pseudococcus spp., Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tertrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylloides chrysocephala, Epilachna varivestis, Atomaria spp., Oryzaephilus surinamensis, Anthonumus spp., Sitophilus spp., Otiorrhynchus sulcatus, Cosmopolites sordidus, Ceuthorrhynchus assimilis, Hypera postica, Dermestes spp., Trogoderma, Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica. Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliophora erythrocephala, Lucilia spp., Chrysomyia spp., Cuterebra spp., Gastrophilus spp., Hypobosca spp., Stomoxys spp., Oestrus spp. Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopsis, Ceratophyllus spp.

Aus der Ordnung der Arachnida z.B. Scorpio maurus, Latrodectus mactans.

Aus der Klasse der Helminthen z.B. Haemonchus, Trichostrongulus, Ostertagia. Cooperia, Chabertia, Strongyloides, Oesophagostomum, Hyostrongulus, Ancylostoma, Ascaris und Heterakis sowie Fasciola und pflanzenschädigende Nematoden z.B. solche der Gattungen Meloidogyne, Heterodera, Ditylenchus, Aphelenchoides, Radopholus, Globodera, Pratylenchus, Longidorus und Xiphinema.

Aus der Klasse der Gastropoda z.B. Deroceras spp., Arion spp., Lymnaea spp., Galba spp., Succinea spp., Biophalaria spp., Bulinus spp., Oncomelania spp.

Aus der Klasse der Bivalva z.B. Dreissena spp.

Gegenstand der Erfindung sich auch insektizide und akarizide Mittel, die die Verbindungen der Formel I neben geeigneten Formulierungshilfsmitteln enthalten.

Die erfindungsgemäßen Mittel enthalten die Wirkstoffe der Formeln I im allgemeinen zu 1 bis 95 Gew.-%.

Sie können auf verschiedene Art formuliert werden, je nachdem wie es durch die biologischen und/oder chemischphysikalischen Parameter vorgegeben ist. Als Formulierungsmöglichkeiten kommen daher infrage: Spritzpulver (WP), emulgierbare Konzentrate (EC), wäßrige Lösungen (SC), Emulsionen, versprühbare Lösungen, Dispersionen auf Öl- oder Wasserbasis (SC), Suspoemulsionen (SC), Stäubemittel (DP), Beizmittel, Granulate in Form von Mikro-, Sprüh-, Aufzugs- und Adsorptionsgranulaten, wasserdispergierbare Granulate (WG), ULV-Formulierungen, Mikrokapseln, Wachse oder Köder.

Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986; van Falkenberg, "Pesticides Formulations", Marcel Dekker N.Y., 2nd Ed. 1972-73; K. Martens, "Spray Drying Handbook", 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J.; H.v.Olphen, "Introduction to Clay Colloid Chemistry", 2nd Ed., J. Wiley & Sons, N.Y.; Marschen, "Solvents Guide", 2nd Ed., Interscience, N.Y. 1950; McCutcheon's, "Detergents und Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Xthylenoxidaddukte", Wiss. Verlagsgesell., Stuttgart 1976; Winnacker-Küchler, "Chemische Technologie", Band 7, C. Hauser Verlag München, 4. Aufl. 1986.

Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 5 bis 80 Gew.-% betragen. Staubförmige Formulierungen enthalten meistens 5 bis 20 Gew.-% an Wirkstoff, versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise

auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Mit den äußeren Bedingungen wie Temperatur, Feuchtigkeit u.a. variiert die erforderliche Aufwandmenge. Sie kann innerhalb weiter Grenzen schwanken, z.B. zwischen 0,005 und 10,0 kg/ha oder mehr Aktivsubstanz, vorzugsweise liegt sie jedoch zwischen 0,01 und 5 kg/ha.

Die erfindungsgemäßen Wirkstoffe können in ihren handelsüblichen Formulierungen sowie in den aus diesen Formulierungen bereiteten Anwendungsformen in Mischungen mit anderen Wirkstoffen, wie Insektiziden, Lockstoffen, Sterilantien, Akariziden, Nematiziden, Fungiziden, wachstumsregulierenden Stoffen oder Herbiziden vorliegen.

Zu den Schädlingsbekämpfungsmitteln zählen beispielsweise Phosphorsäureester, Carbamate, Carbonsäureester, Formamidine, Zinnverbindungen, durch Mikroorganismen hergestellte Stoffe u.a.
Bevorzugte Mischungspartner sind

1. aus der Gruppe der Phosphorverbindungen
Acephate, Azamethiphos, Azinphos-ethyl, Azinphos-methyl, Bromophos, Bromophos-ethyl, Chlorfenvinphos, Chlormephos, Chlorpyrifos, Chlorpyrifos-methyl, Demeton, Demeton-S-methyl, Demeton-S-methyl sulfphone, Dialifos, Diazinon, Dichlorvos, Dicrotophos,
O,O-1,2,2,2-tetrachlorethylphosphorthioate (SD 208 304), Dimethoate, Disulfoton, EPN, Ethion, Ethoprophos, Etrimfos, Famphur, Fenamiphos, Fenitriothion, Fensulfothion, Fenthion, Fonofos, Formothion, Heptenophos, Isozophos, Isothioate, Isoxathion, Malathion, Methacrifos, Methamidophos, Methidation, Salithion, Mevinphos, Monocrotophos, Naled, Omethoate, Oxydemeton-methyl, Parathion, Parathion-methyl, Phenthoate, Phorate, Phosalone, Phosfolan, Phosmet, Phosphamidon, Phoxim, Pirimiphos-ethyl, Pirimiphos-methyl, Profenofos, Propaphos, Proetamphos, Prothiofos, Pyraclofos, Pyridapenthion, Quinalphos, Sulprofos, Temephos, Terbufos, Tetrachlorvinphos, Thiometon, Triazophos, Trichlorphon, Vamidothion;

2. aus der Gruppe der Carbamate
Aldicarb, 2-sec.-Butylphenylmethylcarbamate (BPMC), Carbaryl, Carbofuran, Carbosulfan, Cloethocarb, Benfuracarb, Ethiofencarb, Furathiocarb, Isoprocarb, Methomyl, 5-Methyl-m-cu-menylbutyryl(methyl)-carbamate, Ozamyl, Pirimicarb, Propoxur, Thiodicarb, Thiofanox, Ethyl 4,6,9-triaza-4-benzyl-6,10-dimethyl-8-oxa-7-oxo-5,11-dithia-9-dodecenoate (OK 135), 1-methylthio(ethylideneamino)N-methyl-N-(morpholinothio)carbamate (UC 51717);

3. aus der Gruppe der Carbonsäureester
Allethrin, Alphametrin, 5-Benzyl-3-furylmethyl-(E)-(1R)-cis-2,2-di-methyl-3-(2-oxothiolan-3-ylidenemethyl)-cyclopropanecarboxylate, Bioallethrin, Bioallethrin((S)-cyclopentylisomer), Bioresmethrin, Biphenate, (RS)-1-Cyano-1-(6-phenoxy-2-pyridyl)methyl-(1RS)-trans-3-(4-tert.butylphenyl)-2,2-dimethylcyclopropanecarboxylate (NCI 85193), Cycloprothrin, Cyfluthrin, Cyhalothrin, Cypermethrin, Cyphenothrin, Deltamethrin, Empenthrin, Esfenvalerate, Fenfluthrin, Fenpropathrin, Fenvalerate, Flucythrinate, Flumethrin, Fluvalinate (D-isomer), Permethrin, Pheothrin ((R)-Isomer), d-Pralethrin, Pyrethrine (natürliche Produkte), Resmethrin, Tefluthrin, Tetramethrin, Tralomethrin;

4. aus der Gruppe der Amidine
Amitraz, Chlordimeform;

5. aus der Gruppe der Zinnverbindungen
Cyhexatin, Fenbutatinoxide;

6. Sonstige
Abamectin, Bacillus thuringiensis, Bensultap, Binapacryl, Bromopropylate, Buprofezin, Camphechlor, Cartap, Chlorobenzilate, Chlorfluazuron, 2-(4-(Chlorphenyl)-4,5-di-phenylthiophen (UBI-T 930), Chlorfentezine, Cyclopropancarbonsäure-(2-naphthylmethyl)ester (Ro 12-0470), Cyromazin, N-(3,5-Dichlor-4-(1,1,2,3,3,3-hexafluor-1-propyloxy)phenyl)carbamoyl)-2-chlorbenzcarboximidsäureethylester, DDT, Dicofol, N-(N-(3,5-Di-chlor-4-(1,1,2,2-tetrafluorethoxy)phenylamino)carbonyl)-2,6-difluorbenzamid (XRD 473), Diflubenzuron, N-(2,3-Dihydro-3-methyl-1,3-thiazol-2-ylidene)-2,4-xylidine, Dinobuton, Dinocap, Endosulfan, Ethofenprox, (4-Ethoxyphenyl) (dimethyl)(3-(3-phenoxyphenyl)propyl)silan, (4-Ethoxyphenyl) (3-(4-fluoro-3-phenoxyphenyl)propyl)dimethylsilan, Fenoxycarb, 2-Fluoro-5-(4-(4-ethoxyphenyl)-4-methyl-1-pentyl)diphenylether (MTI 800), Granulose- und Kernpolyederviren, Fenthiocarb, Flubenzimine, Flucycloxuron, Flufenoxuron, Gamma-HCH, Hexythiazox, Hydramethylnon (AC 217300), Ivermectin, 2-Nitromethyl-4,5-dihydro-6H-thiazin (SD 52618), 2-Nitromethyl-3,4-dihydrothiazol (SD 35651), 2-Nitromethylene-1,2-thiazinan-3-ylcarbamaldehyde (WL 108477), Prapargite, Teflubenzuron, Tetradifon, Tetrasul, Thiocyclam, Triflumuron.

Der Wirkstoffgehalt der aus den handelsüblichen Formulierungen bereiteten Anwendungsformen kann von 0,00000001 bis zu 100 Gew.-% Wirkstoff, vorzugsweise zwischen 0,00001 und 1 Gew.-% liegen.

Die Anwendung geschieht in einer den Anwendungsformen angepaßten üblichen Weise.

Die erfindungsgemäßen Wirkstoffe eignen sich auch zur Bekämpfung von Endo- und Ektoparasiten auf dem veterinärmedizinischen Gebiet bzw. auf dem Gebiet der Tierhaltung.

Auf der Basis dieser Formulierungen lassen sich auch Kombinationen mit anderen pestizid wirksamen Stoffen, Düngemitteln und/oder Wachstumsregulatoren herstellen, z.B. in Form einer Fertigformulierung oder als Tankmix. Spritzpulver sind in Wasser gleichmäßig dispergierbare Präparate, die neben dem Wirkstoff außer einem Verdünnungs- oder Inertstoff noch Netzmittel, z.B. polyoxethylierte Alkylphenole, polyoxethylierte Fettalkohole, Alkyl- oder Alkylphenol-sulfonate und Dispergiermittel, z.B. ligninsulfonsaures Natrium, 2,2'-dinaphthylmethan-6,6'-disulfonsaures Natrium, dibutylnaphthalin-sulfonsaures Natrium oder auch oleylmethyltaurinsaures Natrium enthalten. Emulgierbare Konzentrate werden durch Auflösen des Wirkstoffes in einem organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwendet werden: Alkylarylsulfonsaure Calcium-Salze wie Ca-dodecylbenzolsulfonat oder nichtionische Emulgatoren wie Fettsäurepolyglykolester, Alkylarylpolyglykolether, Fettalkoholpolyglykolether, Propylenozid-Ethylenoxoid-Kondensationsprodukte, Alkylpolyether, Sorbitanfettsäureester, Polyoxyethylensorbitan-Fettsäureester oder Polyoxethylensorbitester.

Stäubemittel erhält man durch Vermahlen des Wirkstoffes mit fein verteilten festen Stoffen, z.B. Talkum, natürlichen Tonen wie Kaolin, Bentonit, Pyrophillit oder Diatomeenerde. Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch

Die Anwendung der erfindungsgemäßen Wirkstoffe geschieht hier in bekannter Weise wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgießen (pour-on and spot-on) und des Einpuderns sowie durch parenterale Anwendung in Form beispielsweise der Injektion.

Die erfindungsgemäßen neuen Verbindungen der Formel I können demgemäß auch besonders vorteilhaft in der Viehhaltung (z.B. Rinder, Schafe, Schweine und Geflügel wie Hühner, Gänse usw.) eingesetzt werden. In einer bevorzugten Ausführungsform der Erfindung werden den Tieren die neuen Verbindungen, gegebenenfalls in geeigneten Formulierungen (vgl. oben) und gegebenenfalls mit dem Trinkwasser oder Futter oral verabreicht. Da eine Ausscheidung im Kot in wirksamer Weise erfolgt, läßt sich auf diese Weise sehr einfach die Entwicklung von Insekten im Kot der Tiere verhindern. Die jeweils geeigneten Dosierungen und Formulierungen sind insbesondere von der Art und dem Entwicklungsstadium der Nutztiere und auch vom Befallsdruck abhängig und lassen sich nach den üblichen Methoden leicht ermitteln und festlegen. Die neuen Verbindungen können bei Rindern z.B. in Dosierungen von 0,01 bis 1 mg/kg Körpergewicht eingesetzt werden.

Die erfindungsgemäßen Verbindungen der Formel I zeichnen sich durch eine hervorragende fungizide Wirkung aus. Bereits in das pflanzliche Gewebe eingedrungene pilzliche Krankheitserreger lassen sich erfolgreich kurativ bekämpfen. Dies ist besonders wichtig und vorteilhaft bei solchen Pilzkrankheiten, die nach eingetretener Infektion mit den sonst üblichen Fungiziden nicht mehr wirksam bekämpft werden können. Das Wirkungszentrum der beanspruchten Verbindungen erfaßt eine Vielzahl verschiedener wirtschaftlich bedeutender, phytopathogener Pilze, wie z. B. Piricularia oryzae, Venturia inaegualis, Cercospora beticola, echte Mehltauarten, Fusariumarten, Plasmopora viticola, verschiedene Rostpilze und Pseudocercoporella herpotrichoides.

Die erfindungsgemäßen Verbindungen eignen sich daneben auch für den Einsatz in technischen Bereichen, beispielsweise als Holzschutzmittel, als Konservierungsmittel in Anstrichfarben, in Kühlschmiermittel für die Metallbearbeitung oder als Konservierungsmittel in Bohr- und Schneidölen.

Gegenstand der Erfindung sind auch fungizide Mittel, die die Verbindungen der Formel I neben geeigneten Formulierungshilfsmitteln enthalten. Die erfindungsgemäßen Mittel enthalten die Wirkstoffe der Formel I im allgemeinen zu 1 bis 95 Gew.-%.

Sie können auf verschiedene Art formuliert werden, je nachdem die es durch die biologischen und/oder chemisch-physikalischen Parameter vorgegeben ist. Als Formulierungsmöglichkeiten kommen daher in Frage:

Spritzpulver (WP), emulgierbare Konzentrate (EC), wäßrige Dispersionen auf Öl- oder Wasserbasis (SC), Suspoemulsionen (SC), Stäubemittel (DP), Beizmittel, Granulate in Form von wasserdispergierbare Granulate (WG), ULV-Formulierungen, Mikrokapseln, Wachse oder Köder.

Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986; van Falkenberg, "Pesticides Formulations", Marcel Dekker N.Y., 2nd Ed. 1972-73; K. Martens, "Spray Drying

Handbook", 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in:

Watkins, "Handbook of Insecticide Dust Diluents and Carrier", 2nd Ed., Darland Books, Caldwell N.J.; H.v.Olphen, "Introduction to Clay Colloid Chemistry, 2nd Ed., J. Wiley & Sons, N.Y.; Marsden, "Solvents Guide", 2nd Ed., Interscience, N.Y. 1950; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Auf der Basis dieser Formulierungen lassen sich auch Kombinationen mit anderen pestizid wirksamen Stoffen, Düngemitteln und/oder Wachstumsregulatoren herstellen, z. B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Netzmittel, z. B. polyoxethylierte Alkylphenole, polyoxethylierte Fettalkohole, Alkyl- oder Alkylphenol-sulfonate und Dispergiermittel, z. B. ligninsulfonsaures Natrium, 2,2'-dinaphthylmethan-6,6'-disulfonsaures Natrium, dibutylnaphthalinsulfonsaures Natrium oder auch oleylmethyltaurinsaures Natrium enthalten. Emulgierbare Konzentrate werden durch Auflösen des Wirkstoffes in einem organischen Lösungsmittel, z. B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwendet werden:

Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Sorbitanfettsäureester, Polyoxyethylensorbitan-Fettsäureester oder Polyoxethylensorbitester.

Stäubemittel erhält man durch Vermahlen des Wirkstoffes mit fein verteilten festen Stoffen, z. B. Talkum, natürlichen Tonen wie Kaolin, Bentonit, Poryphillit oder Diatomeenerde. Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z. B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

In Spritzpulvern beträgt die Wirkstoffkonzentration z. B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 5 bis 80 Gew.-% betragen. Staubförmige Formulierungen enthalten meistens 5 bis 20 Gew.-%. Bei Granulaten hängt der Wirkstoffgehalt zum Teil davon ab, ob die wirksame Verbindung flüssig oder fest vorliegt und welche Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Konzentrate gegebenenfalls in üblicher Weise verdünnt, z. B. bei Spritzpulvern, emulgierbaren Konzentraten, Dispersionen und teilweise auch bei Mikrogranulaten mittels Wasser.

Staubförmige und granulierte Zubereitungen sowie versprühbare Lösungen werden vor der Anwendung üblicherweise nicht mehr mit weiteren inerten Stoffen verdünnt.

Mit den äußeren Bedingungen wie Temperatur, Feuchtigkeit u.a. variiert die erforderliche Aufwandmenge. Sie kann innerhalb weiter Grenzen schwanken, z. B. zwischen 0,005 und 10,0 kg/ha oder mehr Aktivsubstanz, vorzugsweise liegt sie jedoch zwischen 0,01 und 5 kg/ha.

Die erfindungsgemäßen Wirkstoffe können in ihren handelsüblichen Formulierungen entweder allein oder in Kombination mit weiteren, literaturbekannten Fungiziden angewendet werden.

Als literaturbekannte Fungizide, die erfindungsgemäß mit den Verbindungen der Formel I kombiniert werden können,

Anilazine, Benalaxyl, Benodanil, Benomyl, Binapacryl, Bitertanol, Buthiobat, Captafol, Captan, Carbendazim, Carboxin, CGD-94240 F, Chlobenzthiazone, Chlorthalonil, Cymoxanil, Cyproconazole, Cyprofuram, Dichlofluanid, Dichlomezin, Diclobutrazol, Diethofencarb, Difluconazole, Dimethirimol, Dimethomorph, Diniconazole, Dinocap, Dithianon, Dodemorph, Dodine, Edifenfos, Ethirimol, Etridiazol, Fenarimol, Fenfuram, Fenpiclonil, Fenpropidin, Fenpropomorph, Fentinacetate, Fentinhydroxide, Fluaziram, Fluobenzimine, Fluorimide, Flusilazole, Flutolanil, Flutriafol, Folpet, Fosetylaluminium, Fuberidazole, Furalaxyl, Furmecyclox, Guazatine, Hexaconazole, Imazalil, Iprobenfos, Iprodione, Isoprothiolane, Kupferverbindungen wie Cu-oxychlorid, Oxine-Cu, Cu-oxide, Mancozeb, Maneb, Mepronil, Metalaxyl, Methasulfocarb, Methfuroxam, Myclobutanil, Nabam,

Nitrothalidopropyl, Nuarimol, Ofurace, Oxadixyl, Oxycarboxin, Penconazol, Pencycuron, PP 969, Probenazole, Probineb, Prochloraz, Procymidon, Propamocarb, Propiconazol, Prothiocarb, Pyracarbolid, Pyrifenox, Pyroquilon, Rabenzazole, Schwefel, Tebuconazole, Thiabendazole, Thiofanatemethyl, Thiram, Tolclofosmethyl, Tolylfluanid, Triadimefon, Triadimenol, Tricyclazole, Tridemorph, Triflumizol, Triforine, Vinchlozolin, Zineb,

Natrium-dodecylsulfonat, Natrium-dodecylsulfat, Natrium-C13/C15-alkoholethersulfonat, Natrium-cetostearylphosphatester, Dioctyl-natriumsulfosuccinat, Natrium-isopropylnapthalinsulfonat, Natrium-methylenbisnaphthalinsulfonat, Cetyl-trimethyl-ammoniumchlorid, Salze von langkettigen primären, sekundären oder tertiären Aminen, Alkyl-propylenamine, Lauryl-pyrimidiniumbromid, ethoxilierte quarternierte Fettamine, Alkyldimethyl-benzyl-ammoniumchlorid und 1-Hydroxyethyl-2-alkyl-imidazolin.

Die oben genannten Kombinationspartner stellen bekannte Wirkstoffe dar, die zum großen Teil in C. R. Worthing, S. B. Walker, The Pesticide Manual, 7. Auflage (1983), British Crop Protection Council,beschrieben sind.

Darüber hinaus können die erfindungsgemäßen Wirkstoffe, insbesondere die der aufgeführten Beispiele, in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen.

Der Wirkstoffgehalt der aus den handelsüblichen Formulierungen bereiteten Anwendungsformen kann in weiten Bereichen variieren, die Wirkstoffkonzentration der Anwendungsformen kann von 0,0001 bis zu 100 Gew.-% Wirkstoff, vorzugsweise zwischen 0,001 und 1 Gew.-% liegen. Die Anwendung geschieht in einer den Anwendungsformen angepaßten üblichen Weisen.

Nachfolgende Beispiele dienen zur Erläuterung der Erfindung.

A) Formulierungsbeispiele

a) Ein Stäubemittel wird erhalten, indem man 10 Gew.-Teile Wirkstoff und 90 Gew.-Teile Talkum als Inertstoff mischt und in einer Schlagmühle zerkleinert.

b) Ein in Wasser leicht dispergierbares, benetzbares Pulver wird erhalten, indem man 25 Gew.-Teile Wirkstoff, 65 Gew.-Teile kaolinhaltigen Quarz als Inertstoff, 10 Gew.-Teile ligninsulfonsaures Kalium und 1 Gew.-Teil oleoylmethyltaurinsaures Natrium als Netz- und Dispergiermittel mischt und in einer Stiftmühle mahlt.

c) Ein in Wasser leicht dispergierbares Dispersionskonzentrat stellt man her, indem man 40 Gew.-Teile Wirkstoff mit 7 Gew.-Teilen eines Sulfobernsteinsäurehalbesters, 2 Gew.-Teilen eines Ligninsulfonsäure-Natriumsalzes und 51 Gew.-Teilen Wasser mischt und in einer Reibkugelmühle auf eine Feinheit von unter 5 Mikron vermahlt.

d) Ein emulgierbares Konzentrat läßt sich herstellen aus 15 Gew.-Teilen Wirkstoff, 75 Gew.-Teilen Cyclohexanon als Lösungsmittel und 10 Gew.-Teilen oxethyliertem Nonylphenol (10 AeO) als Emulgator.

e) Ein Granulat läßt sich herstellen aus 2 bis 15 Gew.-Teilen Wirkstoff und einem inerten Granulatträgermaterial wie Attapulgit, Bimsgranulat und/oder Quarzsand. Zweckmäßigerweise verwendet man eine Suspension des Spritzpulvers aus Beispiel b) mit einem Feststoffanteil von 30 % und spritzt diese auf die Oberfläche eines Attapulgitgranulats, trocknet und vermischt innig. Dabei beträgt der Gewichtsanteil des Spritzpulvers ca. 5 % und der des inerten
Trägermaterials ca.
95 % des fertigen Granulats.

B) Chemische Beispiele

Beispiel A

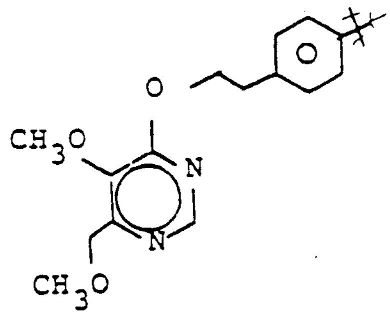

Zu einer Suspension von 1,2 g (0,03 Mol) Natriumhydrid (60%ige Dispersion in Öl) in 50 ml trockenen Dimethylformamid tropfte man bei 50 ° C 5,4 g (0,03 Mol) 2-(4-tert.-Butylphenyl)-ethanol und rührte bis zum Ende der Wasserstoffentwicklung. Man kühlte auf 0 ° C ab und gab 4,7 g (0,025 Mol) 4-Chlor-5-methoxy-6-methoxymethyl-pyrimidin (Coll. Czechoslov. Chem. Commun. 33, 2266 (1968)) zu. Man rührte 1 h bei Raumtemperatur und 2 h bei 100 ° C. Nach Abziehen des Lösungsmittels wurde mit Dichlormethan/Wasser aufgenommen, die organische Phase getrocknet und einrotiert. Das Rohprodukt wurde an Kieselgel mit Ethylacetat chromatographiert. Man erhielt 3,8 g (46 % d.Th.) 4-[2-(4-tert.-Butylphenyl)-ethoxy]-5-methoxy-6-methoxymethyl-pyrimidin als gelbes Öl.

Beispiel B

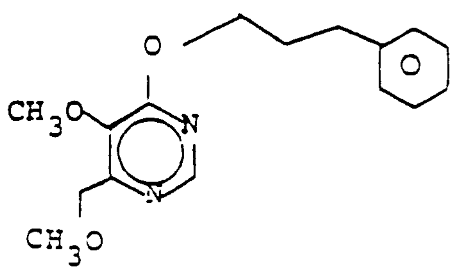

Zu einem Gemisch aus 1,2 g (0,03 Mol) Natriumhydrid (60%ige Dispersion in Öl) und 5,7 g (0,03 Mol) 4-Chlor-5-methoxy-6-methoxymethyl-pyrimidin in 50 ml trockenem Dimethylformamid tropfte man bei 10-20 ° C eine Lösung von 5,8 g (0,03 Mol) 3-(4-tert.-Butylphenyl)-propanol in 20 ml trockenem Dimethylformamid. Man rührte 1 h bei Raumtemperatur und 2 h bei 100 ° C. Nach Aufarbeitung und chromatographischer Reinigung analog Beispiel A erhielt man 2,5 g (24%) 4-[3-(4-tert.-Butylphenyl)propoxy]-5-methoxy-6-methoxymethyl-pyrimdin als gelbes Öl.

Beispiel C

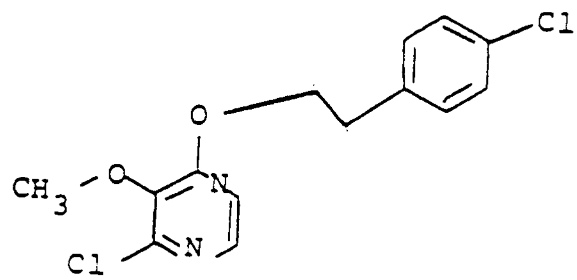

Zu einer Suspension von 680 mg (22,7 mmol) Natriumhydrid (80% Suspension in Öl) in 40 ml trockenem Tetrahydrofuran tropfte man bei 40 °C 2,40 g (15,2 mmol) 2-(4-Chlorphenyl)-ethanol zu und rührte bis zum Ende der Wasserstoffentwicklung. Danach ließ man auf Raumtemperatur abkühlen und gab dann portionsweise 2,7 g (15,2 mmol) 4,6-Dichlor-5-methoxypyrimidin (Monatshefte Chem. 96, 1661 (1965) zu. Man rührte 1 h bei Raumtemperatur und 4 h bei 40 °C. Zur Aufarbeitung goß man das Reaktionsgemisch auf gesättigte Ammoniumchloridlösung und extrahierte mit Diethylether. Die vereinigte organische Phase wurde über Magnesiumsulfat getrocknet und im Vakuum eingeengt. Der Rückstand wurde an Kieselgel mit n-Heptan/Ethylacetat (4:1) chromatographiert. Man erhielt 4,0 g (88% d. Th.) 4-[2-(4-Chlorphenyl)-ethoxy]-5-methoxy-6-chlor-pyrimidin als zähes Öl, das während der Trocknung langsam kristallisierte (Schmelzpunkt 70-71 °C).

Beispiel D

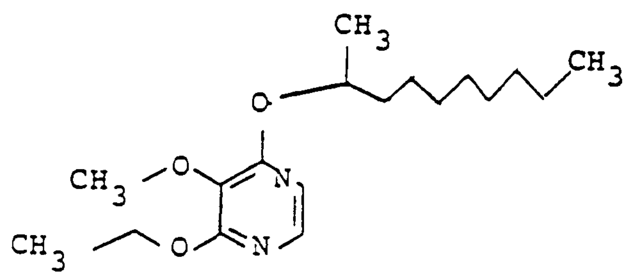

Zu einer Suspension von 650 mg (21,6 mmol) Natriumyhdrid (80% Suspension in Öl) in 60 ml trockenem Diemthylformamid tropfte man bei 50 °C 2,7 g (17,0 mmol) 2-Decanol zu und rührte bis zum Ende der Wasserstoffentwicklung. Man kühlte auf Raumtemperatur ab und gab portionsweise 3,0 g (15,9 mmol) 4-Chlor-5-methoxy-6-ethoxy-pyrimidin (Herstellung analog Beispiel C) zu. Nach 30 Minuten erhöhte man die Reaktionstemperatur auf 50 °C und rührte 12 h weiter. Das Reaktionsgemisch wurde auf gesättigte Ammoniumchloridlösung gegossen und anschließend mit Diethylether extrahiert. Die vereinigte organische Phase wurde mit Wasser gewaschen, über Magnesiumsulfat getrocknet und im Vakuum eingeengt. Der Rückstand wurde an Kieselgel mit n-Heptan/Ethylacetat (4:1) chromatographiert. Man erhielt 1,4 g (28% d. Th.) 4-(2-Decyloxy)-5-methoxy-6-ethoxy-pyrimidin als gelbes Öl.

Beispiel E

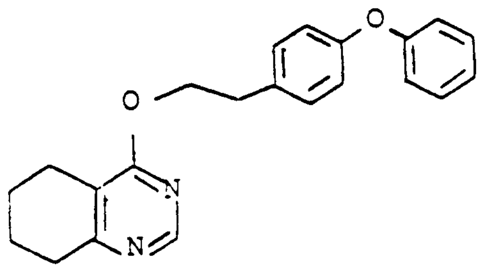

Zu einer Suspension von 700 mg (23,3 mmol) Natriumhydrid (80% Suspension in Öl) in 50 ml trockenem Tetrahydrofuran wurden 3,8 g (17,8 mmol) 2-[4-(Phenoxy)-phenyl]-ethanol zugegeben und bei 50 ° C bis zum Ende der Wasserstoffentwicklung gerührt. Man kühlte auf Raumtemperatur ab und gab 3,0 g (17,8 mmol) 4-Chlor-5,6,7,8-tetrahydro-chinazolin zu. Danach wurde 5 h auf 40 ° C erwärmt. Das Reaktionsgemisch wurde auf gesättigte Ammoniumchloridlösung gegossen und anschließend mit Diethylether extrahiert. Die vereinigte organische Phase wurde über Magnesiumsulfat getrocknet und im Vakuum eingeengt. Der Rückstand wurde an Kieselgel mit n-Heptan/Ethylacetat (2:1) chromatographiert. Man erhielt 3,2 g 4-[2-(4-(Phenoxy)-phenyl)-ethoxy]-5,6,7,8-tetrahydro-chinazolin als zähes gelbes Öl.

Weitere Beispiele finden sich in der folgenden Tabelle 1. Die in der Tabelle aufgeführten Reste $R^1$ bis $R^4$ und Q entsprechen den Symbolen in der Formel I

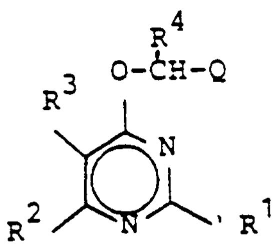

(I).

Tabelle I

| Beisp. Nr. | $R^1$ | $R^2$ | $R^3$ | $R^4$ | Q | Fp [°C] |
|---|---|---|---|---|---|---|
| 1 | H | $CH_2OCH_3$ | $OCH_3$ | $CH_3$ | $(CH_2)_3CH_3$ | Öl |
| 2 | H | $CH_2OCH_3$ | $OCH_3$ | $CH_3$ | $(CH_2)_5CH_3$ (R-Form) | Öl |
| 3 | H | $CH_2OCH_3$ | $OCH_3$ | $CH_3$ | $(CH_2)_5CH_3$ (S-Form) | Öl |
| 4 | H | $CH_2OCH_3$ | $OCH_3$ | $CH_3$ | $(CH_2)_7CH_3$ | Öl |
| 5 | H | $CH_2OCH_3$ | $OCH_3$ | H | $(CH_2)_8CH_3$ | Öl |
| 6 | H | $CH_2OCH_3$ | $OCH_3$ | $CF_3$ | $(CH_2)_7CH_3$ | Öl |
| 7 | H | $-(CH_2)_4-$ | | $CF_3$ | $(CH_2)_7CH_3$ | Öl |
| 8 | H | $CH_2OCH_3$ | $OCH_3$ | $C_2H_5$ | $(CH_2)_4CH_3$ | Öl |
| 9 | H | $OCH_3$ | $OCH_3$ | $CH_3$ | $(CH_2)_7CH_3$ | Öl |
| 10 | H | Cl | $OCH_3$ | $CH_3$ | $(CH_2)_7CH_3$ | Öl |
| 11 | H | $OC_2H_5$ | $OCH_3$ | $CH_3$ | $(CH_2)_7CH_3$ | Öl |
| 12 | H | $C_2H_5$ | $OCH_3$ | $CH_3$ | $(CH_2)_7CH_3$ | Öl |
| 13 | H | $C_2H_5$ | $C_2H_5$ | $CH_3$ | $(CH_2)_7CH_3$ | Öl |
| 14 | H | $-(CH_2)_4-$ | | $CH_3$ | $(CH_2)_7CH_3$ | Öl |
| 15 | H | $CH_2OCH_3$ | $OCH_3$ | H | $CH_2O(CH_2)_5CH_3$ | Öl |

Tabelle I

| Beisp. Nr. | $R^1$ | $R^2$ | $R^3$ | $R^4$ | Q | Fp [°C] |
|---|---|---|---|---|---|---|
| 16 | H | $CH_2OCH_3$ | $OCH_3$ | H | $CH_2O(CH_2)_7CH_3$ | Öl |
| 17 | H | $CH_2OCH_3$ | $OCH_3$ | H | $CH_2O(CH_2)_9CH_3$ | Öl |
| 18 | H | Cl | $OCH_3$ | H | $(CH_2)O(CH_2)_5CH_3$ | Öl |
| 19 | H | Cl | $OCH_3$ | H | $CH_2O(CH_2)_7CH_3$ | Öl |
| 20 | H | Cl | $OCH_3$ | H | $CH_2O(CH_2)_9CH_3$ | Öl |
| 21 | H | $OCH_3$ | $OCH_3$ | H | $CH_2O(CH_2)_7CH_3$ | Öl |
| 22 | H | $OCH_3$ | $OCH_3$ | H | $CH_2O(CH_2)_9CH_3$ | 32 |
| 23 | H | $CH_2OCH_3$ | $OCH_3$ | H | $(CH_2)_3CH(CH_3)-(CH_2)_3CH(CH_3)_2$ | Öl |
| 24 | H | $CH_2OCH_3$ | Cl | $CH_3$ | $(CH_2)_7CH_3$ | Öl |
| 25 | H | $CF_3$ | Cl | $CH_3$ | $(CH_2)_7CH_3$ | Öl |
| 26 | H | $CH_2OCH_3$ | $OCH_3$ | H | $CH_2$-O- 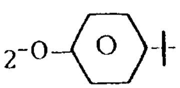 | Öl |
| 27 | H | $OCH_3$ | $OCH_3$ | H | $CH_2$-O 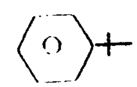 | Öl |
| 28 | H | Cl | $OCH_3$ | H | $CH_2$-O- 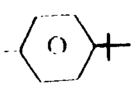 | Öl |

Tabelle I

| Beisp. Nr. | $R^1$ | $R^2$ | $R^3$ | $R^4$ | Q | Fp [°C] |
|---|---|---|---|---|---|---|
| 29 | H | $-(CH_2)_4-$ | | H | 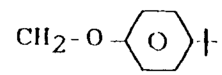 | 91-95 |
| 30 | H | $-(CH_2)_4-$ | | H | 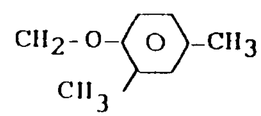 | 62-66 |
| 31 | H | Cl | $OCH_3$ | H | 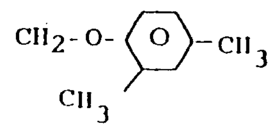 | Öl |
| 32 | H | Cl | $OCH_3$ | H | 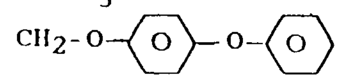 | 55 |
| 33 | H | $CH_2OCH_3$ | $OCH_3$ | H | 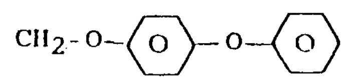 | Öl |
| 34 | H | $CH_2OCH_3$ | $OCH_3$ | H | 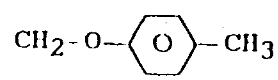 | Öl |
| 35 | H | $CH_2OCH_3$ | $OCH_3$ | H | 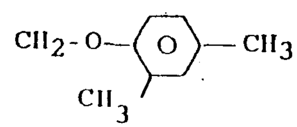 | Öl |
| 36 | H | $CH_2OCH_3$ | $OCH_3$ | H | 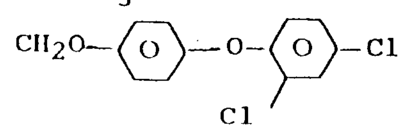 | Öl |
| 37 | H | Cl | $OCH_3$ | H | 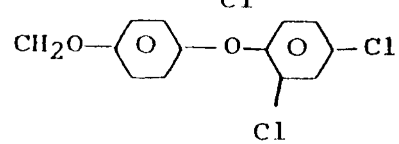 | Öl |

Tabelle I

| Beisp. Nr. | $R^1$ | $R^2$ | $R^3$ | $R^4$ | Q | Fp [°C] |
|---|---|---|---|---|---|---|
| 38 | H | $-(CH_2)_4-$ | | H | 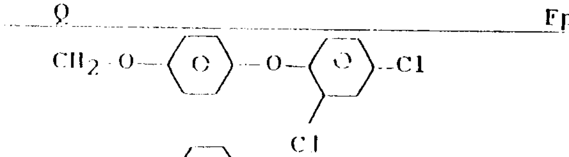 | 77-79 |
| 39 | H | $-(CH_2)_4-$ | | H | 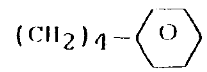 | öl |
| 40 | H | $CH_2OCH_3$ | $OCH_3$ | H | 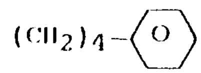 | öl |
| 41 | H | H | $OCH_3$ | H | 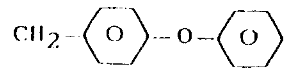 | 42-43 |
| 42 | H | H | $OCH_3$ | H | 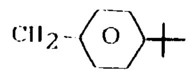 | öl |
| 43 | H | $-(CH_2)_4$ | | H | 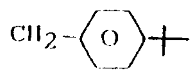 | 62-63 |
| 44 | H | $-(CH_2)_4$ | | H | 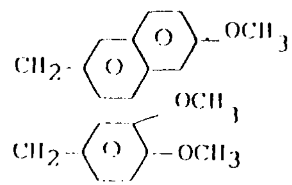 | 87-89 |
| 45 | H | $CH_2OCH_3$ | $OCH_3$ | H | | öl |
| 46 | H | $CH_2OCH_3$ | $OCH_3$ | H | 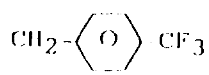 | öl |

Tabelle I

| Beisp. Nr. | $R^1$ | $R^2$ | $R^3$ | $R^4$ | Q | Fp [°C] |
|---|---|---|---|---|---|---|
| 47 | H | $C_2H_5$ | $C_2H_5$ | H | $CH_2$- 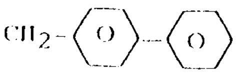 | Öl |
| 48 | H | $C_2H_5$ | $C_2H_5$ | H | $CH_2$- 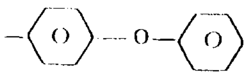 | 65-67 |
| 49 | H | -$(CH_2)_4$- | | H | $CH_2$- 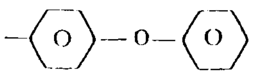 | Öl |
| 50 | H | -$(CH_2)_4$- | | H | $CH_2$- 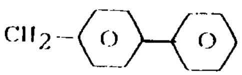 | 94-95 |
| 51 | H | $C_2H_5$ | $C_2H_5$ | H | $CH_2$- 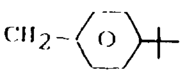 | Öl |
| 52 | H | $C_2H_5$ | $C_2H_5$ | H | $CH_2$- 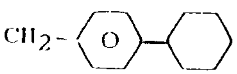 | Öl |
| 53 | H | $C_2H_5$ | H | H | $(CH_2)_6CH_3$ | Öl |
| 54 | H | $CH_2OCH_3$ | $OCH_3$ | H | $CH_2$- 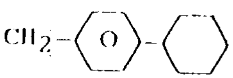 | Öl |
| 55 | H | Cl | $OCH_3$ | $C_2H_5$ | $(CH_2)_6CH_3$ | 63 |
| 56 | H | $C_2H_5$ | Cl | H | $CH_2$- 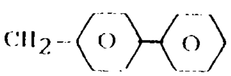 | Öl |

Tabelle I

| Beisp. Nr. | $R^1$ | $R^2$ | $R^3$ | $R^4$ | Q | Fp [°C] |
|---|---|---|---|---|---|---|
| 57 | H | $-(CH_2)_4$ | | H | $-CH_2-$ 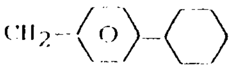 | 83 |
| 58 | H | $CH_2OCH_3$ | $OCH_3$ | H | $CH_2OCH_2-$ 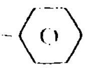 | Öl |
| 59 | H | $OCH_3$ | $OCH_3$ | H | $CH_2OCH_2-$ 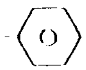 | Öl |
| 60 | H | $C_2H_5$ | Cl | H | $CH_2OCH_2-$ 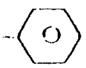 | Öl |
| 61 | H | Cl | $OCH_3$ | H | $-CH_2OCH_2-$ 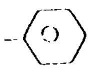 | Öl |
| 62 | H | $CH_2OCH_3$ | $OCH_3$ | $C_2H_5$ | 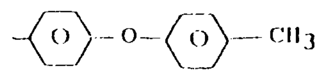 | Öl |
| 63 | H | $CH_2OCH_3$ | $OCH_3$ | H | $-C(CH_3)_2CH_2OCH_2-$ 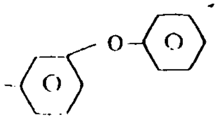 | Öl |
| 64 | H | $CH_2OCH_3$ | $OCH_3$ | H | 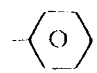 | Öl |
| 65 | H | $CH_2OCH_3$ | $OCH_3$ | H | 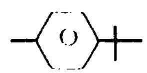 | Öl |
| 66 | H | $CH_2OCH_3$ | $OCH_3$ | H | 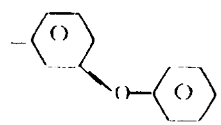 | Öl |

Tabelle I

| Beisp. Nr. | $R^1$ | $R^2$ | $R^3$ | $R^4$ | Q | Fp [°C] |
|---|---|---|---|---|---|---|
| 67 | H | $CH_2OCH_3$ | $OCH_3$ | H | 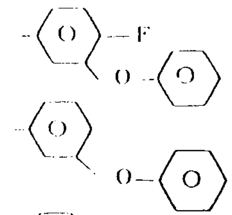 | Öl |
| 68 | H | Cl | $OCH_3$ | H | | Öl |
| 69 | H | $C_2H_5$ | H | H | 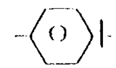 | Öl |
| 70 | H | $C_2H_5$ | Cl | $C_2H_5$ | 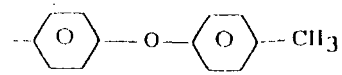 | Öl |
| 71 | H | $CF_2CH_2O$ | H | H | 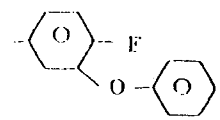 | Öl |
| 72 | H | $CH_2OCH_3$ | $OCH_3$ | H | 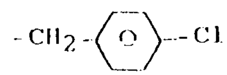 | Öl |
| 73 | H | $CH_2OCH_3$ | $OCH_3$ | H | 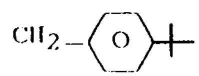 | Öl |
| 74 | H | $CH_2OCH_3$ | $OCH_3$ | H | 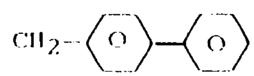 | Öl |
| 75 | H | $CH_2OCH_3$ | $OCH_3$ | H | 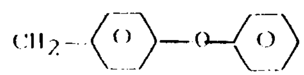 | Öl |

Tabelle I

| Beisp. Nr. | $R^1$ | $R^2$ | $R^3$ | $R^4$ | Q | Fp [°C] |
|---|---|---|---|---|---|---|
| 76 | H | $CH_2OCH_3$ | $OCH_3$ | H | 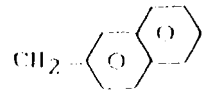 | Öl |
| 77 | H | $CH_2OCH_3$ | $OCH_3$ | H | 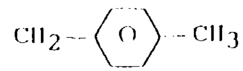 | Öl |
| 78 | H | $CH_2OCH_3$ | $OCH_3$ | H | 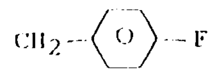 | Öl |
| 79 | H | $CH_2OCH_3$ | $OCH_3$ | H | 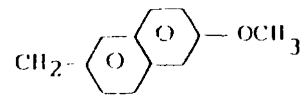 | 43-45 |
| 80 | H | $CH_2OCH_3$ | $OCH_3$ | H | 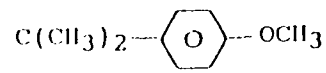 | Öl |
| 81 | H | $CH_2OCH_3$ | $OCH_3$ | H | 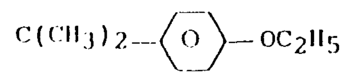 | Öl |
| 82 | H | $CH_2OCH_3$ | $OCH_3$ | H | 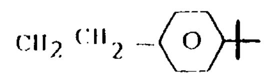 | Öl |
| 83 | H | Cl | $OCH_3$ | H | 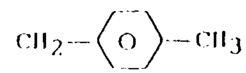 | Öl |
| 84 | H | Cl | $OCH_3$ | H | 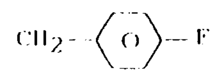 | 57-59 |

Tabelle I

| Beisp. Nr. | $R^1$ | $R^2$ | $R^3$ | $R^4$ | Q | Fp [°C] |
|---|---|---|---|---|---|---|
| 85 | H | Cl | $OCH_3$ | H | 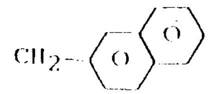 | 79-81 |
| 86 | H | Cl | $OCH_3$ | H | 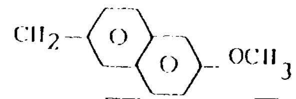 | 81 |
| 87 | H | Cl | $OCH_3$ | H | 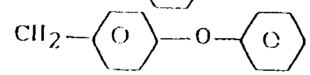 | Öl |
| 88 | H | Cl | $OCH_3$ | H | 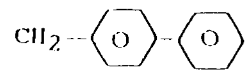 | 63-65 |
| 89 | H | Cl | $OCH_3$ | H | 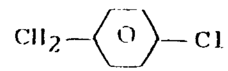 | 70-71 |
| 90 | H | Cl | $OCH_3$ | H | 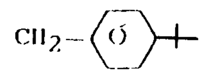 | Öl |
| 91 | H | Cl | $OCH_3$ | H | 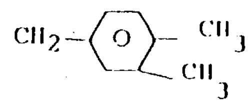 | Öl |
| 92 | H | $OCH_3$ | $OCH_3$ | H | 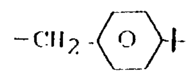 | Öl |
| 93 | H | $CH_2OCH_3$ | $OCH_3$ | $C_2H_5$ | 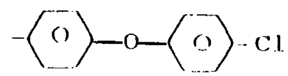 | Öl |

Tabelle I

| Beisp. Nr. | $R^1$ | $R^2$ | $R^3$ | $R^4$ | Q | Fp [°C] |
|---|---|---|---|---|---|---|
| 94 | H | $C_2H_5$ | Cl | $C_2H_5$ | 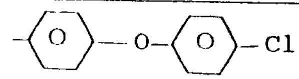 | Öl |
| 95 | H | $C_2H_5$ | Cl | H | $CH_2$- 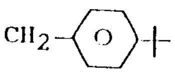 | Öl |
| 96 | H | $C_2H_5$ | Cl | H | $CH_2$- 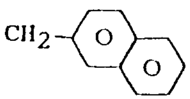 | Öl |
| 97 | H | $C_2H_5$ | Cl | H | $CH_2$- 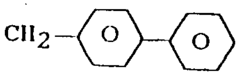 | Öl |
| 98 | H | $C_2H_5$ | Cl | H | $CH_2$- 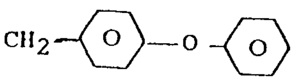 | Öl |
| 99 | H | $C_2H_5$ | Cl | H | $CH_2$- 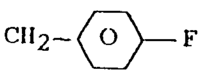 | Öl |
| 100 | H | $OC_2H_5$ | H | H | $CH_2$- 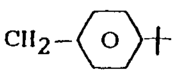 | Öl |
| 101 | H | $OCH_2CF_3$ | H | H | $CH_2$- 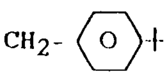 | Öl |
| 102 | H | $OCH_3$ | H | H | $CH_2$- 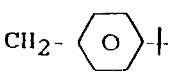 | Öl |

Tabelle I

| Beisp. Nr. | $R^1$ | $R^2$ | $R^3$ | $R^4$ | Q | Fp [°C] |
|---|---|---|---|---|---|---|
| 103 | H | $-(CH_2)_4-$ | | $C_2H_5$ | 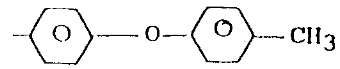 | Öl |
| 104 | H | $-(CH_2)_4-$ | | $C_2H_5$ | 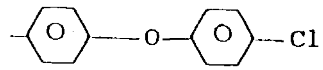 | Öl |
| 105 | H | $CH_2OCH_3$ | $OCH_3$ | $CH_3$ | 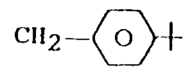 | Öl |
| 106 | H | $C_2H_5$ | Cl | $CH_3$ | 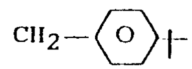 | Öl |
| 107 | H | $-(CH_2)_3-$ | | $CH_3$ | 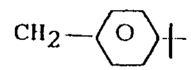 | Öl |
| 108 | H | $-(CH_2)_4-$ | | $CH_3$ | 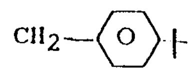 | Öl |
| 109 | $CH_3$ | $CH_2OCH_3$ | $OCH_3$ | H | 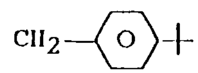 | Öl |
| 110 | H | $CH_2OCH_3$ | $OCH_3$ | $C_2H_5$ | 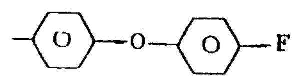 | |
| 111 | H | $C_2H_5$ | Cl | $C_2H_5$ | 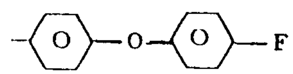 | |
| 112 | H | $-(CH_2)_4$ | | $C_2H_5$ | 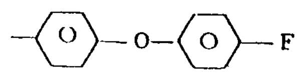 | |

Tabelle I

| Beisp. Nr. | $R^1$ | $R^2$ | $R^3$ | $R^4$ | Q | Fp [°C] |
|---|---|---|---|---|---|---|
| 113 | H | $CH_2OCH_3$ | $OCH_3$ | $C_2H_5$ | 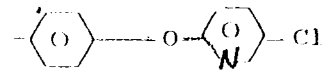 | |
| 114 | H | $C_2H_5$ | Cl | $C_2H_5$ | 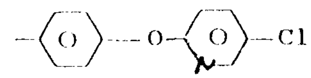 | |
| 115 | H | $-(CH_2)_4-$ | | $C_2H_5$ | 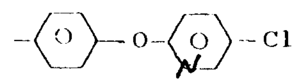 | |
| 116 | H | $CH_2OCH_3$ | $OCH_3$ | $C_2H_5$ | 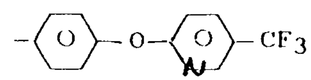 | |
| 117 | H | $C_2H_5$ | Cl | $C_2H_5$ | 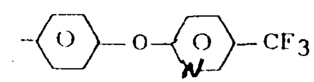 | |
| 118 | H | $-(CH_2)_4-$ | | $C_2H_5$ | 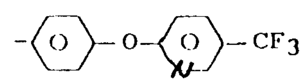 | |
| 119 | H | $CH_2OCH_3$ | $OCH_3$ | $C_2H_5$ | 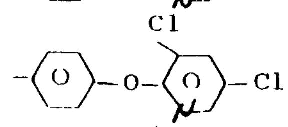 | |
| 120 | H | $C_2H_5$ | Cl | $C_2H_5$ | 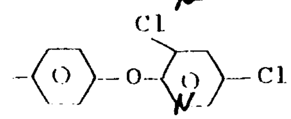 | |

Tabelle I

| Beisp. Nr. | $R^1$ | $R^2$ | $R^3$ | $R^4$ | Q | Fp [°C] |
|---|---|---|---|---|---|---|
| 121 | H | $-(CH_2)_4$ | | $C_2H_5$ | 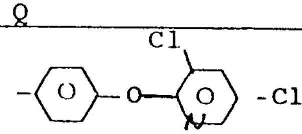 | |
| 122 | H | $CH_2OCH_3$ | $OCH_3$ | $C_2H_5$ | 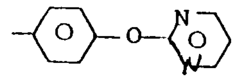 | |
| 123 | H | $C_2H_5$ | Cl | $C_2H_5$ | 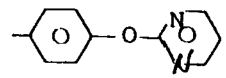 | |
| 124 | H | $-(CH_2)_4$ | | $C_2H_5$ | 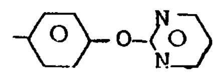 | |
| 125 | H | $CH_2OCH_3$ | $OCH_3$ | $C_2H_5$ | 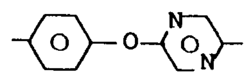 | |
| 126 | H | $C_2H_5$ | Cl | $C_2H_5$ | 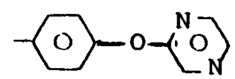 | |
| 127 | H | $-(CH_2)_4$ | | $C_2H_5$ | 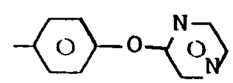 | |

Tabelle I

| Beisp. Nr. | $R^1$ | $R^2$ | $R^3$ | $R^4$ | Q | Fp [°C] |
|---|---|---|---|---|---|---|
| 128 | H | $CH_2OCH_3$ | $OCH_3$ | $C_2H_5$ | 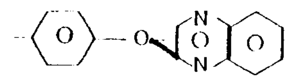 | |
| 129 | H | $C_2H_5$ | Cl | $C_2H_5$ | 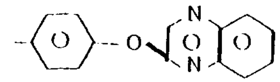 | |
| 130 | H | $-(CH_2)_4$ | | $C_2H_5$ | 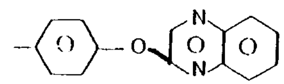 | |
| 131 | H | $CH_2OCH_3$ | $OCH_3$ | $C_2H_5$ | 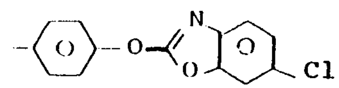 | |
| 132 | H | $C_2H_5$ | Cl | $C_2H_5$ | 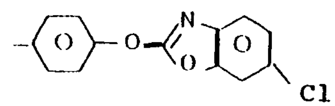 | |
| 133 | H | $-(CH_2)_4$ | | $C_2H_5$ | 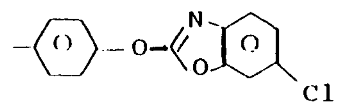 | |
| 134 | H | $CH_2OCH_3$ | $OCH_3$ | $C_2H_5$ | 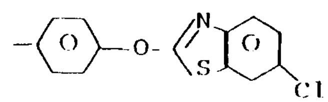 | |

Tabelle I

| Beisp. Nr. | $R^1$ | $R^2$ | $R^3$ | $R^4$ | Q | Fp [°C] |
|---|---|---|---|---|---|---|
| 135 | H | $C_2H_5$ | Cl | $C_2H_5$ | 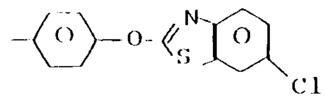 | |
| 136 | H | $-(CH_2)_4-$ | | $C_2H_5$ | 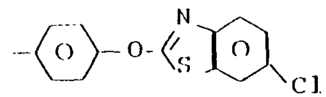 | |
| 137 | H | $CH_2OCH_3$ | $OCH_3$ | $C_2H_5$ | 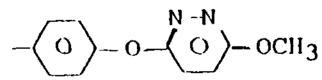 | |
| 138 | H | $C_2H_5$ | Cl | $C_2H_5$ | 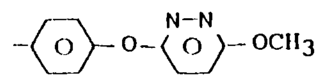 | |
| 139 | H | $-(CH_2)_4-$ | | $C_2H_5$ | 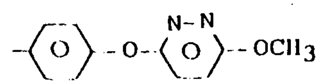 | |
| 140 | H | $CH_2OCH_3$ | $OCH_3$ | $C_2H_5$ | 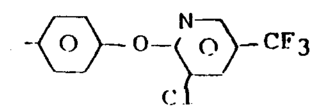 | |
| 141 | H | $C_2H_5$ | Cl | $C_2H_5$ | 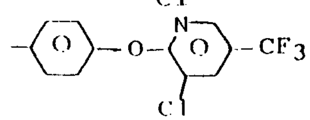 | |

Tabelle I

| Beisp. Nr. | $R^1$ | $R^2$ | $R^3$ | $R^4$ | Q | Fp [°C] |
|---|---|---|---|---|---|---|
| 142 | H | $-(CH_2)_4$ | | $C_2H_5$ | 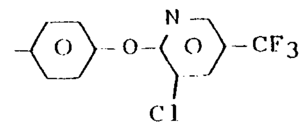 | |
| 143 | H | $CH_2OCH_3$ | $OCH_3$ | H | 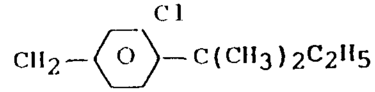 | Öl |
| 144 | H | $C_2H_5$ | Cl | H | 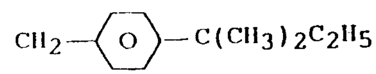 | Öl |
| 145 | H | $-(CH_2)_4$ | | H | 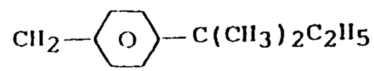 | Öl |
| 146 | H | $-(CH_2)_4$ | | $CH_3$ | 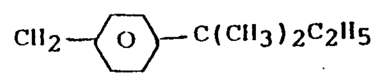 | Öl |
| 147 | H | $CH_2OCH_3$ | $OCH_3$ | $CH_3$ | 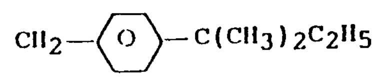 | Öl |
| 148 | H | $C_2H_5$ | Cl | $CH_3$ | 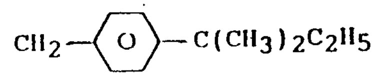 | Öl |
| 149 | H | $C_2H_5$ | Cl | H | 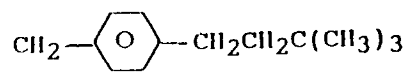 | Öl |
| 150 | H | $CH_2OCH_3$ | $OCH_3$ | H | 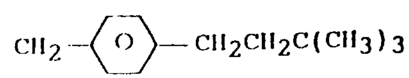 | Öl |

Tabelle I

| Beisp. Nr. | $R^1$ | $R^2$ | $R^3$ | $R^4$ | Q | Fp [°C] |
|---|---|---|---|---|---|---|
| 151 | H | $-(CH_2)_4$ | | H | 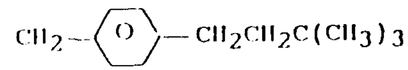 | |
| 152 | H | $-(CH_2)_4$ | | H | 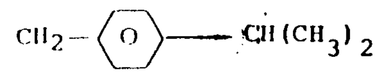 | Öl |
| 153 | H | $C_2H_5$ | Cl | H | 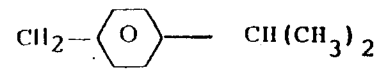 | |
| 154 | H | $CH_2OCH_3$ | $OCH_3$ | H | 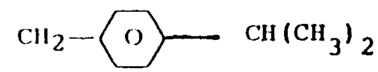 | Öl |
| 155 | H | $CH_2OCH_3$ | $OCH_3$ | H | 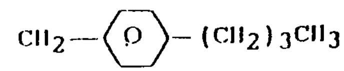 | Öl |
| 156 | H | $C_2H_5$ | Cl | H | 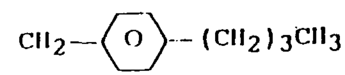 | |
| 157 | H | $-(CH_2)_4-$ | | H | 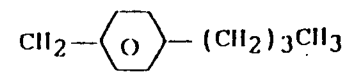 | Öl |
| 158 | H | $-(CH_2)_4-$ | | H | 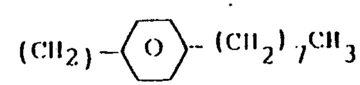 | Öl |
| 159 | H | $C_2H_5$ | Cl | H | 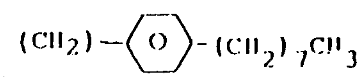 | |
| 160 | H | $CH_2OCH_3$ | $OCH_3$ | H | 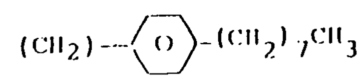 | Öl |

Tabelle I

| Beisp. Nr. | $R^1$ | $R^2$ | $R^3$ | $R^4$ | Q | Fp [°C] |
|---|---|---|---|---|---|---|
| 161 | H | $CH_2OCH_3$ | $OCH_3$ | H | 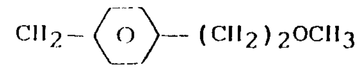 | |
| 162 | H | $C_2H_5$ | Cl | H | 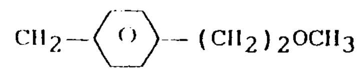 | |
| 163 | H | $-(CH_2)_4-$ | | H | 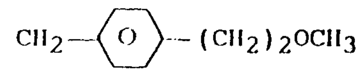 | |
| 164 | H | $CH_2OCH_3$ | $OCH_3$ | H | 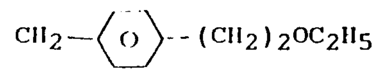 | |
| 165 | H | $C_2H_5$ | Cl | H | 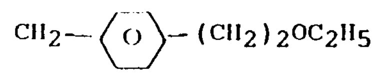 | |
| 166 | H | $-(CH_2)_4-$ | | H | 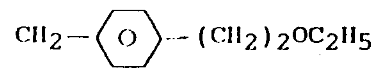 | |
| 167 | H | $-(CH_2)_4-$ | | H | 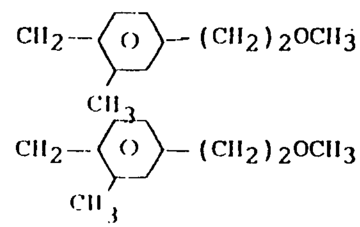 | |
| 168 | H | $CH_2OCH_3$ | $OCH_3$ | H | | |
| 169 | H | $C_2H_5$ | Cl | H | 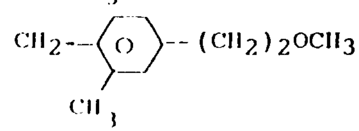 | |

Tabelle I

| Beisp. Nr. | $R^1$ | $R^2$ | $R^3$ | $R^4$ | Q | Fp [°C] |
|---|---|---|---|---|---|---|
| 170 | H | $C_2H_5$ | Cl | H | 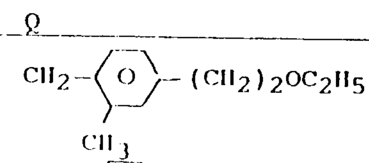 | |
| 171 | H | $CH_2OCH_3$ | $OCH_3$ | H | 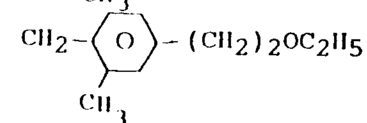 | |
| 172 | H | $-(CH_2)_4$ | | H | 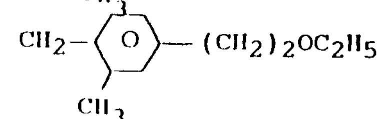 | |
| 173 | H | $-(CH_2)_4$ | | H | 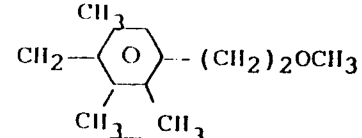 | |
| 174 | H | $C_2H_5$ | Cl | H | 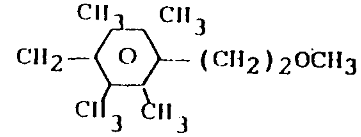 | |
| 175 | H | $CH_2OCH_3$ | $OCH_3$ | H | 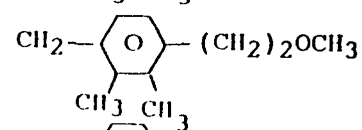 | |
| 176 | H | $CH_2OCH_3$ | $OCH_3$ | H | 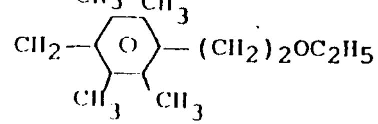 | |

Tabelle I

| Beisp. Nr. | $R^1$ | $R^2$ | $R^3$ | $R^4$ | Q | Fp [°C] |
|---|---|---|---|---|---|---|
| 177 | H | $C_2H_5$ | Cl | H | 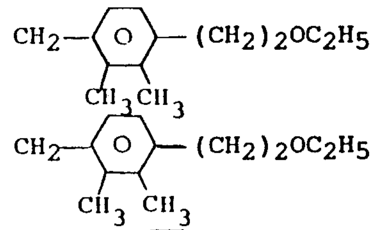 | |
| 178 | H | $-(CH_2)_4-$ | | H | | |
| 179 | H | $-(CH_2)_4-$ | | H | 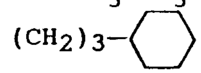 | |
| 180 | H | $CH_2OCH_3$ | $OCH_3$ | H | 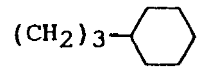 | |
| 181 | H | $C_2H_5$ | Cl | H | 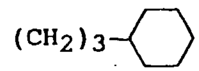 | |
| 182 | H | $C_2H_5$ | Cl | H | 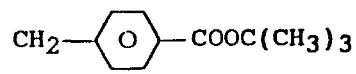 | |
| 183 | H | $CH_2OCH_3$ | $OCH_3$ | H | 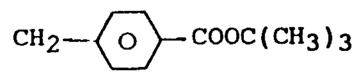 | |
| 184 | H | $-(CH_2)_4$ | | H | 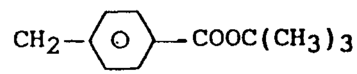 | |
| 185 | H | $-(CH_2)_4$ | | H | 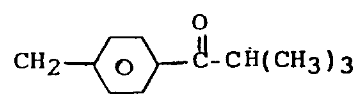 | |

Tabelle I

| Beisp. Nr. | $R^1$ | $R^2$ | $R^3$ | $R^4$ | Q | Fp [°C] |
|---|---|---|---|---|---|---|
| 186 | H | $CH_2OCH_3$ | $OCH_3$ | H | 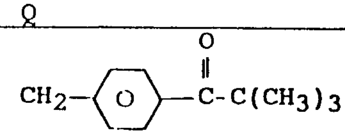 | |
| 187 | H | $C_2H_5$ | $OCH_3$ | H | 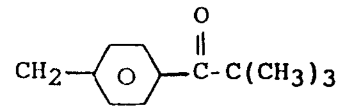 | |
| 188 | H | $C_2H_5$ | $OCH_3$ | H | 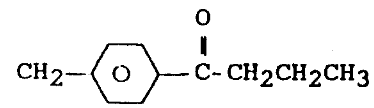 | |
| 189 | H | $CH_2OCH_3$ | $OCH_3$ | H | 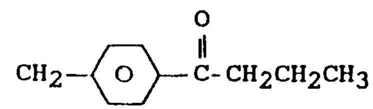 | |
| 190 | H | $(CH_2)_4$ | | H | 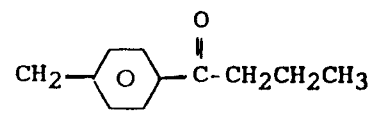 | |
| 191 | H | $(CH_2)_4$ | | H | 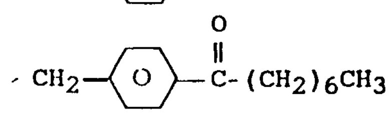 | |
| 192 | H | $CH_2OCH_3$ | $OCH_3$ | H | 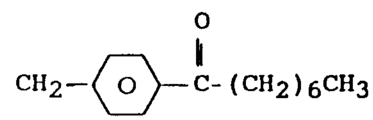 | |
| 193 | H | $C_2H_5$ | $OCH_3$ | H | 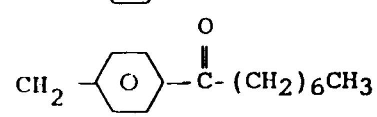 | |

Tabelle I

| Beisp. Nr. | $R^1$ | $R^2$ | $R^3$ | $R^4$ | Q | Fp [°C] |
|---|---|---|---|---|---|---|
| 194 | H | $-(CH_2)_3-S$ | | H | 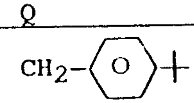 | 74 |
| 195 | H | $-(CH_2)_3-S$ | | H | 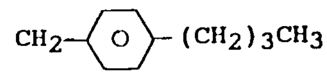 | |
| 196 | H | $-(CH_2)_3-S$ | | H | 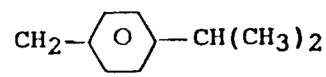 | |
| 197 | H | $-(CH_2)_3-S$ | | $C_2H_5$ | 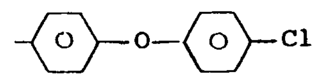 | |
| 198 | H | $-(CH_2)_3-S$ | | H | 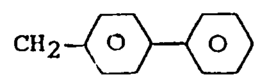 | |
| 199 | H | $-(CH_2)_4$ | | H | 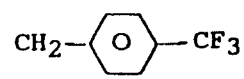 | 55 |

| Bei-spiel Nr. | $R^1$ | $R^2$ | $R^3$ | $R^4$ | Q | Fp. |
|---|---|---|---|---|---|---|
| 200 | H | Cl | $OCH_3$ | $CH_3$ | $(CH_2)_3CH_3$ | Öl |
| 201 | H | Cl | $OCH_3$ | $C_2H_5$ | $(CH_2)_6CH_3$ | Öl |
| 202 | H | $CH_2OCH_3$ | $OCH_3$ | H | $CH_2O(CH_2)_2OCH_3$ | Öl |
| 203 | H | $O(CH_2)_2O(CH_2)_7CH3$ | $OCH_3$ | H | $CH_2O(CH_2)_2OCH_3$ | Öl |
| 204 | H | $CH_2OCH_3$ | $OCH_3$ | $CH_3$ | $(CH_2)_2CH=C(CH_3)CH_2CH_2CH=C(CH_2)_2$ | Öl |
| 205 | H | $O(CH_2)O(CH_2)_9CH_3$ | $OCH_3$ | H | $CH_2O(CH_2)_9CH_3$ | 32°C |
| 206 | H | $-(CH_2)_4-$ | | H | $CH_2O_2C(CH_2)_2CH_3$ | Öl |
| 207 | H | $-(CH_2)_4-$ | | H | $CH_2O_2C(CH_2)_3CH_3$ | Öl |
| 208 | H | Cl | $OCH_3$ | $C_2H_5$ | $(CH_2)_6CH_3$ | Öl |
| 209 | H | $C_2H_5$ | Cl | $CH_3$ | $(CH_2)_7CH_3$ | Öl |
| 210 | H | $CH_2OCH_3$ | $OCH_3$ | H | $C(CH_3)_2CH_2OCH_2$– 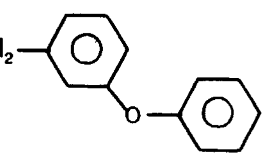 | Öl |

| Beispiel Nr. | $R^1$ | $R^2$ | | $R^3$ | $R^4$ | Q | Fp. |
|---|---|---|---|---|---|---|---|
| 211 | H | | $-(CH_2)_4-$ | | H | 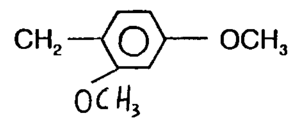 | 77-80°C |
| 212 | H | | $-(CH_2)_4-$ | | H | 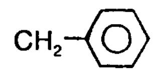 | Öl |
| 213 | H | | $-(CH_2)_5-$ | | H | 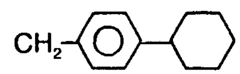 | Öl |
| 214 | H | Cl | | $OCH_3$ | H | 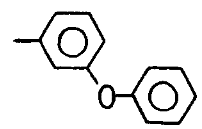 | Öl |
| 215 | H | 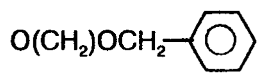 | | $OCH_3$ | H | 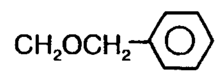 | Öl |
| 216 | H | $C_2H_5$ | | Cl | H | 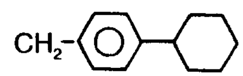 | Öl |
| 217 | H | $CH_2OCH_3$ | | $OCH_3$ | H | 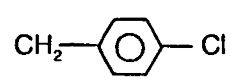 | Öl |

| Beispiel Nr. | $R^1$ | $R^2$ | $R^3$ | $R^4$ | Q | Fp. |
|---|---|---|---|---|---|---|
| 218 | H | $C_2H_5$ | Cl | H | $CH_2$- 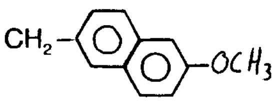 | 68°C |
| 219 | H | $OCH_2CH_2$- 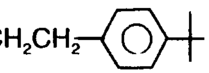 | $OCH_3$ | H | $CH_2$- 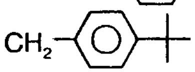 | 69-73°C |
| 220 | H | $-(CH_2)_3-$ | | H | $CH_2$- 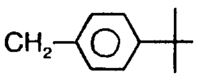 | Öl |
| 221 | H | $-(CH_2)_5-$ | | H | $CH_2$- 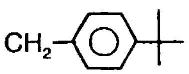 | Öl |
| 222 | H | $-(CH_2)_4-$ | | H | $CH_2$- 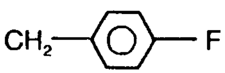 | 50°C |
| 223 | H | $-(CH_2)_4-$ | | H | $CH_2$- 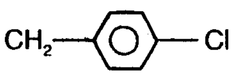 | 49°C |
| 224 | H | $-(CH_2)_4-$ | | H | $CH_2$- 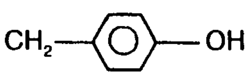 | Öl |
| 225 | H | $-(CH_2)_4-$ | | H | $CH_2$- 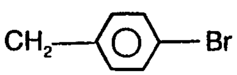 | Öl |
| 226 | H | $-(CH_2)_4-$ | | $CH_3$ | $CH_2$- 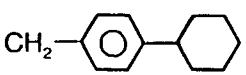 | 82°C |

| Beispiel Nr. | $R^1$ | $R^2$ | $R^3$ | $R^4$ | Q | Fp. |
|---|---|---|---|---|---|---|
| 227 | H | $CH_2OCH_3$ | $OCH_3$ | $CH_3$ | $CH_2$- 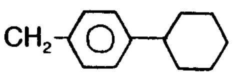 | Öl |
| 228 | H | $-(CH_2)_4-$ | | H | $CH_2$ 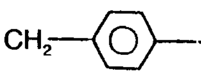 $CH(CH_3)CH_2CH_3$ | Öl |
| 229 | H | $CH_2OCH_3$ | $OCH_3$ | H | $CH_2$ 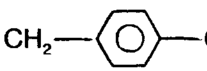 $CH(CH_3)CH_2CH_3$ | Öl |
| 230 | H | $-(CH_2)_4-$ | | $CH_3$ | 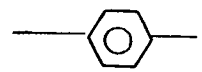 $CH_2CH_3$ | Öl |
| 231 | H | $CH_2OCH_3$ | $OCH_3$ | $CH_3$ | 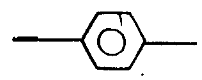 $CH_2CH_3$ | Öl |
| 232 | H | $C_2H_5$ | Cl | $CH_3$ | 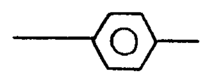 $CH_2CH_3$ | Öl |
| 233 | H | $-(CH)_4-$ | | $CH_3$ | 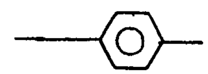 $CH_2CH_3$ | Öl |
| 234 | H | $-(CH_2)_4-$ | | $CH_3$ | 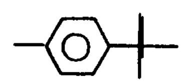 | 92°C |
| 235 | H | $CH_2OCH_3$ | $OCH_3$ | $CH_3$ | 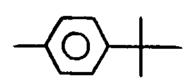 | Öl |

| Beispiel Nr. | $R^1$ | $R^2$ | $R^3$ | $R^4$ | Q | Fp. |
|---|---|---|---|---|---|---|
| 236 | H | $-(CH_2)_4-$ | | $CH_3$ | 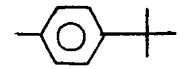 | Öl |
| 237 | H | $C_2H_5$ | Cl | $CH_3$ | 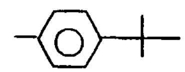 | Öl |
| 238 | H | $-(CH_2)_4-$ | | $CH_3$ | 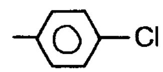 | Öl |
| 239 | H | $CH_2OCH_3$ | $OCH_3$ | $CH_3$ | 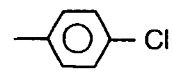 | Öl |
| 240 | H | $-(CH)_4-$ | | $CH_3$ | 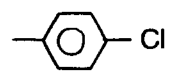 | Öl |
| 241 | H | $-C_2H_5$ | Cl | $CH_3$ | 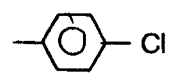 | Öl |
| 242 | H | $-(CH_2)_4-$ | | $CH_3$ | 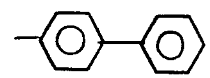 | Öl |
| 243 | H | $CH_2OCH_3$ | $OCH_3$ | $CH_3$ | 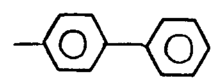 | Öl |
| 244 | H | $-(CH)_4-$ | | $CH_3$ | 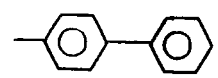 | Öl |

| Beispiel Nr. | $R^1$ | $R^2$ | $R^3$ | $R^4$ | Q | Fp. |
|---|---|---|---|---|---|---|
| 245 | H | $C_2H_5$ | Cl | $CH_3$ | 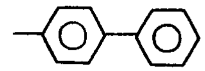 | Öl |
| 246 | H | $C_2H_5$ | Cl | H | 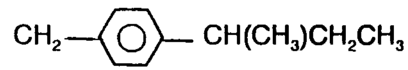 | Öl |
| 247 | H | $C_2H_5$ | Cl | H | 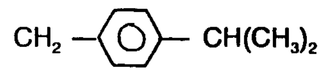 | Öl |
| 248 | H | $C_2H_5$ | Cl | H | 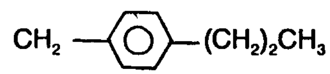 | Öl |
| 249 | H | $C_2H_5$ | Cl | H | 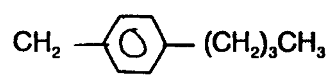 | Öl |
| 250 | H | $C_2H_5$ | Cl | H | 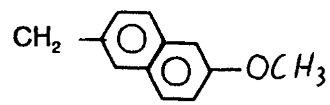 | 68°C |
| 251 | H | $C_2H_5$ | Cl | $C_2H_5$ | 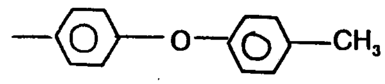 | Öl |
| 252 | H | $-(CH_2)_4-$ | | $CH_3$ | 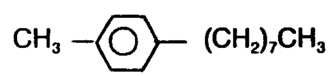 | Öl |

| Beispiel Nr. | $R^1$ | $R^2$ | $R^3$ | $R^4$ | Q | Fp. |
|---|---|---|---|---|---|---|
| 253 | H | $CH_2OCH_3$ | $OCH_3$ | $CH_3$ | 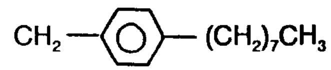 | Öl |
| 254 | H | $C_2H_5$ | Cl | $CH_3$ | 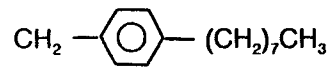 | |
| 255 | H | $-(CH_2)_4-$ | | H | 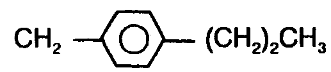 | Öl |
| 256 | H | $CH_2OCH_3$ | $OCH_3$ | H | 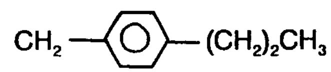 | Öl |
| 257 | H | $C_2H_5$ | Cl | H | 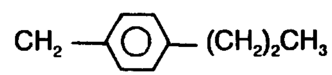 | |
| 258 | H | $-(CH_2)_4-$ | | H | 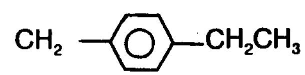 | Öl |
| 259 | H | $-(CH_2)_4-$ | | H | 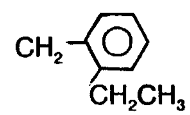 | Öl |
| 260 | H | $CH_2OCH_3$ | $OCH_3$ | H | 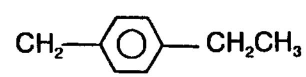 | Öl |

| Beispiel Nr. | $R^1$ | $R^2$ | $R^3$ | $R^4$ | Q | Fp. |
|---|---|---|---|---|---|---|
| 261 | H | $CH_2OCH_3$ | $OCH_3$ | H | $CH_2$ 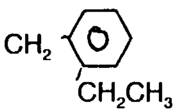 | Öl |
| 262 | H | $-(CH_2)_4-$ | | H | $CH(CH_3)CH_2-$ 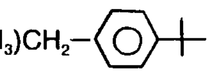 | Öl |
| 263 | H | $CH_2OCH_3$ | $OCH_3$ | H | $CH(CH_3)CH_2-$ 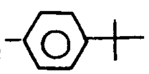 | Öl |
| 264 | H | $-(CH_2)-$ | | H | $CH(CH_3)CH_2-$ 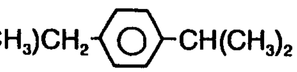 | Öl |
| 265 | H | $CH_2OCH_3$ | $OCH_3$ | H | $CH(CH_3)CH_2-$ 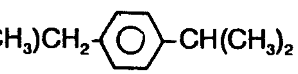 | Öl |
| 266 | H | $-(CH_2)_4-$ | | H | $CH_2$ 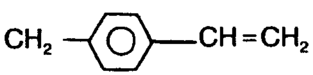 | Öl |
| 267 | H | $CH_2OCH_3$ | $OCH_3$ | H | $CH_2$ 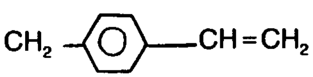 | Öl |
| 268 | H | $C_2H_5$ | Cl | H | $CH(CH_3)CH_2-$ 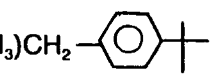 | Öl |

| Beispiel Nr. | $R^1$ | $R^2$ | $R^3$ | $R^4$ | Q | Fp. |
|---|---|---|---|---|---|---|
| 269 | H | $-(CH_2)_4-$ | | H | 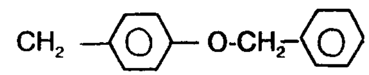 | Öl |
| 270 | H | $CH_2OCH_3$ | $OCH_3$ | H | 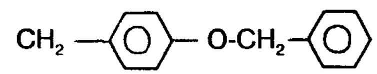 | Öl |
| 271 | H | $C_2H_5$ | Cl | H | 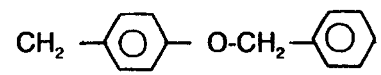 | Öl |
| 272 | H | $-(CH_2)_4-$ | | H | 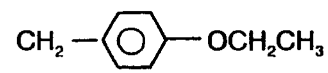 | |
| 273 | H | $CH_2OCH_3$ | $OCH_3$ | H | 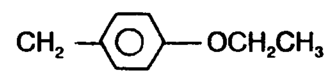 | |
| 274 | H | $C_2H_5$ | Cl | H | 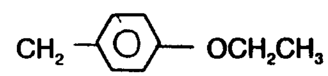 | |
| 275 | H | $-(CH_2)_4-$ | | H | 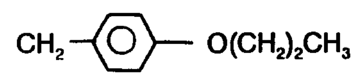 | Öl |
| 276 | H | $CH_2OCH_3$ | $OCH_3$ | H | 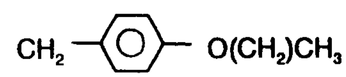 | Öl |
| 277 | H | $C_2H_5$ | Cl | H | 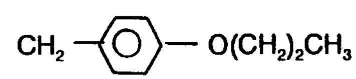 | Öl |

| Beispiel Nr. | $R^1$ | $R^2$ | $R^3$ | $R^4$ | Q | Fp. |
|---|---|---|---|---|---|---|
| 278 | H | $-(CH_2)_4-$ | | H | $CH_2$ 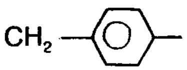 $O(CH_2)_3CH_3$ | |
| 279 | H | $CH_2OCH_3$ | $OCH_3$ | H | $CH_2$ 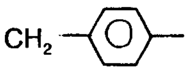 $O(CH_2)_3CH_3$ | |
| 280 | H | $C_2H_5$ | Cl | H | $CH_2$ 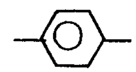 $O(CH_2)_3CH_3$ | |
| 281 | H | $-(CH_2)_4-$ | | H | $CH_2$ 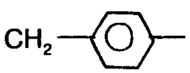 $O(CH_2)_7CH_3$ | |
| 282 | H | $CH_2OCH_3$ | $OCH_3$ | H | $CH_2$ 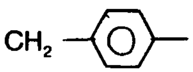 $O(CH_2)_7CH_3$ | |
| 283 | H | $C_2H_5$ | Cl | H | $CH_2$ 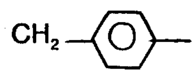 $O(CH_2)_7CH_3$ | |
| 284 | H | $-(CH_2)_4-$ | | H | $CH_2$ 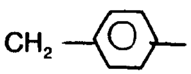 $OCH(CH_3)_2$ | Öl |
| 285 | H | $CH_2OCH_3$ | $OCH_3$ | H | $CH_2$ 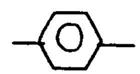 $OCH(CH_3)_2$ | Öl |
| 286 | H | $C_2H_5$ | Cl | H | $CH_2$ 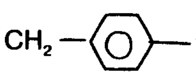 $OCH(CH_3)_2$ | |

| Bei-spiel Nr. | $R^1$ | $R^2$ | $R^3$ | $R^4$ | Q | Fp. |
|---|---|---|---|---|---|---|
| 287 | H | $-(CH_2)_4-$ | | H | $CH_2$ 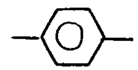 $OC(CH_3)_3$ | |
| 288 | H | $CH_2OCH_3$ | $OCH_3$ | H | $CH_2$ 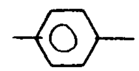 $OC(CH_3)_3$ | |
| 289 | H | $C_2H_5$ | Cl | H | $CH_2$ 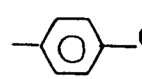 $OC(CH_3)_3$ | |
| 290 | H | $-(CH_2)_4-$ | | H | $CH_2$ 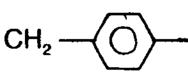 $OCH_2CHCH_2$ | Öl |
| 291 | H | $CH_2CH_3$ | $OCH_3$ | H | $CH_2$ 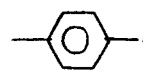 $OCH_2CHCH_2$ | |
| 292 | H | $C_2H_5$ | Cl | H | $CH_2$ 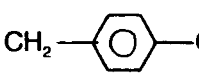 $OCH_2CHCH_2$ | |
| 293 | H | $-(CH_2)_4-$ | | H | $CH_2$ 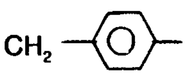 $O-Si(CH_3)_2C(CH_3)_3$ | Öl |
| 294 | H | $CH_2OCH_3$ | $OCH_3$ | H | $CH_2$ 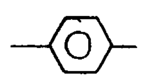 $O-Si(CH_3)_2C(CH_3)_3$ | |
| 295 | H | $C_2H_5$ | Cl | H | $CH_2$ 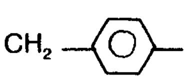 $O-Si(CH_3)_2C(CH_3)_3$ | |

| Bei-spiel Nr. | $R^1$ | $R^2$ | $R^3$ | $R^4$ | Q | Fp. |
|---|---|---|---|---|---|---|
| 296 | H | $-(CH_2)_4-$ | | H | $CH_2$ 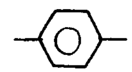 $Si(CH_3)_2CH_2Cl$ | Öl |
| 297 | H | $CH_2OCH_3$ | $OCH_3$ | H | $CH_2$ 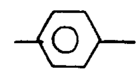 $Si(CH_3)_2CH_2Cl$ | |
| 298 | H | $C_2H_5$ | Cl | H | $CH_2$ 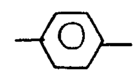 $Si(CH_3)_2CH_2Cl$ | |
| 299 | H | $-(CH_2)_4-$ | | H | $CH_2$ 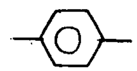 $Si(CH_3)_3$ | Öl |
| 300 | H | $CH_2OCH_3$ | $OCH_3$ | H | $CH_2$ 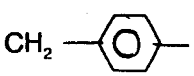 $Si(CH_3)_3$ | Öl |
| 301 | H | $C_2H_5$ | Cl | H | $CH_2$ 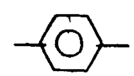 $Si(CH_3)_3$ | Öl |
| 302 | H | $-(CH_2)_4-$ | | H | $CH_2$ 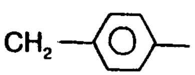 $Si(CH_3)_2(CH_2)_7CH_3$ | Öl |
| 303 | H | $CH_2OCH_3$ | $OCH_3$ | H | $CH_2$ 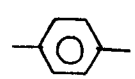 $Si(CH_3)_2(CH_2)_7CH_3$ | Öl |
| 304 | H | $C_2H_5$ | Cl | H | $CH_2$ 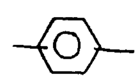 $Si(CH_3)_2(CH_2)_7CH_3$ | |

| Beispiel Nr. | $R^1$ | $R^2$ | $R^3$ | $R^4$ | Q | Fp. |
|---|---|---|---|---|---|---|
| 305 | H | $-(CH_2)_4-$ | | H | 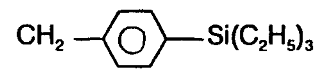 | Öl |
| 306 | H | $CH_2OCH_3$ | $OCH_3$ | H | 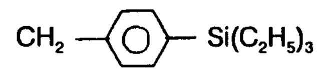 | Öl |
| 307 | H | $C_2H_5$ | Cl | H | 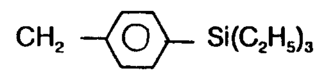 | |
| 308 | H | $-(CH_2)_4-$ | | H | 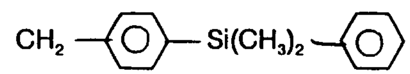 | |
| 309 | H | $CH_2OCH_3$ | $OCH_3$ | H | 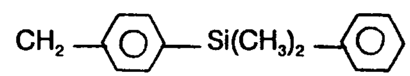 | |
| 310 | H | $C_2H_5$ | Cl | H | 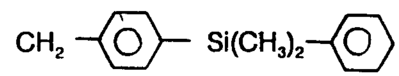 | |
| 311 | H | $-(CH_2)_4-$ | | H | 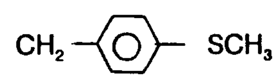 | öl |
| 312 | H | $CH_2OCH_3$ | $OCH_3$ | H | 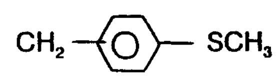 | öl |
| 313 | H | $C_2H_5$ | Cl | H | 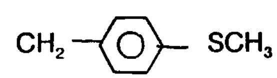 | öl |

| Beispiel Nr. | $R^1$ | $R^2$ | $R^3$ | $R^4$ | Q | Fp. |
|---|---|---|---|---|---|---|
| 314 | H | $-(CH_2)_4-$ | | H | $CH_2$ 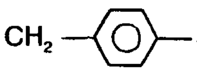 $SC_2H_5$ | |
| 315 | H | $CH_2OCH_3$ | $OCH_3$ | H | $CH_2$ 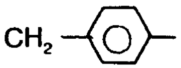 $SC_2H_5$ | |
| 316 | H | $C_2H_5$ | Cl | H | $CH_2$ 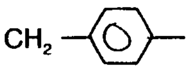 $SC_2H_5$ | |
| 317 | H | $-(CH_2)_4-$ | | H | $CH_2$ 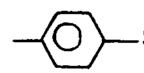 $S(CH_2)_2CH_3$ | |
| 318 | H | $CH_2OCH_3$ | $OCH_3$ | H | $CH_2$ 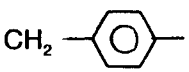 $S(CH_2)_2CH_3$ | |
| 319 | H | $C_2H_5$ | Cl | H | $CH_2$ 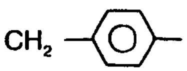 $S(CH_2)_2CH_3$ | |
| 320 | H | $-(CH_2)_4-$ | | H | $CH_2$ 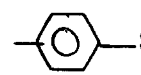 $S(CH_2)_3CH_3$ | Öl |
| 321 | H | $CH_2OCH_3$ | $OCH_3$ | H | $CH_2$ 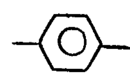 $S(CH_2)_3CH_3$ | Öl |
| 322 | H | $C_2H_5$ | Cl | H | $CH_2$ 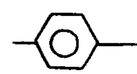 $S(CH_2)_3CH_3$ | Öl |

| Beispiel Nr. | $R^1$ | $R^2$ | $R^3$ | $R^4$ | Q | Fp. |
|---|---|---|---|---|---|---|
| 323 | H | $-(CH_2)_4-$ | | H | $CH_2$ 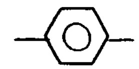 $S(CH_2)_4CH_3$ | |
| 324 | H | $CH_2OCH_3$ | $OCH_3$ | H | $CH_2$ 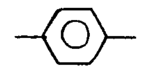 $S(CH_2)_4CH_3$ | |
| 325 | H | $C_2H_5$ | Cl | H | $CH_2$ 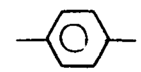 $S(CH_2)_4CH_3$ | |
| 326 | H | $-(CH_2)_4-$ | | H | $CH_2$ 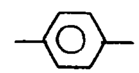 $S(CH_2)_5CH_3$ | |
| 327 | H | $CH_2OCH_3$ | $OCH_3$ | H | $CH_2$ 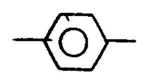 $S(CH_2)_5CH_3$ | |
| 328 | H | $C_2H_5$ | Cl | H | $CH_2$ 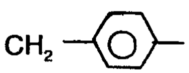 $S(CH_2)_5CH_3$ | |
| 329 | H | $-(CH_2)_4-$ | | H | $CH_2$ 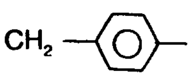 $S(CH_2)_6CH_3$ | |
| 330 | H | $CH_2OCH_3$ | $OCH_3$ | H | $CH_2$ 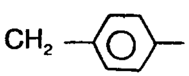 $S(CH_2)_6CH_3$ | |
| 331 | H | $C_2H_5$ | Cl | H | $CH_2$ 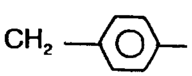 $S(CH_2)_6CH_3$ | |

| Beispiel Nr. | $R^1$ | $R^2$ | $R^3$ | $R^4$ | Q | Fp. |
|---|---|---|---|---|---|---|
| 332 | H | $-(CH_2)_4-$ | | H | 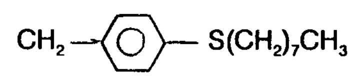 | |
| 333 | H | $CH_2OCH_3$ | $OCH_3$ | H | 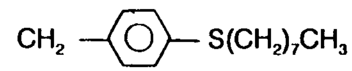 | |
| 334 | H | $C_2H_5$ | Cl | H | 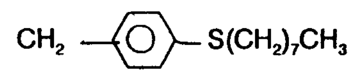 | |
| 335 | H | $-(CH_2)_4-$ | | H | 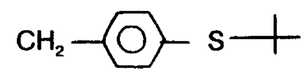 | |
| 336 | H | $CH_2OCH_3$ | $OCH_3$ | H | 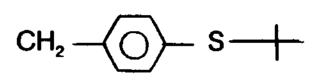 | |
| 337 | H | $C_2H_5$ | Cl | H | 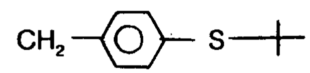 | |
| 338 | H | $-(CH_2)_4-$ | | H | 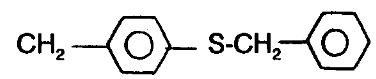 | |
| 339 | H | $CH_2OCH_3$ | $OCH_3$ | H | 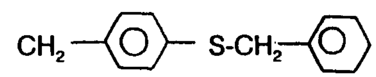 | |
| 340 | H | $C_2H_5$ | Cl | H | 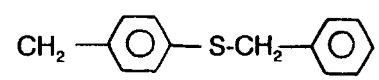 | |

| Beispiel Nr. | $R^1$ | $R^2$ | $R^3$ | $R^4$ | Q | Fp. |
|---|---|---|---|---|---|---|
| 341 | H | $-(CH_2)_4-$ | | H | 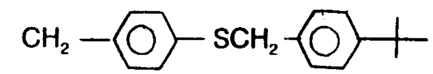 | |
| 342 | H | $CH_2OCH_3$ | $OCH_3$ | H | 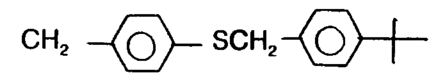 | |
| 343 | H | $C_2H_5$ | Cl | H | 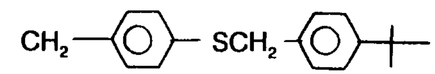 | |
| 344 | H | $-(CH_2)_4-$ | | H | 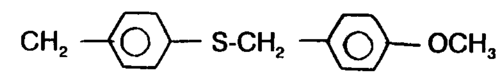 | |
| 345 | H | $CH_2OCH_3$ | $OCH_3$ | H | 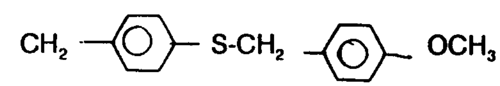 | |
| 346 | H | $C_2H_5$ | Cl | H | 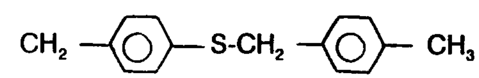 | |
| 347 | H | $-(CH_2)_4-$ | | H | 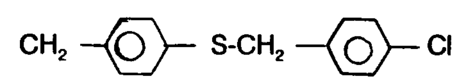 | |
| 348 | H | $CH_2OCH_3$ | $OCH_3$ | H | 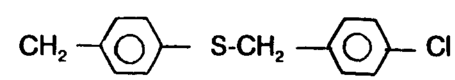 | |
| 349 | H | $C_2H_5$ | Cl | H | 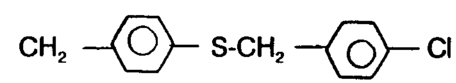 | |

| Bei-spiel Nr. | $R^1$ | $R^2$ | $R^3$ | $R^4$ | Q | Fp. |
|---|---|---|---|---|---|---|
| 350 | H | $-(CH_2)_4-$ | | H | $CH_2$ 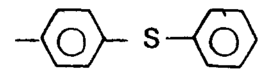 | |
| 351 | H | $CH_2OCH_3$ | $OCH_3$ | H | $CH_2$ 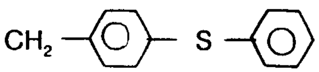 | |
| 352 | H | $C_2H_5$ | Cl | H | $CH_2$ 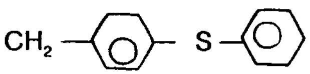 | |
| 353 | H | $-(CH_2)_4-$ | | H | $CH_2$ 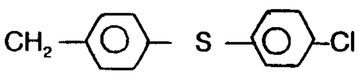 | |
| 354 | H | $CH_2OCH_3$ | $OCH_3$ | H | $CH_2$ 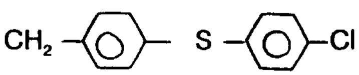 | |
| 355 | H | $C_2H_5$ | Cl | H | $CH_2$ 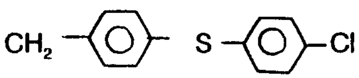 | |
| 356 | H | $-(CH_2)_4-$ | | H | $CH_2$ 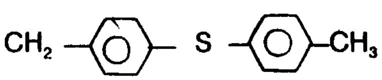 | |
| 357 | H | $CH_2OCH_3$ | $OCH_3$ | H | $CH_2$ 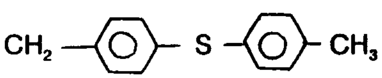 | |
| 358 | H | $C_2H_5$ | Cl | H | $CH_2$ 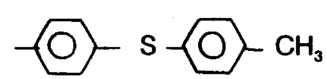 | |

| Beispiel Nr. | $R^1$ | $R^2$ | $R^3$ | $R^4$ | Q | Fp. |
|---|---|---|---|---|---|---|
| 359 | H | $-(CH_2)_4-$ | | H | 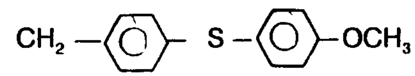 | |
| 360 | H | $CH_2OCH_3$ | $OCH_3$ | H | 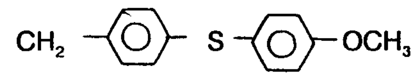 | |
| 361 | H | $C_2H_5$ | Cl | H | 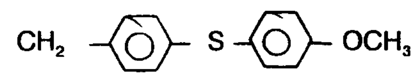 | |
| 362 | H | $-(CH_2)_4-$ | | H | 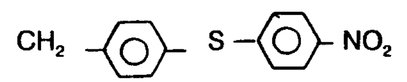 | |
| 363 | H | $CH_2OCH_3$ | $OCH_3$ | H | 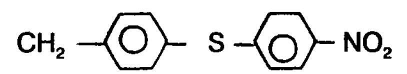 | |
| 364 | H | $C_2H_5$ | Cl | H | 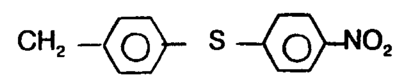 | |
| 365 | H | $-(CH_2)_4-$ | | $CH_3$ | 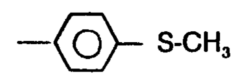 | |
| 366 | H | $CH_2OCH_3$ | $OCH_3$ | $CH_3$ | 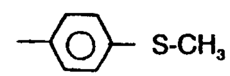 | |
| 367 | H | $C_2H_5$ | Cl | $CH_3$ | 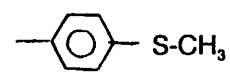 | |

| Beispiel Nr. | $R^1$ | $R^2$ | $R^3$ | $R^4$ | Q | Fp. |
|---|---|---|---|---|---|---|
| 368 | H | $-(CH_2)_4-$ | | $CH_3$ | 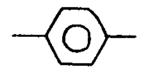 $S-CH_2CH_3$ | |
| 369 | H | $CH_2OCH_3$ | $OCH_3$ | $CH_3$ | 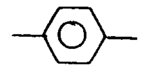 $S-CH_2CH_3$ | |
| 370 | H | $C_2H_5$ | Cl | $CH_3$ | 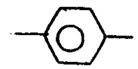 $S-CH_2CH_3$ | |
| 371 | H | $-(CH_2)_4-$ | | $CH_3$ | 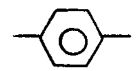 $S(CH_2)_2CH_3$ | |
| 372 | H | $CH_2OCH_3$ | $OCH_3$ | $CH_3$ | 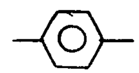 $S(CH_2)_2CH_3$ | |
| 373 | H | $C_2H_5$ | Cl | $CH_3$ | 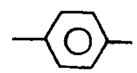 $S(CH_2)_2CH_3$ | |
| 374 | H | $-(CH_2)_4-$ | | $CH_3$ | 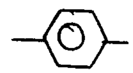 $S(CH_2)_3CH_3$ | |
| 375 | H | $CH_2OCH_3$ | $OCH_3$ | $CH_3$ | 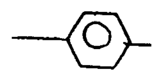 $S(CH_2)_3CH_3$ | |
| 376 | H | $C_2H_5$ | Cl | $CH_3$ | 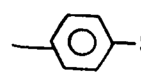 $S(CH_2)_3CH_3$ | |

| Beispiel Nr. | $R^1$ | $R^2$ | $R^3$ | $R^4$ | Q | Fp. |
|---|---|---|---|---|---|---|
| 377 | H | $-(CH_2)_4-$ | | $CH_3$ | 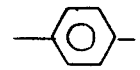 $S(CH_2)_4CH_3$ | |
| 378 | H | $CH_2OCH_3$ | $OCH_3$ | $CH_3$ | 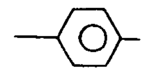 $S(CH_2)_4CH_3$ | |
| 379 | H | $C_2H_5$ | Cl | $CH_3$ | 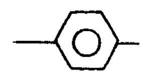 $S(CH_2)_4CH_3$ | |
| 380 | H | $-(CH_2)_4-$ | | $CH_3$ | 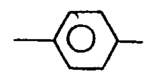 $S(CH_2)_5CH_3$ | |
| 381 | H | $CH_2OCH_3$ | $OCH_3$ | $CH_3$ | 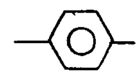 $S(CH_2)_5CH_3$ | |
| 382 | H | $C_2H_5$ | Cl | $CH_3$ | 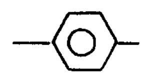 $S(CH_2)_5CH_3$ | |
| 383 | H | $-(CH_2)_4-$ | | $CH_3$ | 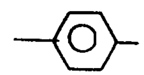 $S(CH_2)_6CH_3$ | |
| 384 | H | $CH_2OCH_3$ | $OCH_3$ | $CH_3$ | 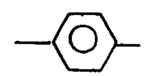 $S(CH_2)_6CH_3$ | |
| 385 | H | $C_2H_5$ | Cl | $CH_3$ | 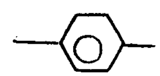 $S(CH_2)_6CH_3$ | |

| Beispiel Nr. | $R^1$ | $R^2$ | $R^3$ | $R^4$ | Q | Fp. |
|---|---|---|---|---|---|---|
| 386 | H | $-(CH_2)_4-$ | | $CH_3$ | 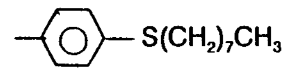 | |
| 387 | H | $CH_2OCH_3$ | $OCH_3$ | $CH_3$ | 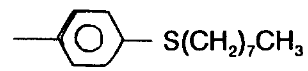 | |
| 388 | H | $C_2H_5$ | Cl | $CH_3$ | 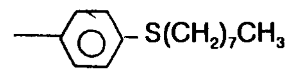 | |
| 389 | H | $-(CH_2)_4-$ | | $CH_3$ | 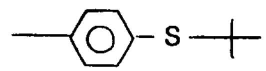 | |
| 390 | H | $CH_2OCH_3$ | $OCH_3$ | $CH_3$ | 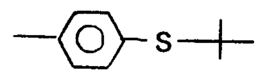 | |
| 391 | H | $C_2H_5$ | Cl | $CH_3$ | 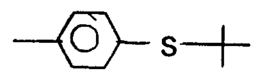 | |
| 392 | H | $-(CH_2)_4-$ | | $CH_3$ | 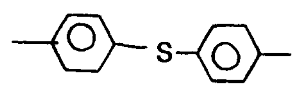 | |
| 393 | H | $CH_2OCH_3$ | $OCH_3$ | $CH_3$ | 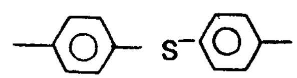 | |
| 394 | H | $C_2H_5$ | Cl | $CH_3$ | 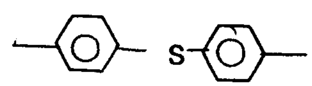 | |

| Beispiel Nr. | $R^1$ | $R^2$ | $R^3$ | $R^4$ | Q | Fp. |
|---|---|---|---|---|---|---|
| 395 | H | $-(CH_2)_4-$ | | $CH_3$ | 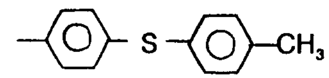 | |
| 396 | H | $CH_2OCH_3$ | $OCH_3$ | $CH_3$ | 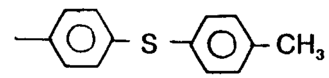 | |
| 397 | H | $C_2H_5$ | Cl | $CH_3$ | 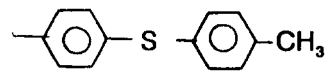 | |
| 398 | H | $-(CH_2)_4-$ | | $CH_3$ | 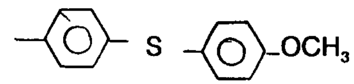 | |
| 399 | H | $CH_2OCH_3$ | $OCH_3$ | $CH_3$ | 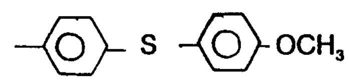 | |
| 400 | H | $C_2H_5$ | Cl | $CH_3$ | 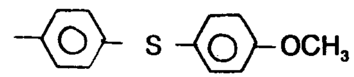 | |
| 401 | H | $-(CH_2)_4-$ | | $CH_3$ | 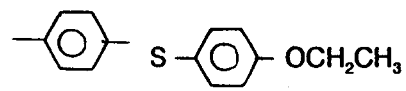 | |
| 402 | H | $CH_2OCH_3$ | $OCH_3$ | $CH_3$ | 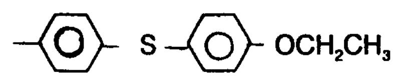 | |
| 403 | H | $C_2H_5$ | Cl | $CH_3$ | 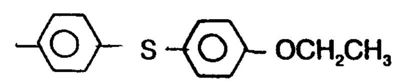 | |

| Beispiel Nr. | $R^1$ | $R^2$ | $R^3$ | $R^4$ | Q | Fp. |
|---|---|---|---|---|---|---|
| 404 | H | $-(CH_2)_4-$ | | $CH_3$ | 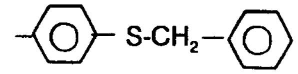 | |
| 405 | H | $CH_2OCH_3$ | $OCH_3$ | $CH_3$ | 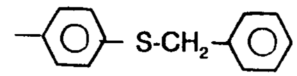 | |
| 406 | H | $C_2H_5$ | Cl | $CH_3$ | 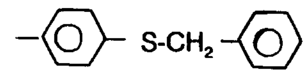 | |
| 407 | H | $-(CH_2)_4-$ | | $CH_3$ | 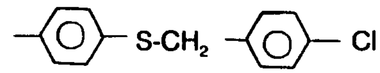 | |
| 408 | H | $CH_2OCH_3$ | $OCH_3$ | $CH_3$ | 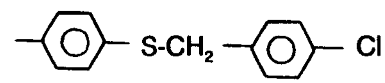 | |
| 409 | H | $C_2H_5$ | Cl | $CH_3$ | 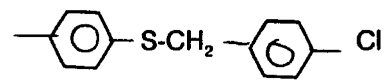 | |
| 410 | H | $-(CH_2)_4-$ | | $CH_3$ | 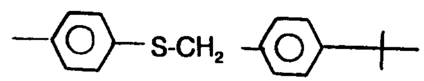 | |
| 411 | H | $CH_2OCH_3$ | $OCH_3$ | $CH_3$ | 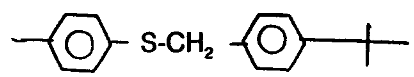 | |
| 412 | H | $C_2H_5$ | Cl | $CH_3$ | 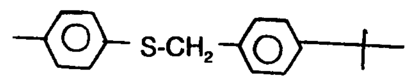 | |

| Beispiel Nr. | $R^1$ | $R^2$ | $R^3$ | $R^4$ | Q | Fp. |
|---|---|---|---|---|---|---|
| 413 | H | $-(CH_2)_4-$ | | H | $CH_2$ 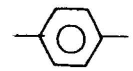 $SOCH_3$ | |
| 414 | H | $CH_2OCH_3$ | $OCH_3$ | H | $CH_2$ 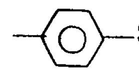 $SOCH_3$ | |
| 415 | H | $C_2H_5$ | Cl | H | $CH_2$ 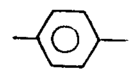 $SOCH_3$ | |
| 416 | H | $-(CH_2)_4-$ | | H | $CH_2$ 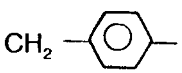 $SOC_2H_5$ | |
| 417 | H | $CH_2OCH_3$ | $OCH_3$ | H | $CH_2$ 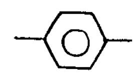 $SOC_2H_5$ | |
| 418 | H | $C_2H_5$ | Cl | H | $CH_2$ 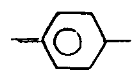 $SOC_2H_5$ | |
| 419 | H | $-(CH_2)_4-$ | | H | $-CH_2$ 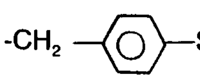 $SO(CH_2)_2CH_3$ | |
| 420 | H | $CH_2OCH_3$ | $OCH_3$ | H | $-CH_2$ 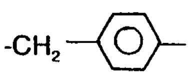 $SO(CH_2)_2CH_3$ | |
| 421 | H | $C_2H_5$ | Cl | H | $-CH_2$ 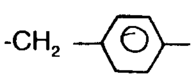 $SO(CH_2)_2CH_3$ | |

| Beispiel Nr. | $R^1$ | $R^2$ | $R^3$ | $R^4$ | Q | Fp. |
|---|---|---|---|---|---|---|
| 422 | H | $-(CH_2)_4-$ | | H | $CH_2$ 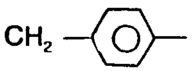 $SO(CH_2)_3CH_3$ | Öl |
| 423 | H | $CH_2OCH_3$ | $OCH_3$ | H | $CH_2$ 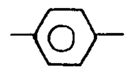 $SO(CH_2)_3CH_3$ | |
| 424 | H | $C_2H_5$ | Cl | H | $CH_2$ 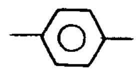 $SO(CH_2)_3CH_4$ | |
| 425 | H | $-(CH_2)_4-$ | | H | $CH_2$ 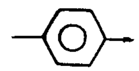 $SO(CH_2)_4CH_3$ | |
| 426 | H | $CH_2OCH_3$ | $OCH_3$ | H | $CH_2$ 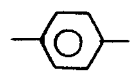 $SO(CH_2)_4CH_3$ | |
| 427 | H | $C_2H_5$ | Cl | H | $CH_2$ 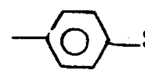 $SO(CH_2)_4CH_3$ | |
| 428 | H | $-(CH_2)_4-$ | | H | $CH_2$ 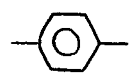 $SO(CH_2)_5CH_3$ | |
| 429 | H | $CH_2OCH_3$ | $OCH_3$ | H | $CH_2$ 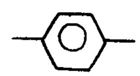 $SO(CH_2)_5CH_3$ | |
| 430 | H | $C_2H_5$ | Cl | H | $CH_2$ 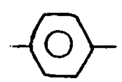 $SO(CH_2)_5CH_3$ | |

| Bei-spiel Nr. | $R^1$ | $R^2$ | $R^3$ | $R^4$ | Q | Fp. |
|---|---|---|---|---|---|---|
| 431 | H | $-(CH_2)_4-$ | | H | $CH_2$ 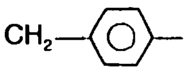 $SO(CH_2)_6CH_3$ | |
| 432 | H | $CH_2OCH_3$ | $OCH_3$ | H | $CH_2$ 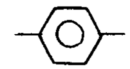 $SO(CH_2)_6CH_3$ | |
| 433 | H | $C_2H_5$ | Cl | H | $CH_2$ 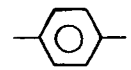 $SO(CH_2)_6CH_3$ | |
| 434 | H | $-(CH_2)_4-$ | | H | $CH_2$ 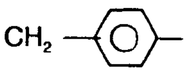 $SO(CH_2)_7CH_3$ | |
| 435 | H | $CH_2OCH_3$ | $OCH_3$ | H | $CH_2$ 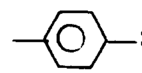 $SO(CH_2)_7CH_3$ | |
| 436 | H | $C_2H_5$ | Cl | H | $CH_2$ 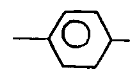 $SO(CH_2)_7CH_4$ | |
| 437 | H | $-(CH_2)_4-$ | | H | $CH_2$ 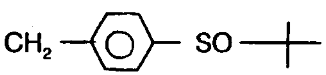 SO | |
| 438 | H | $CH_2OCH_3$ | $OCH_3$ | H | $CH_2$ 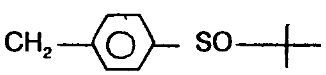 SO | |
| 439 | H | $C_2H_5$ | Cl | H | $CH_2$ 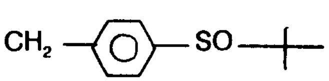 SO | |

| Beispiel Nr. | $R^1$ | $R^2$ | $R^3$ | $R^4$ | Q | Fp. |
|---|---|---|---|---|---|---|
| 440 | H | $-(CH_2)_4-$ | | H | 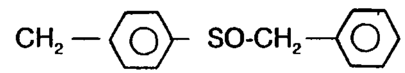 | |
| 441 | H | $CH_2OCH_3$ | $OCH_3$ | H | 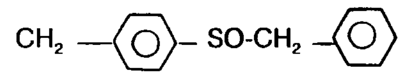 | |
| 442 | H | $C_2H_5$ | Cl | H | 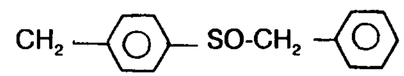 | |
| 443 | H | $-(CH_2)_4-$ | | H | 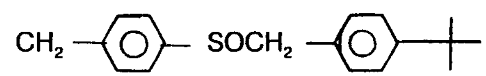 | |
| 444 | H | $CH_2OCH_3$ | $OCH_3$ | H | 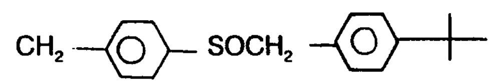 | |
| 445 | H | $C_2H_5$ | Cl | H | 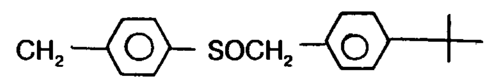 | |
| 446 | H | $-(CH_2)_4-$ | | H | 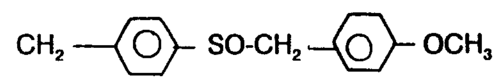 | |
| 447 | H | $CH_2OCH_3$ | $OCH_3$ | H | 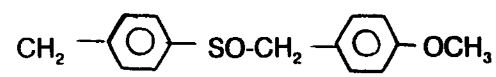 | |
| 448 | H | $C_2H_5$ | Cl | H | 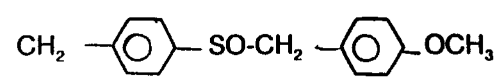 | |

| Beispiel Nr. | $R^1$ | $R^2$ | $R^3$ | $R^4$ | Q | Fp. |
|---|---|---|---|---|---|---|
| 449 | H | $-(CH_2)_4-$ | | H | 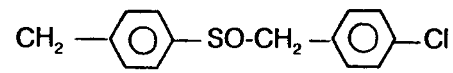 | |
| 450 | H | $CH_2OCH_3$ | $OCH_3$ | H | 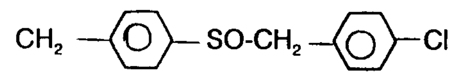 | |
| 451 | H | $C_2H_5$ | Cl | H | 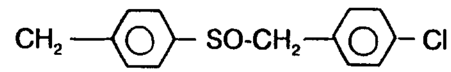 | |
| 452 | H | $-(CH_2)_4-$ | | H | 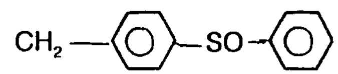 | |
| 453 | H | $CH_2OCH_3$ | $OCH_3$ | H | 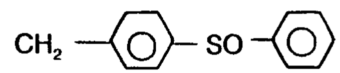 | |
| 454 | H | $C_2H_5$ | Cl | H | 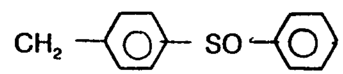 | |
| 455 | H | $-(CH_2)_4-$ | | H | 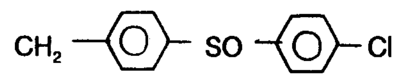 | |
| 456 | H | $CH_2OCH_3$ | $OCH_3$ | H | 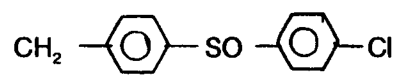 | |
| 457 | H | $C_2H_5$ | Cl | H | 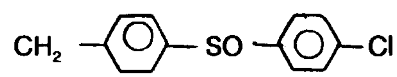 | |

| Bei-spiel Nr. | $R^1$ | $R^2$ | $R^3$ | $R^4$ | Q | Fp. |
|---|---|---|---|---|---|---|
| 458 | H | $-(CH_2)_4-$ | | H | 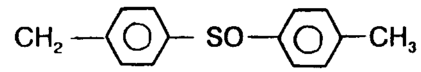 | |
| 459 | H | $CH_2OCH_3$ | $OCH_3$ | H | 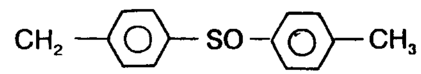 | |
| 460 | H | $C_2H_5$ | Cl | H | 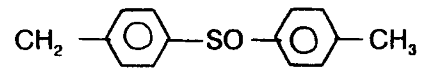 | |
| 461 | H | $-(CH_2)_4-$ | | H | 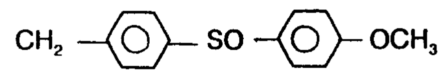 | |
| 462 | H | $CH_2OCH_3$ | $OCH_3$ | H | 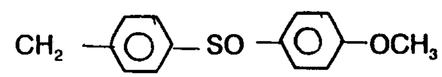 | |
| 463 | H | $C_2H_5$ | Cl | H | 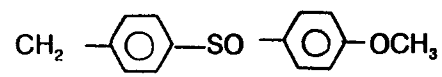 | |
| 464 | H | $-(CH_2)_4-$ | | H | 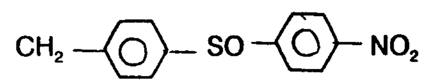 | |
| 465 | H | $CH_2OCH_3$ | $OCH_3$ | H | 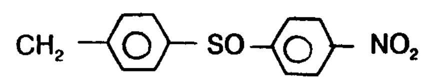 | |
| 466 | H | $C_2H_5$ | Cl | H | 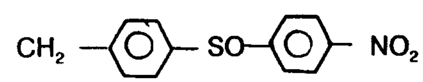 | |

| Beispiel Nr. | $R^1$ | $R^2$ | $R^3$ | $R^4$ | Q | Fp. |
|---|---|---|---|---|---|---|
| 467 | H | $-(CH_2)_4-$ | | $CH_3$ | 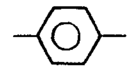 $SO-CH_3$ | |
| 468 | H | $CH_2OCH_3$ | $OCH_3$ | $CH_3$ | 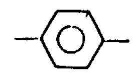 $SO-CH_3$ | |
| 469 | H | $C_2H_5$ | Cl | $CH_3$ | 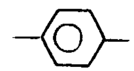 $SO-CH_3$ | |
| 470 | H | $-(CH_2)_4-$ | | $CH_3$ | 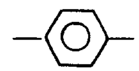 $SO-CH_2CH_3$ | |
| 471 | H | $CH_2OCH_3$ | $OCH_3$ | $CH_3$ | 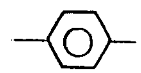 $SO-CH_2CH_3$ | |
| 472 | H | $C_2H_5$ | Cl | $CH_3$ | 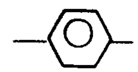 $SO-CH_2CH_3$ | |
| 473 | H | $-(CH_2)_4-$ | | $CH_3$ | 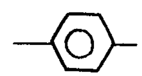 $SO-(CH_2)_2CH_3$ | |
| 474 | H | $CH_2OCH_3$ | $OCH_3$ | $CH_3$ | 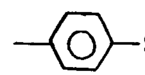 $SO-(CH_2)_2CH_3$ | |
| 475 | H | $C_2H_5$ | Cl | $CH_3$ | 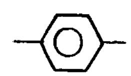 $SO-(CH_2)_2CH_3$ | |

| Beispiel Nr. | $R^1$ | $R^2$ | $R^3$ | $R^4$ | Q | Fp. |
|---|---|---|---|---|---|---|
| 476 | H | $-(CH_2)_4-$ | | $CH_3$ | 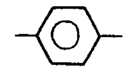 $SO-(CH_2)_3CH_3$ | |
| 477 | H | $CH_2OCH_3$ | $OCH_3$ | $CH_3$ | 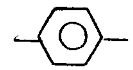 $SO-(CH_2)_3CH_3$ | |
| 478 | H | $C_2H_5$ | Cl | $CH_3$ | 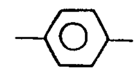 $SO-(CH_2)_3CH_3$ | |
| 479 | H | $-(CH_2)_4-$ | | $CH_3$ | 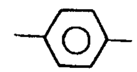 $SO-(CH_2)_4CH_3$ | |
| 480 | H | $CH_2OCH_3$ | $OCH_3$ | $CH_3$ | 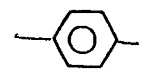 $SO-(CH_2)_4CH_3$ | |
| 481 | H | $C_2H_5$ | Cl | $CH_3$ | 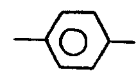 $SO-(CH_2)_4CH_3$ | |
| 482 | H | $-(CH_2)_4-$ | | $CH_3$ | 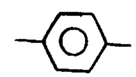 $SO-(CH_2)_5CH_3$ | |
| 483 | H | $CH_2OCH_3$ | $OCH_3$ | $CH_3$ | 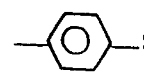 $SO-(CH_2)_5CH_3$ | |
| 484 | H | $C_2H_5$ | Cl | $CH_3$ | 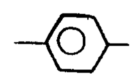 $SO-(CH_2)_5CH_3$ | |

| Beispiel Nr. | $R^1$ | $R^2$ | $R^3$ | $R^4$ | Q | Fp. |
|---|---|---|---|---|---|---|
| 485 | H | $-(CH_2)_4-$ | | $CH_3$ | 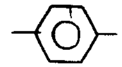 $SO-(CH_2)_6CH_3$ | |
| 486 | H | $CH_2OCH_3$ | $OCH_3$ | $CH_3$ | 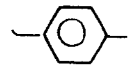 $SO-(CH_2)_6CH_3$ | |
| 487 | H | $C_2H_5$ | Cl | $CH_3$ | 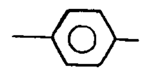 $SO-(CH_2)_6CH_3$ | |
| 488 | H | $-(CH_2)_4-$ | | $CH_3$ | 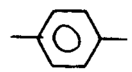 $SO-(CH_2)_7CH_3$ | |
| 489 | H | $CH_2OCH_3$ | $OCH_3$ | $CH_3$ | 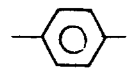 $SO-(CH_2)_7CH_3$ | |
| 490 | H | $C_2H_5$ | Cl | $CH_3$ | 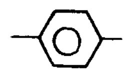 $SO-(CH_2)_7CH_3$ | |
| 491 | H | $-(CH_2)_4-$ | | $CH_3$ | 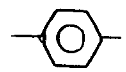 SO 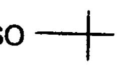 | |
| 492 | H | $CH_2OCH_3$ | $OCH_3$ | $CH_3$ | 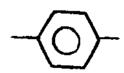 SO 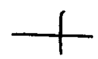 | |
| 493 | H | $C_2H_5$ | Cl | $CH_3$ | 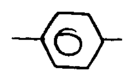 SO 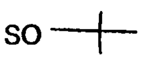 | |

| Beispiel Nr. | $R^1$ | $R^2$ | $R^3$ | $R^4$ | Q | Fp. |
|---|---|---|---|---|---|---|
| 494 | H | $-(CH_2)_4-$ | | $CH_3$ | 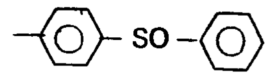 | |
| 495 | H | $CH_2OCH_3$ | $OCH_3$ | $CH_3$ | 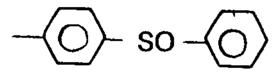 | |
| 496 | H | $C_2H_5$ | Cl | $CH_3$ | 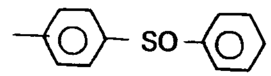 | |
| 497 | H | $-(CH_2)_4-$ | | $CH_3$ | 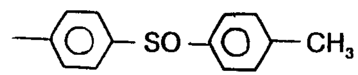 | |
| 498 | H | $CH_2OCH_3$ | $OCH_3$ | $CH_3$ | 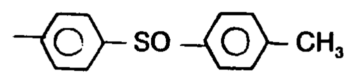 | |
| 499 | H | $C_2H_5$ | Cl | $CH_3$ | 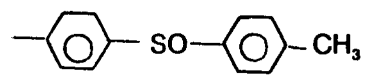 | |
| 500 | H | $-(CH_2)_4-$ | | $CH_3$ | 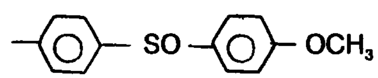 | |
| 501 | H | $CH_2OCH_3$ | $OCH_3$ | $CH_3$ | 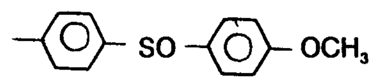 | |
| 502 | H | $C_2H_5$ | Cl | $CH_3$ | 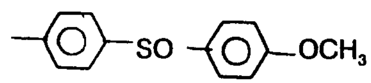 | |

| Beispiel Nr. | $R^1$ | $R^2$ | $R^3$ | $R^4$ | Q | Fp. |
|---|---|---|---|---|---|---|
| 503 | H | $-(CH_2)_4-$ | | $CH_3$ | 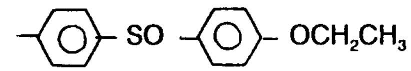 | |
| 504 | H | $CH_2OCH_3$ | $OCH_3$ | $CH_3$ | 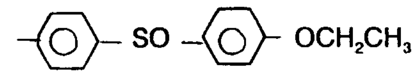 | |
| 505 | H | $C_2H_5$ | Cl | $CH_3$ | 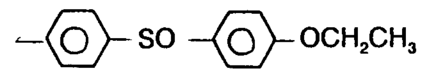 | |
| 506 | H | $-(CH_2)_4-$ | | $CH_3$ | 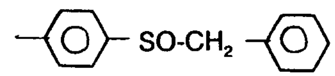 | |
| 507 | H | $CH_2OCH_3$ | $OCH_3$ | $CH_3$ | 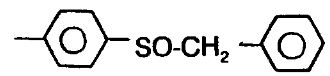 | |
| 508 | H | $C_2H_5$ | Cl | $CH_3$ | 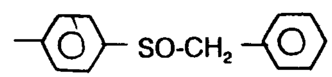 | |
| 509 | H | $-(CH_2)_4-$ | | $CH_3$ | 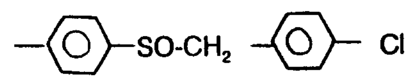 | |
| 510 | H | $CH_2OCH_3$ | $OCH_3$ | $CH_3$ | 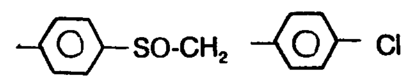 | |
| 511 | H | $C_2H_5$ | Cl | $CH_3$ | 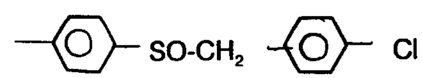 | |

| Bei-spiel Nr. | $R^1$ | $R^2$ | $R^3$ | $R^4$ | Q | Fp. |
|---|---|---|---|---|---|---|
| 512 | H | $-(CH_2)_4-$ | | $CH_3$ | 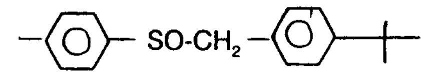 | |
| 513 | H | $CH_2OCH_3$ | $OCH_3$ | $CH_3$ | 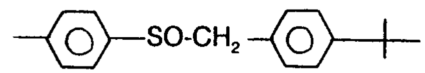 | |
| 514 | H | $C_2H_5$ | Cl | $CH_3$ | 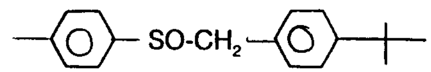 | |
| 515 | H | $-(CH_2)_4-$ | | H | 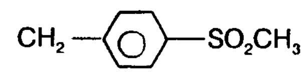 | |
| 516 | H | $CH_2OCH_3$ | $OCH_3$ | H | 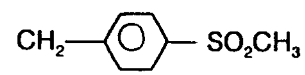 | |
| 517 | H | $C_2H_5$ | Cl | H | 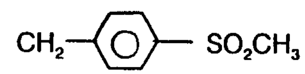 | |
| 518 | H | $-(CH_2)_4-$ | | H | 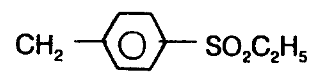 | |
| 519 | H | $CH_2OCH_3$ | $OCH_3$ | H | 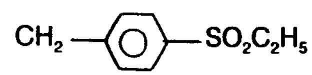 | |
| 520 | H | $C_2H_5$ | Cl | H | 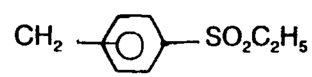 | |

| Beispiel Nr. | $R^1$ | $R^2$ | $R^3$ | $R^4$ | Q | Fp. |
|---|---|---|---|---|---|---|
| 521 | H | $-(CH_2)_4-$ | | H | $-CH_2$ 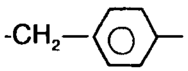 $SO_2(CH_2)_2CH_3$ | |
| 522 | H | $CH_2OCH_3$ | $OCH_3$ | H | $-CH_2$ 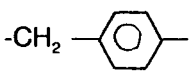 $SO_2(CH_2)_2CH_3$ | |
| 523 | H | $C_2H_5$ | Cl | H | $-CH_2$ 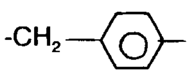 $SO_2(CH_2)_2CH_3$ | |
| 524 | H | $-(CH_2)_4-$ | | H | $CH_2$ 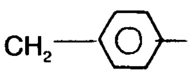 $SO_2(CH_2)_3CH_3$ | Öl |
| 525 | H | $CH_2OCH_3$ | $OCH_3$ | H | $CH_2$ 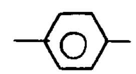 $SO_2(CH_2)_3CH_3$ | Öl |
| 526 | H | $C_2H_5$ | Cl | H | $CH_2$ 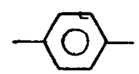 $SO_2(CH_2)_3CH_3$ | Öl |
| 527 | H | $-(CH_2)_4-$ | | H | $CH_2$ 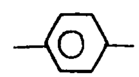 $SO_2(CH_2)_4CH_3$ | |
| 528 | H | $CH_2OCH_3$ | $OCH_3$ | H | $CH_2$ 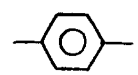 $SO_2(CH_2)_4CH_3$ | |
| 529 | H | $C_2H_5$ | Cl | H | $CH_2$ 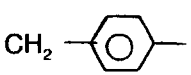 $SO_2(CH_2)_4CH_3$ | |

| Beispiel Nr. | $R^1$ | $R^2$ | $R^3$ | $R^4$ | Q | Fp. |
|---|---|---|---|---|---|---|
| 530 | H | $-(CH_2)_4-$ | | H | $CH_2$ 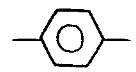 $SO_2(CH_2)_5CH_3$ | |
| 531 | H | $CH_2OCH_3$ | $OCH_3$ | H | $CH_2$ 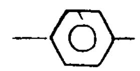 $SO_2(CH_2)_5CH_3$ | |
| 532 | H | $C_2H_5$ | Cl | H | $CH_2$ 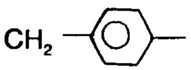 $SO_2(CH_2)_5CH_3$ | |
| 533 | H | $-(CH_2)_4-$ | | H | $CH_2$ 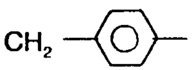 $SO_2(CH_2)_6CH_3$ | |
| 534 | H | $CH_2OCH_3$ | $OCH_3$ | H | $CH_2$ 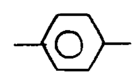 $SO_2(CH_2)_6CH_3$ | |
| 535 | H | $C_2H_5$ | Cl | H | $CH_2$ 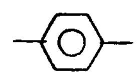 $SO_2(CH_2)_6CH_3$ | |
| 536 | H | $-(CH_2)_4-$ | | H | $CH_2$ 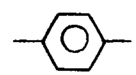 $SO_2(CH_2)_7CH_3$ | |
| 537 | H | $CH_2OCH_3$ | $OCH_3$ | H | $CH_2$ 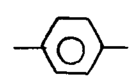 $SO_2(CH_2)_7CH_3$ | |
| 538 | H | $C_2H_5$ | Cl | H | $CH_2$ 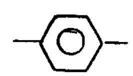 $SO_2(CH_2)_7CH_3$ | |

| Beispiel Nr. | $R^1$ | $R^2$ | $R^3$ | $R^4$ | Q | Fp. |
|---|---|---|---|---|---|---|
| 539 | H | $-(CH_2)_4-$ | | H | $CH_2$ 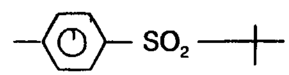 | |
| 540 | H | $CH_2OCH_3$ | $OCH_3$ | H | $CH_2$ 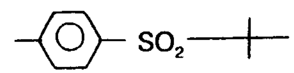 | |
| 541 | H | $C_2H_5$ | Cl | H | $CH_2$ 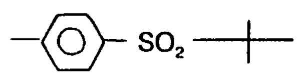 | |
| 542 | H | $-(CH_2)_4-$ | | H | $CH_2$ 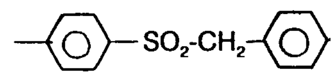 | |
| 543 | H | $CH_2OCH_3$ | $OCH_3$ | H | $CH_2$ 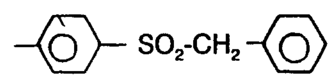 | |
| 544 | H | $C_2H_5$ | Cl | H | $CH_2$ 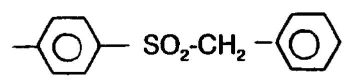 | |
| 545 | H | $-(CH_2)_4-$ | | H | $CH_2$ 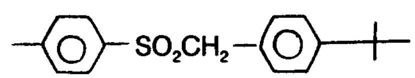 | |
| 546 | H | $CH_2OCH_3$ | $OCH_3$ | H | $CH_2$ 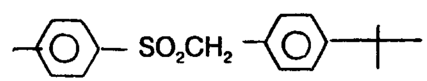 | |
| 547 | H | $C_2H_5$ | Cl | H | $CH_2$ 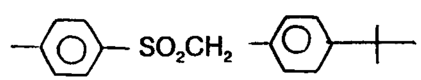 | |

| Beispiel Nr. | $R^1$ | $R^2$ | $R^3$ | $R^4$ | Q | Fp. |
|---|---|---|---|---|---|---|
| 548 | H | $-(CH_2)_4-$ | | H | 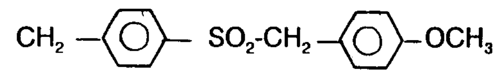 | |
| 549 | H | $CH_2OCH_3$ | $OCH_3$ | H | 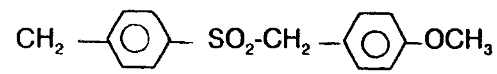 | |
| 550 | H | $C_2H_5$ | Cl | H | 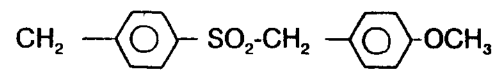 | |
| 551 | H | $-(CH_2)_4-$ | | H | 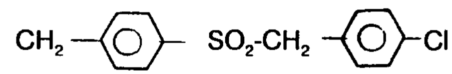 | |
| 552 | H | $CH_2OCH_3$ | $OCH_3$ | H | 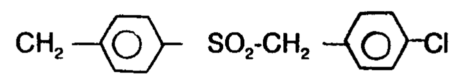 | |
| 553 | H | $C_2H_5$ | Cl | H | 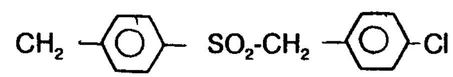 | |
| 554 | H | $-(CH_2)_4-$ | | H | 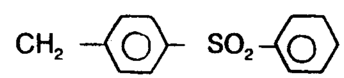 | |
| 555 | H | $CH_2OCH_3$ | $OCH_3$ | H | 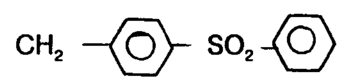 | |
| 556 | H | $C_2H_5$ | Cl | H | 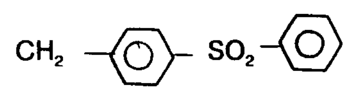 | |

| Beispiel Nr. | $R^1$ | $R^2$ | $R^3$ | $R^4$ | Q | Fp. |
|---|---|---|---|---|---|---|
| 557 | H | $-(CH_2)_4-$ | | H | 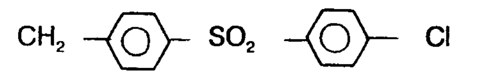 | |
| 558 | H | $CH_2OCH_3$ | $OCH_3$ | H | 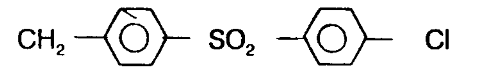 | |
| 559 | H | $C_2H_5$ | Cl | H | 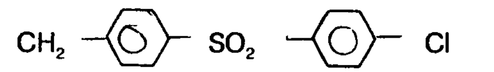 | |
| 560 | H | $-(CH_2)_4-$ | | H | 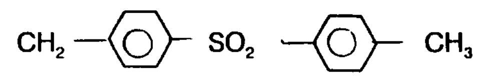 | |
| 561 | H | $CH_2OCH_3$ | $OCH_3$ | H | 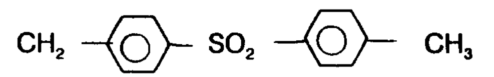 | |
| 562 | H | $C_2H_5$ | Cl | H | 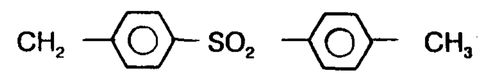 | |
| 563 | H | $-(CH_2)_4-$ | | H | 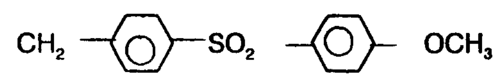 | |
| 564 | H | $CH_2OCH_3$ | $OCH_3$ | H | 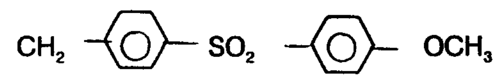 | |
| 565 | H | $C_2H_5$ | Cl | H | 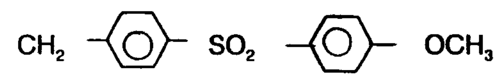 | |

| Beispiel Nr. | $R^1$ | $R^2$ | $R^3$ | $R^4$ | Q | Fp. |
|---|---|---|---|---|---|---|
| 566 | H | $-(CH_2)_4-$ | | H | 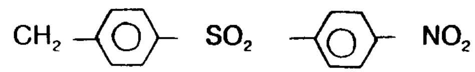 | |
| 567 | H | $CH_2OCH_3$ | $OCH_3$ | H | 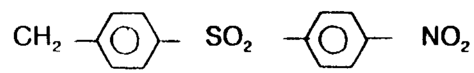 | |
| 568 | H | $C_2H_5$ | Cl | H | 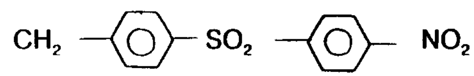 | |
| 569 | H | $-(CH_2)_4-$ | | $CH_3$ | 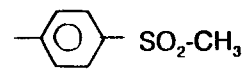 | |
| 570 | H | $CH_2OCH_3$ | $OCH_3$ | $CH_3$ | 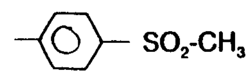 | |
| 571 | H | $C_2H_5$ | Cl | $CH_3$ | 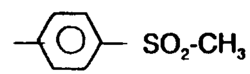 | |
| 572 | H | $-(CH_2)_4-$ | | $CH_3$ | 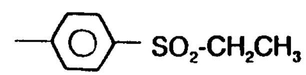 | |
| 573 | H | $CH_2OCH_3$ | $OCH_3$ | $CH_3$ | 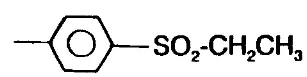 | |
| 574 | H | $C_2H_5$ | Cl | $CH_3$ | 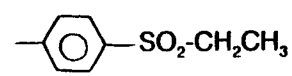 | |

| Beispiel Nr. | $R^1$ | $R^2$ | $R^3$ | $R^4$ | Q | Fp. |
|---|---|---|---|---|---|---|
| 575 | H | $-(CH_2)_4-$ | | $CH_3$ | 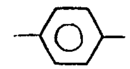 $SO_2-(CH_2)_2CH_3$ | |
| 576 | H | $CH_2OCH_3$ | $OCH_3$ | $CH_3$ | 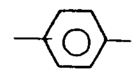 $SO_2-(CH_2)_2CH_3$ | |
| 577 | H | $C_2H_5$ | Cl | $CH_3$ | 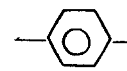 $SO_2-(CH_2)_2CH_3$ | |
| 578 | H | $-(CH_2)_4-$ | | $CH_3$ | 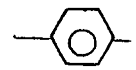 $SO_2-(CH_2)_3CH_3$ | |
| 579 | H | $CH_2OCH_3$ | $OCH_3$ | $CH_3$ | 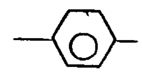 $SO_2-(CH_2)_3CH_3$ | |
| 580 | H | $C_2H_5$ | Cl | $CH_3$ | 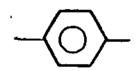 $SO_2-(CH_2)_3CH_3$ | |
| 581 | H | $-(CH_2)_4-$ | | $CH_3$ | 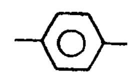 $SO_2-(CH_2)_4CH_3$ | |
| 582 | H | $CH_2OCH_3$ | $OCH_3$ | $CH_3$ | 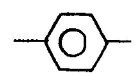 $SO_2-(CH_2)_4CH_3$ | |
| 583 | H | $C_2H_5$ | Cl | $CH_3$ | 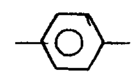 $SO_2-(CH_2)_4CH_3$ | |

| Bei-spiel Nr. | $R^1$ | $R^2$ | $R^3$ | $R^4$ | Q | Fp. |
|---|---|---|---|---|---|---|
| 584 | H | $-(CH_2)_4-$ | | $CH_3$ | 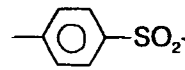 $SO_2-(CH_2)_5CH_3$ | |
| 585 | H | $CH_2OCH_3$ | $OCH_3$ | $CH_3$ | 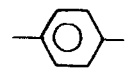 $SO_2-(CH_2)_5CH_3$ | |
| 586 | H | $C_2H_5$ | Cl | $CH_3$ | 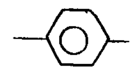 $SO_2-(CH_2)_5CH_3$ | |
| 587 | H | $-(CH_2)_4-$ | | $CH_3$ | 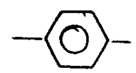 $SO_2-(CH_2)_6CH_3$ | |
| 588 | H | $CH_2OCH_3$ | $OCH_3$ | $CH_3$ | 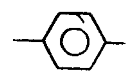 $SO_2-(CH_2)_6CH_3$ | |
| 589 | H | $C_2H_5$ | Cl | $CH_3$ | 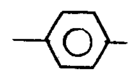 $SO_2-(CH_2)_6CH_3$ | |
| 590 | H | $-(CH_2)_4-$ | | $CH_3$ | 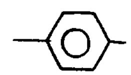 $SO_2-(CH_2)_7CH_3$ | |
| 591 | H | $CH_2OCH_3$ | $OCH_3$ | $CH_3$ | 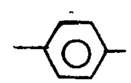 $SO_2-(CH_2)_7CH_3$ | |
| 592 | H | $C_2H_5$ | Cl | $CH_3$ | 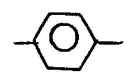 $SO_2-(CH_2)_7CH_3$ | |

| Bei-spiel Nr. | $R^1$ | $R^2$ | $R^3$ | $R^4$ | Q | Fp. |
|---|---|---|---|---|---|---|
| 593 | H | $-(CH_2)_4-$ | | $CH_3$ | 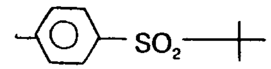 | |
| 594 | H | $CH_2OCH_3$ | $OCH_3$ | $CH_3$ | 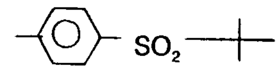 | |
| 595 | H | $C_2H_5$ | Cl | $CH_3$ | 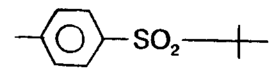 | |
| 596 | H | $-(CH_2)_4-$ | | $CH_3$ | 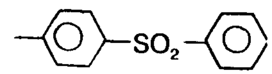 | |
| 597 | H | $CH_2OCH_3$ | $OCH_3$ | $CH_3$ | 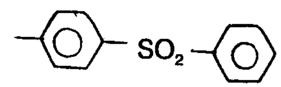 | |
| 598 | H | $C_2H_5$ | Cl | $CH_3$ | 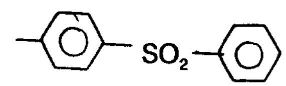 | |
| 599 | H | $-(CH_2)_4-$ | | $CH_3$ | 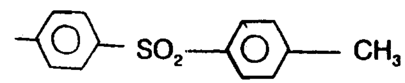 | |
| 600 | H | $CH_2OCH_3$ | $OCH_3$ | $CH_3$ | 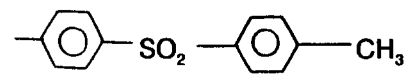 | |
| 601 | H | $C_2H_5$ | Cl | $CH_3$ | 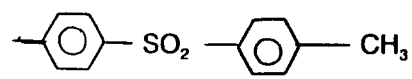 | |

| Bei-spiel Nr. | $R^1$ | $R^2$ | $R^3$ | $R^4$ | Q | Fp. |
|---|---|---|---|---|---|---|
| 602 | H | $-(CH_2)_4-$ | | $CH_3$ | 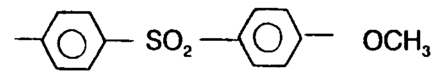 | |
| 603 | H | $CH_2OCH_3$ | $OCH_3$ | $CH_3$ | 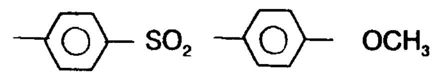 | |
| 604 | H | $C_2H_5$ | Cl | $CH_3$ | 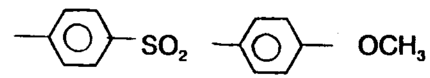 | |
| 605 | H | $-(CH_2)_4-$ | | $CH_3$ | 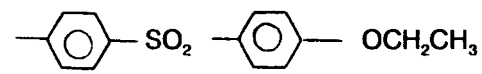 | |
| 606 | H | $CH_2OCH_3$ | $OCH_3$ | $CH_3$ | 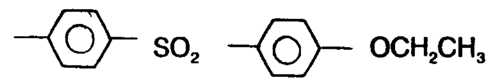 | |
| 607 | H | $C_2H_5$ | Cl | $CH_3$ | 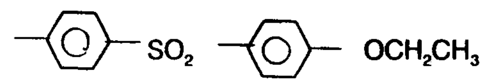 | |
| 608 | H | $-(CH_2)_4-$ | | $CH_3$ | 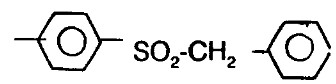 | |
| 609 | H | $CH_2OCH_3$ | $OCH_3$ | $CH_3$ | 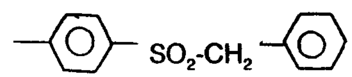 | |
| 610 | H | $C_2H_5$ | Cl | $CH_3$ | 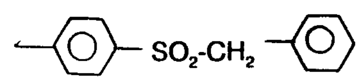 | |

| Bei-spiel Nr. | $R^1$ | $R^2$ | $R^3$ | $R^4$ | Q | Fp. |
|---|---|---|---|---|---|---|
| 611 | H | $-(CH_2)_4-$ | | $CH_3$ | 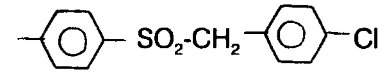 | |
| 612 | H | $CH_2OCH_3$ | $OCH_3$ | $CH_3$ | 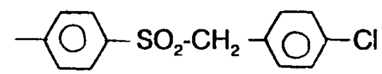 | |
| 613 | H | $C_2H_5$ | Cl | $CH_3$ | 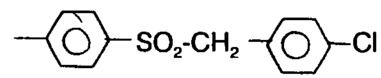 | |
| 614 | H | $-(CH_2)_4-$ | | $CH_3$ | 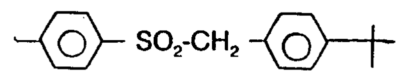 | |
| 615 | H | $CH_2OCH_3$ | $OCH_3$ | $CH_3$ | 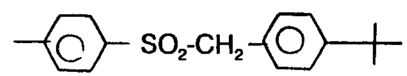 | |
| 616 | H | $C_2H_5$ | Cl | $CH_3$ | 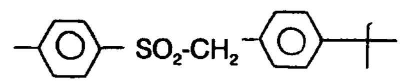 | |
| 617 | H | $-(CH_2)_4-$ | | $CH_3$ | $CH_2$ 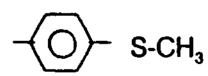 | |
| 618 | H | $CH_2OCH_3$ | $OCH_3$ | $CH_3$ | $CH_2$ 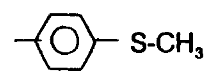 | |
| 619 | H | $C_2H_5$ | Cl | $CH_3$ | $CH_2$ 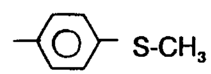 | |

| Beispiel Nr. | $R^1$ | $R^2$ | $R^3$ | $R^4$ | Q | Fp. |
|---|---|---|---|---|---|---|
| 620 | H | $-(CH_2)_4-$ | | $CH_3$ | $CH_2$ 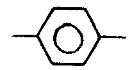 $S-CH_2CH_3$ | |
| 621 | H | $CH_2OCH_3$ | $OCH_3$ | $CH_3$ | $CH_2$ 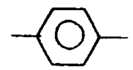 $S-CH_2CH_3$ | |
| 622 | H | $C_2H_5$ | Cl | $CH_3$ | $CH_2$ 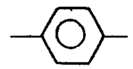 $S-CH_2CH_3$ | |
| 623 | H | $-(CH_2)_4-$ | | $CH_3$ | $CH_2$ 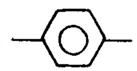 $S-(CH_2)_2CH_3$ | |
| 624 | H | $CH_2OCH_3$ | $OCH_3$ | $CH_3$ | $CH_2$ 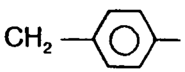 $S-(CH_2)_2CH_3$ | |
| 625 | H | $C_2H_5$ | Cl | $CH_3$ | $CH_2$ 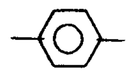 $S-(CH_2)_2CH_3$ | |
| 626 | H | $-(CH_2)_4-$ | | $CH_3$ | $CH_2$ 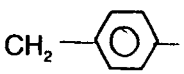 $S-(CH_2)_3CH_3$ | |
| 627 | H | $CH_2OCH_3$ | $OCH_3$ | $CH_3$ | $CH_2$ 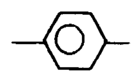 $S-(CH_2)_3CH_3$ | |
| 628 | H | $C_2H_5$ | Cl | $CH_3$ | $CH_2$ 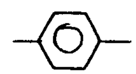 $S-(CH_2)_3CH_3$ | |

| Beispiel Nr. | $R^1$ | $R^2$ | $R^3$ | $R^4$ | Q | Fp. |
|---|---|---|---|---|---|---|
| 629 | H | $-(CH_2)_4-$ | | $CH_3$ | $CH_2$ 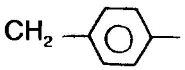 $S-(CH_2)_4CH_3$ | |
| 630 | H | $CH_2OCH_3$ | $OCH_3$ | $CH_3$ | $CH_2$ 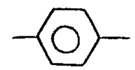 $S-(CH_2)_4CH_3$ | |
| 631 | H | $C_2H_5$ | Cl | $CH_3$ | $CH_2$ 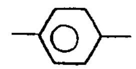 $S-(CH_2)_4CH_3$ | |
| 632 | H | $-(CH_2)_4-$ | | $CH_3$ | $CH_2$ 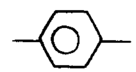 $S-(CH_2)_5CH_3$ | |
| 633 | H | $CH_2OCH_3$ | $OCH_3$ | $CH_3$ | $CH_2$ 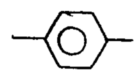 $S-(CH_2)_5CH_3$ | |
| 634 | H | $C_2H_5$ | Cl | $CH_3$ | $CH_2$ 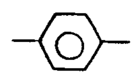 $S-(CH_2)_5CH_3$ | |
| 635 | H | $-(CH_2)_4-$ | | $CH_3$ | $CH_2$ 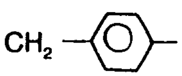 $S-(CH_2)_6CH_3$ | |
| 636 | H | $CH_2OCH_3$ | $OCH_3$ | $CH_3$ | $CH_2$ 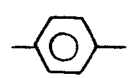 $S-(CH_2)_6CH_3$ | |
| 637 | H | $C_2H_5$ | Cl | $CH_3$ | $CH_2$ 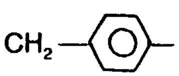 $S-(CH_2)_6CH_3$ | |

| Beispiel Nr. | $R^1$ | $R^2$ | $R^3$ | $R^4$ | Q | Fp. |
|---|---|---|---|---|---|---|
| 638 | H | $-(CH_2)_4-$ | | $CH_3$ | $CH_2$ 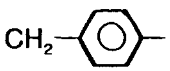 $S-(CH_2)_7CH_3$ | |
| 639 | H | $CH_2OCH_3$ | $OCH_3$ | $CH_3$ | $CH_2$ 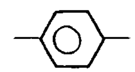 $S-(CH_2)_7CH_3$ | |
| 640 | H | $C_2H_5$ | Cl | $CH_3$ | $CH_2$ 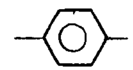 $S-(CH_2)_7CH_3$ | |
| 641 | H | $-(CH_2)_4-$ | | $CH_3$ | $CH_2$ 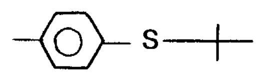 | |
| 642 | H | $CH_2OCH_3$ | $OCH_3$ | $CH_3$ | $CH_2$ 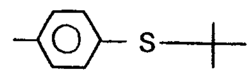 | |
| 643 | H | $C_2H_5$ | Cl | $CH_3$ | $CH_2$ 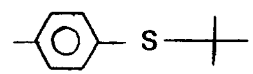 | |
| 644 | H | $-(CH_2)_4-$ | | $CH_3$ | $CH_2$ 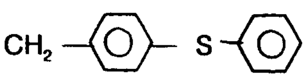 | |
| 645 | H | $CH_2OCH_3$ | $OCH_3$ | $CH_3$ | $CH_2$ 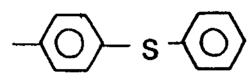 | |
| 646 | H | $C_2H_5$ | Cl | $CH_3$ | $CH_2$ 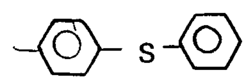 | |

| Beispiel Nr. | $R^1$ | $R^2$ | $R^3$ | $R^4$ | Q | Fp. |
|---|---|---|---|---|---|---|
| 647 | H | $-(CH_2)_4-$ | | $CH_3$ | 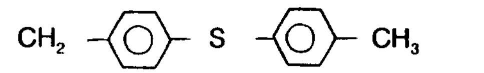 | |
| 648 | H | $CH_2OCH_3$ | $OCH_3$ | $CH_3$ | 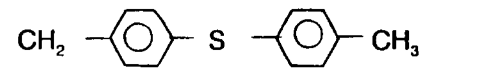 | |
| 649 | H | $C_2H_5$ | Cl | $CH_3$ | 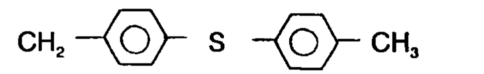 | |
| 650 | H | $-(CH_2)_4-$ | | $CH_3$ | 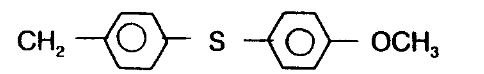 | |
| 651 | H | $CH_2OCH_3$ | $OCH_3$ | $CH_3$ | 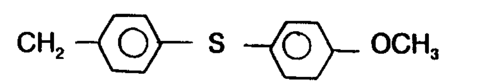 | |
| 652 | H | $C_2H_5$ | Cl | $CH_3$ | 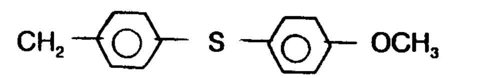 | |
| 653 | H | $-(CH_2)_4-$ | | $CH_3$ | 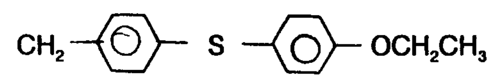 | |
| 654 | H | $CH_2OCH_3$ | $OCH_3$ | $CH_3$ | 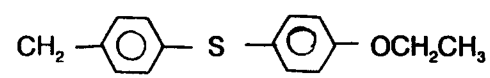 | |
| 655 | H | $C_2H_5$ | Cl | $CH_3$ | 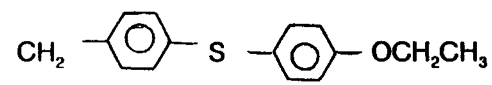 | |

| Beispiel Nr. | $R^1$ | $R^2$ | $R^3$ | $R^4$ | Q | Fp. |
|---|---|---|---|---|---|---|
| 656 | H | $-(CH_2)_4-$ | | $CH_3$ | 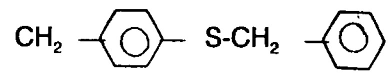 | |
| 657 | H | $CH_2OCH_3$ | $OCH_3$ | $CH_3$ | 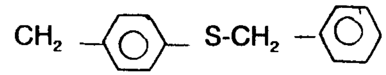 | |
| 658 | H | $C_2H_5$ | Cl | $CH_3$ | 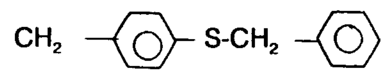 | |
| 659 | H | $-(CH_2)_4-$ | | $CH_3$ | 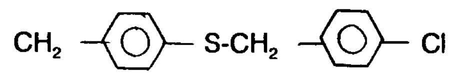 | |
| 660 | H | $CH_2OCH_3$ | $OCH_3$ | $CH_3$ | 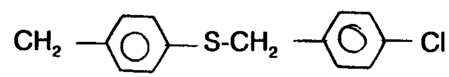 | |
| 661 | H | $C_2H_5$ | Cl | $CH_3$ | 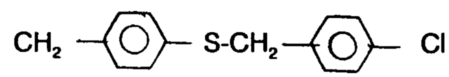 | |
| 662 | H | $-(CH_2)_4-$ | | $CH_3$ | 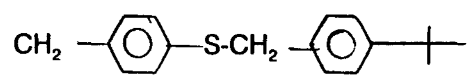 | |
| 663 | H | $CH_2OCH_3$ | $OCH_3$ | $CH_3$ | 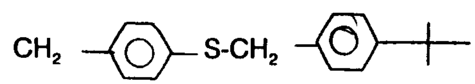 | |
| 664 | H | $C_2H_5$ | Cl | $CH_3$ | 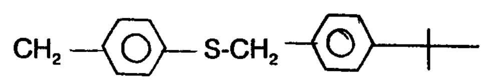 | |

| Bei-spiel Nr. | $R^1$ | $R^2$ | $R^3$ | $R^4$ | Q | Fp. |
|---|---|---|---|---|---|---|
| 665 | H | $-(CH_2)_4-$ | | $CH_3$ | $CH_2$ 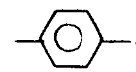 $SO-CH_3$ | |
| 666 | H | $CH_2OCH_3$ | $OCH_3$ | $CH_3$ | $CH_2$ 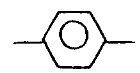 $SO-CH_3$ | |
| 667 | H | $C_2H_5$ | Cl | $CH_3$ | $CH_2$ 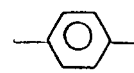 $SO-CH_3$ | |
| 668 | H | $-(CH_2)_4-$ | | $CH_3$ | $CH_2$ 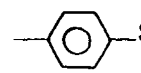 $SO-CH_2CH_3$ | |
| 669 | H | $CH_2OCH_3$ | $OCH_3$ | $CH_3$ | $CH_2$ 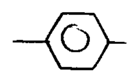 $SO-CH_2CH_3$ | |
| 670 | H | $C_2H_5$ | Cl | $CH_3$ | $CH_2$ 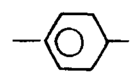 $SO-CH_2CH_3$ | |
| 671 | H | $-(CH_2)_4-$ | | $CH_3$ | $CH_2$ 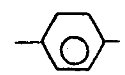 $SO-(CH_2)_2CH_3$ | |
| 672 | H | $CH_2OCH_3$ | $OCH_3$ | $CH_3$ | $CH_2$ 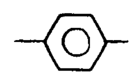 $SO-(CH_2)_2CH_3$ | |
| 673 | H | $C_2H_5$ | Cl | $CH_3$ | $CH_2$ 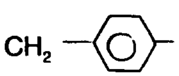 $SO-(CH_2)_2CH_3$ | |

| Beispiel Nr. | $R^1$ | $R^2$ | $R^3$ | $R^4$ | Q | Fp. |
|---|---|---|---|---|---|---|
| 674 | H | $-(CH_2)_4-$ | | $CH_3$ | $CH_2$ 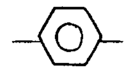 $SO-(CH_2)_3CH_3$ | |
| 675 | H | $CH_2OCH_3$ | $OCH_3$ | $CH_3$ | $CH_2$ 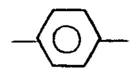 $SO-(CH_2)_3CH_3$ | |
| 676 | H | $C_2H_5$ | Cl | $CH_3$ | $CH_2$ 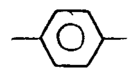 $SO-(CH_2)_3CH_3$ | |
| 677 | H | $-(CH_2)_4-$ | | $CH_3$ | $CH_2$ 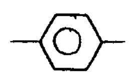 $SO-(CH_2)_4CH_3$ | |
| 678 | H | $CH_2OCH_3$ | $OCH_3$ | $CH_3$ | $CH_2$ 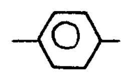 $SO-(CH_2)_4CH_3$ | |
| 679 | H | $C_2H_5$ | Cl | $CH_3$ | $CH_2$ 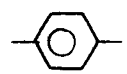 $SO-(CH_2)_4CH_3$ | |
| 680 | H | $-(CH_2)_4-$ | | $CH_3$ | $CH_2$ 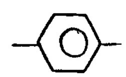 $SO-(CH_2)_5CH_3$ | |
| 681 | H | $CH_2OCH_3$ | $OCH_3$ | $CH_3$ | $CH_2$ 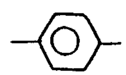 $SO-(CH_2)_5CH_3$ | |
| 682 | H | $C_2H_5$ | Cl | $CH_3$ | $CH_2$ 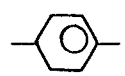 $SO-(CH_2)_5CH_3$ | |

| Beispiel Nr. | $R^1$ | $R^2$ | $R^3$ | $R^4$ | Q | Fp. |
|---|---|---|---|---|---|---|
| 683 | H | $-(CH_2)_4-$ | | $CH_3$ | 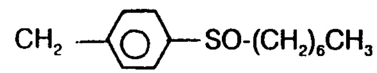 | |
| 684 | H | $CH_2OCH_3$ | $OCH_3$ | $CH_3$ | 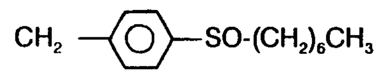 | |
| 685 | H | $C_2H_5$ | Cl | $CH_3$ | 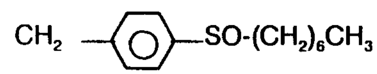 | |
| 686 | H | $-(CH_2)_4-$ | | $CH_3$ | 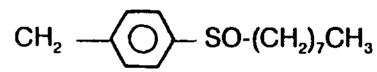 | |
| 687 | H | $CH_2OCH_3$ | $OCH_3$ | $CH_3$ | 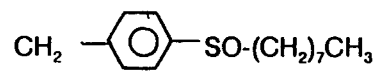 | |
| 688 | H | $C_2H_5$ | Cl | $CH_3$ | 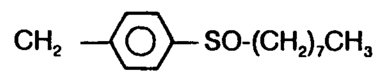 | |
| 689 | H | $-(CH_2)_4-$ | | $CH_3$ | 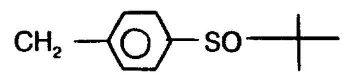 | |
| 690 | H | $CH_2OCH_3$ | $OCH_3$ | $CH_3$ | 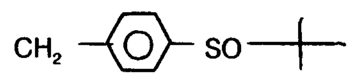 | |
| 691 | H | $C_2H_5$ | Cl | $CH_3$ | 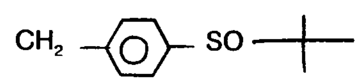 | |

| Beispiel Nr. | $R^1$ | $R^2$ | $R^3$ | $R^4$ | Q | Fp. |
|---|---|---|---|---|---|---|
| 692 | H | $-(CH_2)_4-$ | | $CH_3$ | $CH_2$ 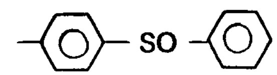 | |
| 693 | H | $CH_2OCH_3$ | $OCH_3$ | $CH_3$ | $CH_2$ 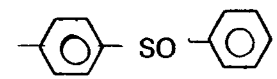 | |
| 694 | H | $C_2H_5$ | Cl | $CH_3$ | $CH_2$ 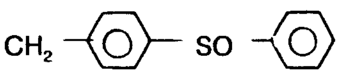 | |
| 695 | H | $-(CH_2)_4-$ | | $CH_3$ | $CH_2$ 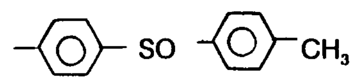 | |
| 696 | H | $CH_2OCH_3$ | $OCH_3$ | $CH_3$ | $CH_2$ 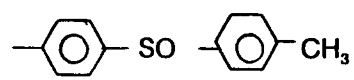 | |
| 697 | H | $C_2H_5$ | Cl | $CH_3$ | $CH_2$ 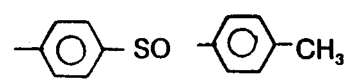 | |
| 698 | H | $-(CH_2)_4-$ | | $CH_3$ | $CH_2$ 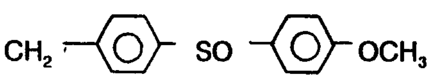 | |
| 699 | H | $CH_2OCH_3$ | $OCH_3$ | $CH_3$ | $CH_2$ 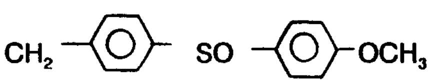 | |
| 700 | H | $C_2H_5$ | Cl | $CH_3$ | $CH_2$ 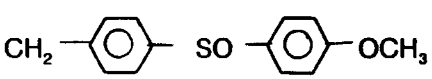 | |

| Beispiel Nr. | $R^1$ | $R^2$ | $R^3$ | $R^4$ | Q | Fp. |
|---|---|---|---|---|---|---|
| 701 | H | $-(CH_2)_4-$ | | $CH_3$ | $CH_2$ 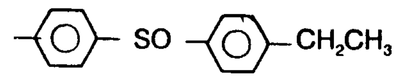 | |
| 702 | H | $CH_2OCH_3$ | $OCH_3$ | $CH_3$ | $CH_2$ 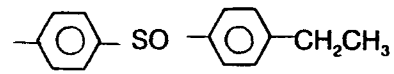 | |
| 703 | H | $C_2H_5$ | Cl | $CH_3$ | $CH_2$ 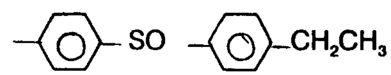 | |
| 704 | H | $-(CH_2)_4-$ | | $CH_3$ | $CH_2$ 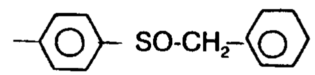 | |
| 705 | H | $CH_2OCH_3$ | $OCH_3$ | $CH_3$ | $CH_2$ 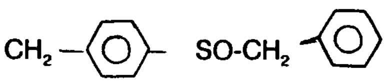 | |
| 706 | H | $C_2H_5$ | Cl | $CH_3$ | $CH_2$ 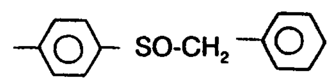 | |
| 707 | H | $-(CH_2)_4-$ | | $CH_3$ | $CH_2$ 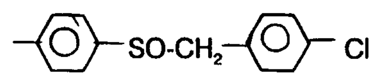 | |
| 708 | H | $CH_2OCH_3$ | $OCH_3$ | $CH_3$ | $CH_2$ 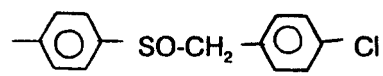 | |
| 709 | H | $C_2H_5$ | Cl | $CH_3$ | $CH_2$ 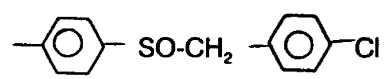 | |

| Beispiel Nr. | $R^1$ | $R^2$ | $R^3$ | $R^4$ | Q | Fp. |
|---|---|---|---|---|---|---|
| 710 | H | $-(CH_2)_4-$ | | $CH_3$ | 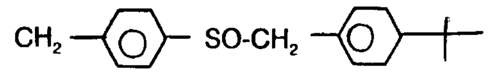 | |
| 711 | H | $CH_2OCH_3$ | $OCH_3$ | $CH_3$ | 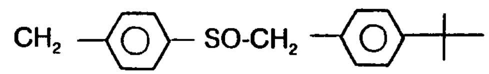 | |
| 712 | H | $C_2H_5$ | Cl | $CH_3$ | 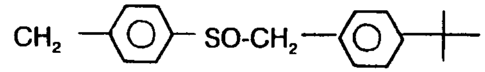 | |
| 713 | H | $-(CH_2)_4-$ | | $CH_3$ | 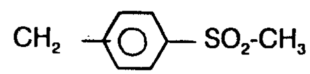 | |
| 714 | H | $CH_2OCH_3$ | $OCH_3$ | $CH_3$ | 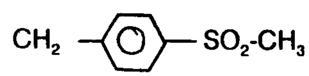 | |
| 715 | H | $C_2H_5$ | Cl | $CH_3$ | 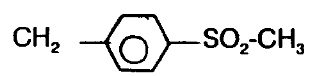 | |
| 716 | H | $-(CH_2)_4-$ | | $CH_3$ | 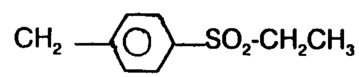 | |
| 717 | H | $CH_2OCH_3$ | $OCH_3$ | $CH_3$ | 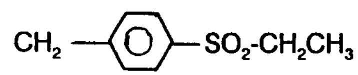 | |
| 718 | H | $C_2H_5$ | Cl | $CH_3$ | 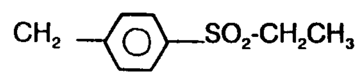 | |

| Beispiel Nr. | $R^1$ | $R^2$ | $R^3$ | $R^4$ | Q | Fp. |
|---|---|---|---|---|---|---|
| 719 | H | $-(CH_2)_4-$ | | $CH_3$ | $CH_2$ 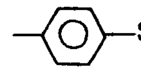 $SO_2(CH_2)_2CH_3$ | |
| 720 | H | $CH_2OCH_3$ | $OCH_3$ | $CH_3$ | $CH_2$ 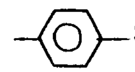 $SO_2(CH_2)_2CH_3$ | |
| 721 | H | $C_2H_5$ | Cl | $CH_3$ | $CH_2$ 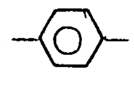 $SO_2(CH_2)_2CH_3$ | |
| 722 | H | $-(CH_2)_4-$ | | $CH_3$ | $CH_2$ 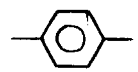 $SO_2(CH_2)_3CH_3$ | |
| 723 | H | $CH_2OCH_3$ | $OCH_3$ | $CH_3$ | $CH_2$ 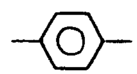 $SO_2(CH_2)_3CH_3$ | |
| 724 | H | $C_2H_5$ | Cl | $CH_3$ | $CH_2$ 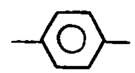 $SO_2(CH_2)_3CH_3$ | |
| 725 | H | $-(CH_2)_4-$ | | $CH_3$ | $CH_2$ 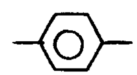 $SO_2(CH_2)_4CH_3$ | |
| 726 | H | $CH_2OCH_3$ | $OCH_3$ | $CH_3$ | $CH_2$ 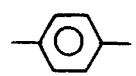 $SO_2(CH_2)_4CH_3$ | |
| 727 | H | $C_2H_5$ | Cl | $CH_3$ | $CH_2$ 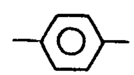 $SO_2(CH_2)_4CH_3$ | |

| Beispiel Nr. | $R^1$ | $R^2$ | $R^3$ | $R^4$ | Q | Fp. |
|---|---|---|---|---|---|---|
| 728 | H | $-(CH_2)_4-$ | | $CH_3$ | $CH_2$ 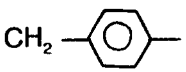 $SO_2(CH_2)_5CH_3$ | |
| 729 | H | $CH_2OCH_3$ | $OCH_3$ | $CH_3$ | $CH_2$ 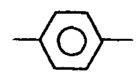 $SO_2(CH_2)_5CH_3$ | |
| 730 | H | $C_2H_5$ | Cl | $CH_3$ | $CH_2$ 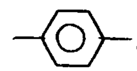 $SO_2(CH_2)_5CH_3$ | |
| 731 | H | $-(CH_2)_4-$ | | $CH_3$ | $CH_2$ 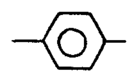 $SO_2(CH_2)_6CH_3$ | |
| 732 | H | $CH_2OCH_3$ | $OCH_3$ | $CH_3$ | $CH_2$ 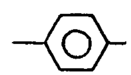 $SO_2(CH_2)_6CH_3$ | |
| 733 | H | $C_2H_5$ | Cl | $CH_3$ | $CH_2$ 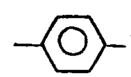 $SO_2(CH_2)_6CH_3$ | |
| 734 | H | $-(CH_2)_4-$ | | $CH_3$ | $CH_2$ 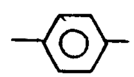 $SO_2(CH_2)_7CH_3$ | |
| 735 | H | $CH_2OCH_3$ | $OCH_3$ | $CH_3$ | $CH_2$ 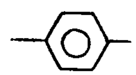 $SO_2(CH_2)_7CH_3$ | |
| 736 | H | $C_2H_5$ | Cl | $CH_3$ | $CH_2$ 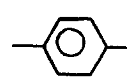 $SO_2(CH_2)_7CH_3$ | |

| Beispiel Nr. | $R^1$ | $R^2$ | $R^3$ | $R^4$ | Q | Fp. |
|---|---|---|---|---|---|---|
| 737 | H | $-(CH_2)_4-$ | | $CH_3$ | 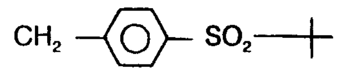 | |
| 738 | H | $CH_2OCH_3$ | $OCH_3$ | $CH_3$ | 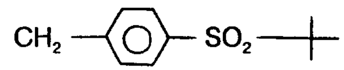 | |
| 739 | H | $C_2H_5$ | Cl | $CH_3$ | 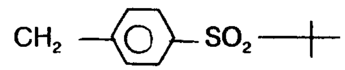 | |
| 740 | H | $-(CH_2)_4-$ | | $CH_3$ | 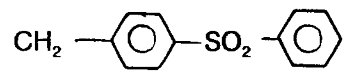 | |
| 741 | H | $CH_2OCH_3$ | $OCH_3$ | $CH_3$ | 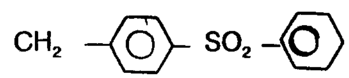 | |
| 742 | H | $C_2H_5$ | Cl | $CH_3$ | 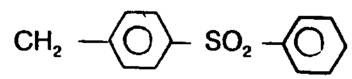 | |
| 743 | H | $-(CH_2)_4-$ | | $CH_3$ | 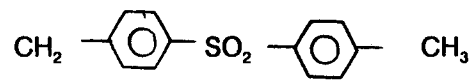 | |
| 744 | H | $CH_2OCH_3$ | $OCH_3$ | $CH_3$ | 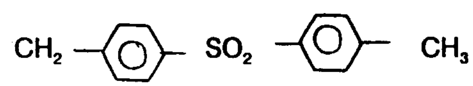 | |
| 745 | H | $C_2H_5$ | Cl | $CH_3$ | 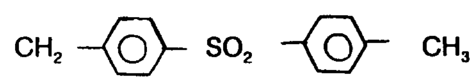 | |

| Bei-spiel Nr. | $R^1$ | $R^2$ | $R^3$ | $R^4$ | Q | Fp. |
|---|---|---|---|---|---|---|
| 746 | H | $-(CH_2)_4-$ | | $CH_3$ | $CH_2$ 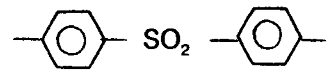 $SO_2$ $OCH_3$ | |
| 747 | H | $CH_2OCH_3$ | $OCH_3$ | $CH_3$ | $CH_2$ 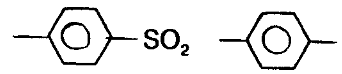 $SO_2$ $OCH_3$ | |
| 748 | H | $C_2H_5$ | Cl | $CH_3$ | $CH_2$ 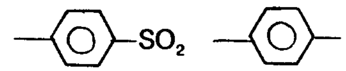 $SO_2$ $OCH_3$ | |
| 749 | H | $-(CH_2)_4-$ | | $CH_3$ | $CH_2$ 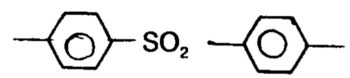 $SO_2$ $OCH_2CH_3$ | |
| 750 | H | $CH_2OCH_3$ | $OCH_3$ | $CH_3$ | $CH_2$ 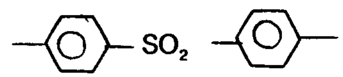 $SO_2$ $OCH_2CH_3$ | |
| 751 | H | $C_2H_5$ | Cl | $CH_3$ | $CH_2$ 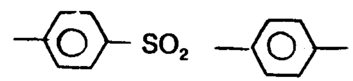 $SO_2$ $OCH_2CH_3$ | |
| 752 | H | $-(CH_2)_4-$ | | $CH_3$ | $CH_2$ 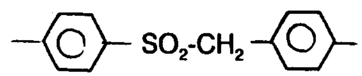 $SO_2$-$CH_2$ | |
| 753 | H | $CH_2OCH_3$ | $OCH_3$ | $CH_3$ | $CH_2$ 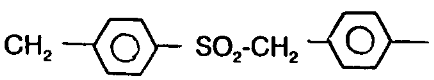 $SO_2$-$CH_2$ | |
| 754 | H | $C_2H_5$ | Cl | $CH_3$ | $CH_2$ 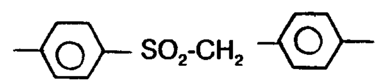 $SO_2$-$CH_2$ | |

| Beispiel Nr. | $R^1$ | $R^2$ | $R^3$ | $R^4$ | Q | Fp. |
|---|---|---|---|---|---|---|
| 755 | H | $-(CH_2)_4-$ | | $CH_3$ | $CH_2$ 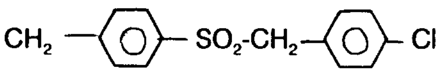 | |
| 756 | H | $CH_2OCH_3$ | $OCH_3$ | $CH_3$ | $CH_2$ 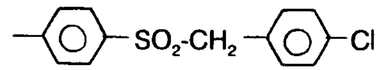 | |
| 757 | H | $C_2H_5$ | Cl | $CH_3$ | $CH_2$ 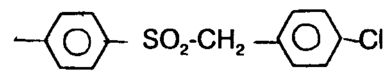 | |
| 758 | H | $-(CH_2)_4-$ | | $CH_3$ | $CH_2$ 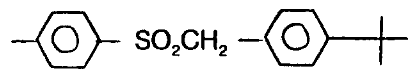 | |
| 759 | H | $CH_2OCH_3$ | $OCH_3$ | $CH_3$ | $CH_2$ 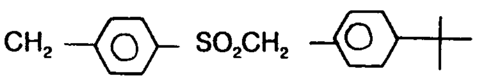 | |
| 760 | H | $C_2H_5$ | Cl | $CH_3$ | $CH_2$ 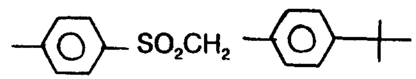 | |
| 761 | H | $-(CH_2)_4-$ | | H | $CH_2$ 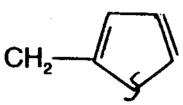 | Öl |
| 762 | H | $CH_2OCH_3$ | $OCH_3$ | H | $CH_2$ 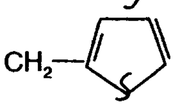 | Öl |
| 763 | H | $C_2H_5$ | Cl | H | $CH_2$ 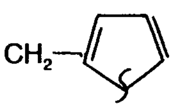 | Öl |

| Beispiel Nr. | $R^1$ | $R^2$ | $R^3$ | $R^4$ | Q | Fp. |
|---|---|---|---|---|---|---|
| 764 | H | $-(CH_2)_4-$ | | H | $CH_2$ 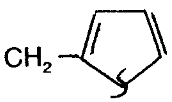 | 69°C |
| 765 | H | $-(CH_2)_4-$ | | H | $CH_2$ 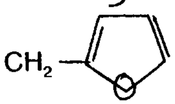 | |
| 766 | H | $CH_2OH_3$ | $OCH_3$ | H | $CH_2$ 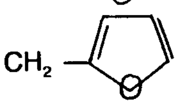 | |
| 767 | H | $C_2H_5$ | Cl | H | $CH_2$ 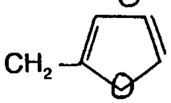 | |
| 768 | H | $-(CH_2)_4-$ | | H | $CH_2$ 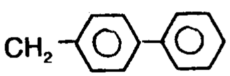 | Öl |
| 769 | H | $-CH_2(CH_3)-CH_2CH_2CH_2-$ | | H | $CH_2$ 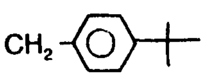 | Öl |
| 770 | H | $-(CH_2)_3-S-$ | | H | $CH_2$ 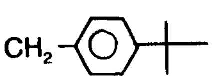 | 62-66°C |
| 771 | H | $-CH_2-S-CH_2-$ | | H | $CH_2$ 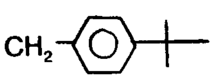 | Öl |
| 772 | H | $-CH_2CH_2-S-CH_2-$ | | H | $CH_2$ 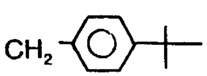 | Öl |

| Beispiel Nr. | $R^1$ | $R^2$ | $R^3$ | $R^4$ | Q | Fp. |
|---|---|---|---|---|---|---|
| 773 | H | $-(CH_2)_3-S-$ | | H | $CH_2$ 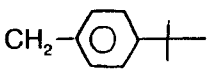 | 99-102°C |
| 774 | H | $-(CH_2)_2-S-CH_2-$ | | H | $CH_2$ 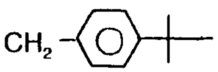 | 97-99°C |
| 775 | H | $-(CH_2)_4$ | | H | $CH_2$ 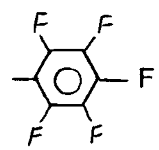 | |
| 776 | H | $CH_2OH_2$ | $OCH_3$ | H | $CH_2$ 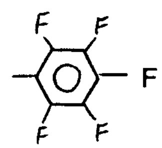 | |

| Bei-spiel Nr. | $R^1$ | $R^2$ | $R^3$ | $R^4$ | Q | Fp. |
|---|---|---|---|---|---|---|
| 777 | H | $C_2H_5$ | Cl | H | $CH_2$ 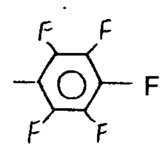 | |
| 778 | H | $-(CH_2)_4-$ | | H | $CH_2$ 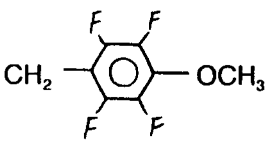 | |
| 779 | H | $CH_2OCH_3$ | $OCH_3$ | H | $CH_2$ 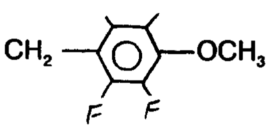 | |

| Beispiel Nr. | $R^1$ | $R^2$ | $R^3$ | $R^4$ | Q | Fp. |
|---|---|---|---|---|---|---|
| 780 | H | $C_2H_5$ | Cl | H | 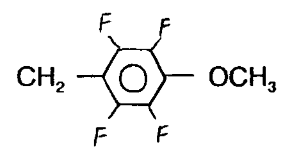 | |
| 781 | H | $-(CH_2)_4-$ | | H | 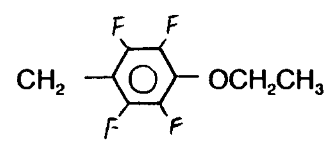 | |
| 782 | H | $CH_2OCH_3$ | $OCH_3$ | H | 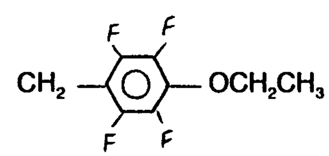 | |

| Beispiel Nr. | $R^1$ | $R^2$ | $R^3$ | $R^4$ | Q | Fp. |
|---|---|---|---|---|---|---|
| 783 | H | $C_2H_5$ | Cl | H | 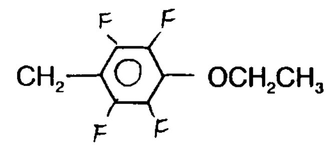 | |
| 784 | H | $-(CH_2)_4-$ | | H | 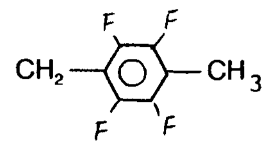 | |
| 785 | H | $CH_2OCH_3$ | $OCH_3$ | H | 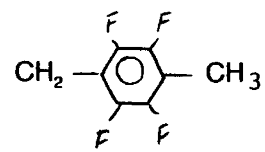 | |

| Bei-spiel Nr. | $R^1$ | $R^2$ | $R^3$ | $R^4$ | Q | Fp. |
|---|---|---|---|---|---|---|
| 786 | H | $C_2H_5$ | Cl | H | 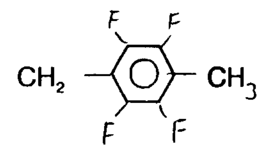 | |
| 787 | H | $-(CH_2)_4-$ | | H | 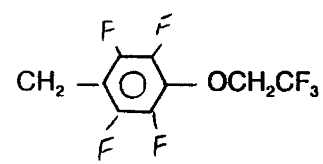 | |
| 788 | H | $CH_2OCH_3$ | $OCH_3$ | H | 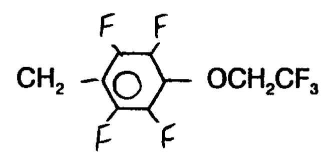 | |

| Beispiel Nr. | $R^1$ | $R^2$ | $R^3$ | $R^4$ | Q | Fp. |
|---|---|---|---|---|---|---|
| 789 | H | $C_2H_5$ | Cl | H | 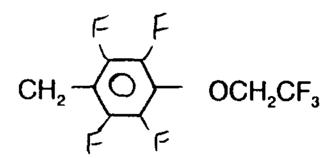 | |
| 790 | H | $-(CH_2)_4-$ | | H | 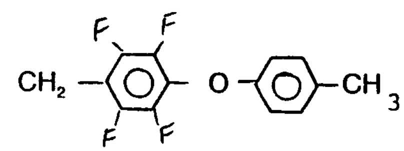 | |
| 791 | H | $CH_2OCH_3$ | $OCH_3$ | H | 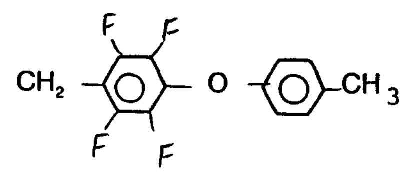 | |

| Bei-spiel Nr. | $R^1$ | $R^2$ | $R^3$ | $R^4$ | Q | Fp. |
|---|---|---|---|---|---|---|
| 792 | H | $C_2H_5$ | Cl | H | $CH_2$ 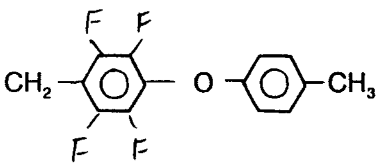 | |
| 793 | H | $-(CH_2)_4-$ | | $CH_3$ | $CH_2$ 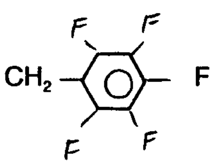 | |
| 794 | H | $CH_2OCH_3$ | $OCH_3$ | $CH_3$ | $CH_2$ 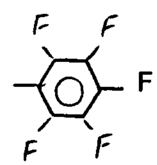 | |

| Beispiel Nr. | $R^1$ | $R^2$ | $R^3$ | $R^4$ | Q | Fp. |
|---|---|---|---|---|---|---|
| 795 | H | $C_2H_5$ | Cl | $CH_3$ | 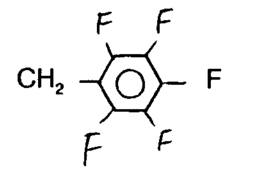 | |
| 796 | H | $-(CH_2)_4-$ | | $CH_3$ | 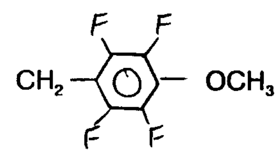 | |
| 797 | H | $CH_2OCH_3$ | $OCH_3$ | $CH_3$ | 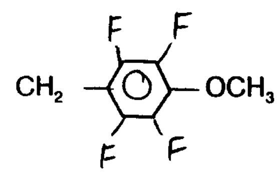 | |

| Beispiel Nr. | $R^1$ | $R^2$ | $R^3$ | $R^4$ | Q | Fp. |
|---|---|---|---|---|---|---|
| 798 | H | $C_2H_5$ | Cl | $CH_3$ | 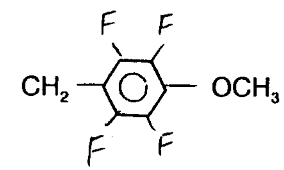 | |
| 799 | H | $-(CH_2)_4-$ | | $CH_3$ | 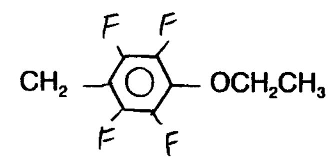 | |
| 800 | H | $CH_2OCH_3$ | $OCH_3$ | $CH_3$ | 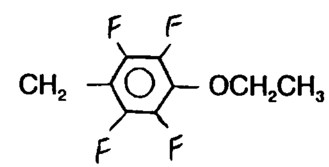 | |

| Beispiel Nr. | $R^1$ | $R^2$ | $R^3$ | $R^4$ | Q | Fp. |
|---|---|---|---|---|---|---|
| 801 | H | $C_2H_5$ | Cl | $CH_3$ | 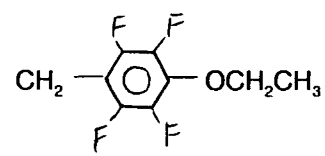 | |
| 802 | H | $-(CH_2)_4-$ | | $CH_3$ | 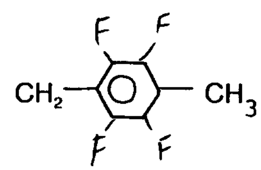 | |
| 803 | H | $CH_2OCH_3$ | $OCH_3$ | $CH_3$ | 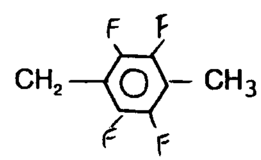 | |

| Bei-spiel Nr. | $R^1$ | $R^2$ | $R^3$ | $R^4$ | Q | Fp. |
|---|---|---|---|---|---|---|
| 804 | H | $C_2H_5$ | Cl | $CH_3$ | 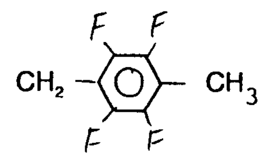 | |
| 805 | H | $-(CH_2)_4-$ | | $CH_3$ | 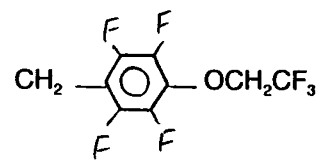 | |
| 806 | H | $CH_2OCH_3$ | $OCH_3$ | $CH_3$ | 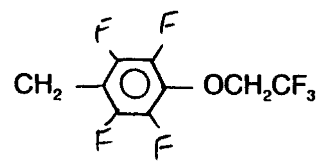 | |

| Beispiel Nr. | $R^1$ | $R^2$ | $R^3$ | $R^4$ | Q | Fp. |
|---|---|---|---|---|---|---|
| 807 | H | $C_2H_5$ | Cl | $CH_3$ | 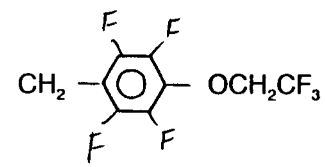 | |
| 808 | H | $-(CH_2)_4-$ | | $CH_3$ | 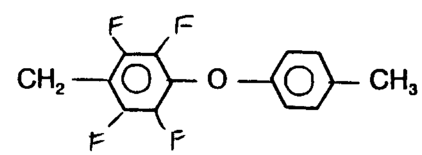 | |
| 809 | H | $CH_2OCH_3$ | $OCH_3$ | $CH_3$ | 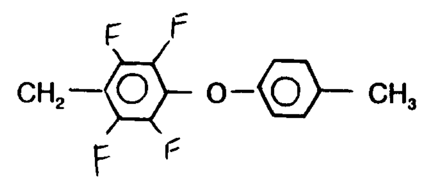 | |

| Beispiel Nr. | $R^1$ | $R^2$ | $R^3$ | $R^4$ | Q | Fp. |
|---|---|---|---|---|---|---|
| 810 | H | $C_2H_5$ | Cl | $CH_3$ | 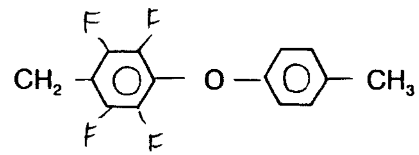 | |
| 811 | H | $-(CH_2)_4-$ | | H | 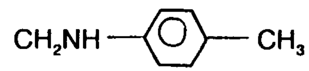 | Öl |
| 812 | H | $CH_2OCH_3$ | $OCH_3$ | H | 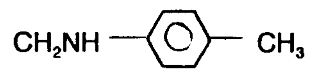 | Öl |
| 813 | H | $C_2H_5$ | Cl | H | 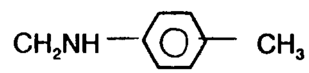 | Öl |
| 814 | H | $-(CH_2)_4-$ | | H | 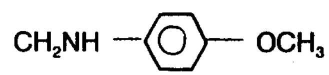 | 71-73°C |
| 815 | H | $CH_2OCH_3$ | $OCH_3$ | H | 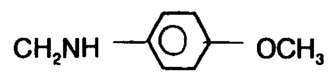 | Öl |
| 816 | H | $C_2H_5$ | Cl | H | 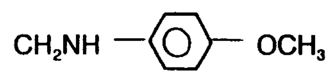 | Öl |

| Beispiel Nr. | $R^1$ | $R^2$ | $R^3$ | $R^4$ | Q | Fp. |
|---|---|---|---|---|---|---|
| 817 | H | $-(CH_2)_4-$ | | H | $CH_2NH$ 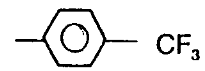 | 142-144°C |
| 818 | H | $CH_2OCH_3$ | $OCH_3$ | H | $CH_2NH$ 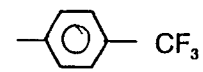 | Öl |
| 819 | H | $C_2H_5$ | Cl | H | $CH_2NH$ 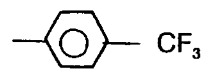 | Öl |
| 820 | H | $-(CH_2)_4-$ | | H | $CH_2NH$ 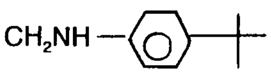 | Öl |
| 821 | H | $CH_2OCH_3$ | $OCH_3$ | H | $CH_2NH$ 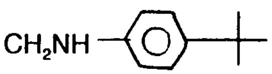 | Öl |
| 822 | H | $C_2H_5$ | Cl | H | $CH_2NH$ 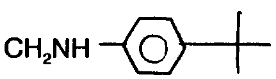 | Öl |
| 823 | H | $-(CH_2)_4-$ | | H | $CH_2$-NH 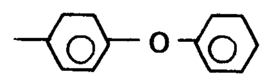 | 123-125°C |
| 824 | H | $CH_2OCH_3$ | $OCH_3$ | H | $CH_2$-NH 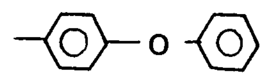 | Öl |
| 825 | H | $C_2H_5$ | Cl | H | $CH_2$-NH 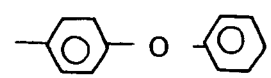 | Öl |

| Beispiel Nr. | $R^1$ | $R^2$ | $R^3$ | $R^4$ | Q | Fp. |
|---|---|---|---|---|---|---|
| 826 | H | $-(CH_2)_4-$ | | H | $CH_2$-NH 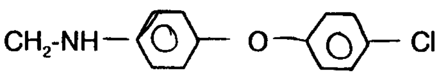 | 108-110°C |
| 827 | H | $CH_2OCH_3$ | $OCH_3$ | H | $CH_2$-NH 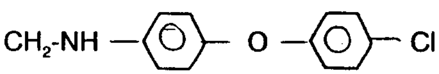 | Öl |
| 828 | H | $C_2H_5$ | Cl | H | $CH_2$-NH 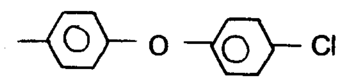 | Öl |
| 829 | H | $-(CH_2)_4-$ | | H | $CH_2$-NH 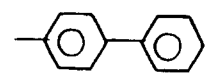 | 102°C |
| 830 | H | $CH_2OCH_3$ | $OCH_3$ | H | $CH_2$-NH 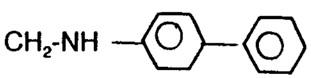 | Öl |
| 831 | H | $C_2H_5$ | Cl | H | $CH_2$-NH 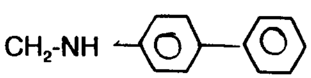 | Öln |

Tabelle II beinhaltet Verbindungen der Formel

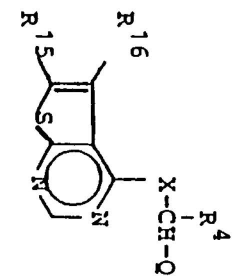

,

die sich von Formel I herleitet.

Tabelle II

| Beisp. Nr. | $R^{15}$ | $R^{16}$ | $R^4$ | X | Q | Kp [°C] |
|---|---|---|---|---|---|---|
| 832 | $CH_3$ | H | H | O | 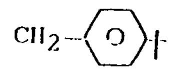 | Öl |
| 833 | $C_2H_5$ | H | H | O | 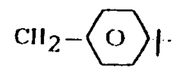 | Öl |
| 834 | $C_2H_5$ | H | H | O | 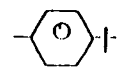 | Öl |
| 835 | $CH_3$ | $CH_3$ | H | O | 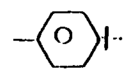 | Öl |
| 836 | $C_2H_5$ | H | H | O | 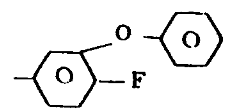 | Öl |
| 837 | $CH_3$ | $CH_3$ | H | O | 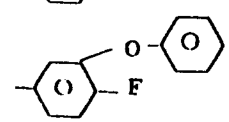 | Öl |
| 838 | $CH_3$ | H | H | O | 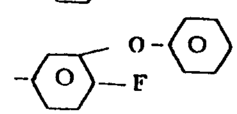 | Öl |

Tabelle III beinhaltet Verbindungen der Formel

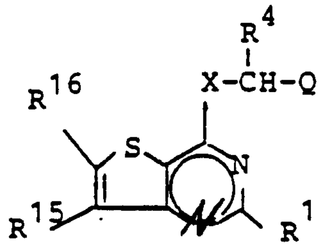

,

die sich von Formel I herleitet.

Tabelle III

| Beisp. Nr. | $R^1$ | $R^{15}$ | $R^{16}$ | $R^4$ | X | Q | Kp [°C] |
|---|---|---|---|---|---|---|---|
| 839 | H | H | H | H | O | $(CH_2)_6CH_3$ | Öl |
| 840 | H | H | H | H | O | 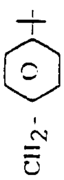 | Öl |
| 841 | H | H | H | H | O | 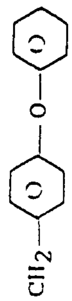 | Öl |
| 842 | H | H | H | H | O | 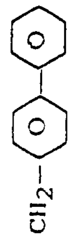 | Öl |
| 843 | H | H | H | H | O | 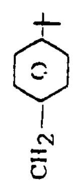 | Öl |

C) Biologische Beispiele

Beispiel 1

Gerstenpflanzen wurden im 3-Blattstadium mit Konidien des Gerstenmehltaus (Erysiphe graminis f. sp. hordei) stark inokuliert und in einem Gewächshaus bei 20 ° C und einer relativen Luftfeuchte von 90 - 95% aufgestellt. 24 Stunden nach Inokulation wurden die Pflanzen mit den in Tabelle IV aufgeführten Verbindungen in den angegebenen Wirkstoffkonzentrationen gleichmäßig benetzt. Nach einer Inkubationszeit von 10 Tagen wurden die Pflanzen auf Befall mit Gerstenmehltau untersucht. Der Befallsgrad wurde ausgedrückt in % befallener Blattfläche, bezogen auf unbehandelte, infizierte Kontrollpflanzen ( = 100% Befall). Das Ergebnis ist in Tabelle IV zusammengefaßt.

Tabelle IV

| Verbindungen gemäß Beispiel Nr. | mit Gerstenmehltau befallene Blattfläche in % bei mg Wirkstoff/Liter Spritzbrühe | |
|---|---|---|
| | 500 | 250 |
| 4 | 0 | 0 |
| 34 | 0 | 0 |
| 40 | 0 | 0 |
| 43 | 0 | 0 |
| 45 | 0 | 0 |
| 46 | 0 | 0 |
| 65 | 0 | 3 |
| 66 | 0 | 5 |
| 72 | 0 | 0 |
| 73 | 0 | 0 |
| 74 | 0 | 0 |
| 75 | 0 | 0 |
| 76 | 0 | 5 |
| 77 | 0 | 0 |
| 78 | 0 | 0 |
| unbehandelte, infizierte Pflanzen | 100 | |

Beispiel 2

Weizenpflanzen der Sorte "Jubilar" wurden im 2-Blattstadium mit wäßrigen Suspensionen der in Tabelle V angegebenen Präparate tropfnaß behandelt.

Nach dem Antrocknen des Spritzbelages wurden die Pflanzen mit einer wäßrigen Pyknosporen-Suspension von Leptosphaeria nodorum inokuliert und mehrere Stunden bei 100% rel. Luftfeuchte in einer Klimakammer inkubiert. Bis zur Symptonausprägung wurden die Pflanzen im Gewächshaus bei ca. 90% rel. Luftfeuchte weiterkultiviert.

Der Befallsgrad wurde in % befallener Blattfläche im Vergleich zu unbehandelten, infizierten Kontrollpflanzen ausgedrückt. Das Ergebnis ist in Tabelle V zusammengefaßt.

Tabelle V

| Verbindungen gemäß Beispiel Nr. | mit Leptosphaeria nodorum befallene Blattfläche in % bei mg Wirkstoff/Liter Spritzbrühe | | |
|---|---|---|---|
| | 500 | 250 | 125 |
| 5 | 0 | 3 | 5 |
| 41 | 0 | 0 | 0 |
| 61 | 0 | 3 | - |
| 64 | 0 | 0 | 3 |
| 72 | 0 | 0 | - |
| 74 | 0 | 0 | 0 |
| 75 | 0 | 0 | 0 |
| 76 | 0 | 0 | 0 |
| 78 | 0 | 0 | 5 |
| 83 | 0 | 5 | - |
| 100 | 0 | 5 | - |
| unbehandelte, infizierte Pflanzen | 100 | | |

Beispiel 3

Gerstenpflanzen der Sorte "Igri" wurden im 2-Blatt-Stadium mit einer wäßrigen Suspension der beanspruchten Verbindungen tropfnaß behandelt.

Nach dem Antrocknen des Spritzbelages wurden die Pflanzen mit einer wäßrigen Sporensuspension von Pyrenophora teres inokuliert und für 16 h in einer Klimakammer bei 100% rel. Luftfeuchte inkubiert. Anschließend wurden die infizierten Pflanzen im Gewächshaus bei 25°C und 80% rel. Luftfeuchte weiter kultiviert.

Ca. 1 Woche nach Inokubation wurde der Befall ausgewertet. Der Befallsgrad wurde in % befallener Blattfläche im Vergleich zur unbehandelten, infizierten Kontrolle bonitiert und ist in Tabelle VI wiedergegeben.

Tabelle VI

| Verbindungen gemäß Beispiel Nr. | % Pyrenophora teres befallene Blattfläche in % bei mg Wirkstoff/Liter Spritzbrühe | | |
|---|---|---|---|
| | 500 | 250 | 125 |
| 3 | 0 | 0 | - |
| 4 | 0 | 0 | 5 |
| 5 | 0 | 0 | 0 |
| 15 | 0 | 0 | 0 |
| 34 | 0 | 0 | 0 |
| 42 | 0 | 0 | - |
| 66 | 0 | 0 | 0 |
| 67 | 0 | 0 | 3 |
| 74 | 0 | 0 | 0 |
| 75 | 0 | 0 | 0 |
| 76 | 0 | 0 | 0 |
| 77 | 0 | 0 | 0 |
| 81 | 0 | 0 | 5 |
| 82 | 0 | 0 | 0 |
| 98 | 0 | 0 | 0 |
| unbehandelte, infizierte Pflanzen | 100 | | |

Beispiel 4

Weizen der Sorte "Jubilar" wurde im 2-Blatt-Stadium mit wäßrigen Suspensionen der beanspruchten Verbindungen tropfnaß behandelt.

Nach dem Antrocknen des Spritzbelages wurden die Pflanzen mit einer wäßrigen Sporensuspensionen von Puccinia recondita inokuliert. Die Pflanzen wurden für ca. 16 Stunden tropfnaß in eine Klimakammer mit 20°C und ca. 100% rel. Luftfeuchte gestellt. Anschließend wurden die infizierten Pflanzen in einem Gewächshaus bei einer Temperatur von 22 - 25°C und 50 - 70% rel. Luftfeuchte weiterkultiviert.

Nach einer Inkubationszeit von ca. 2 Wochen sporulierte der Pilz auf der gesamten Blattoberfläche der nicht behandelten Kontrollpflanzen, so daß eine Befallsauswertung der Versuchspflanzen vorgenommen werden konnte. Der Befallsgrad wurde in % befallener Blattfläche im Vergleich zu unbehandelten, infizierten Kontrollpflanzen ausgedrückt und ist in Tabelle VII wiedergegeben.

Tabelle VII

| Verbindungen gemäß Beispiel Nr. | mit Puccinia recondita befallene Blattfläche in % bei mg Wirkstoff/Liter Spritzbrühe | | |
|---|---|---|---|
| | 500 | 250 | 125 |
| 1 | 0 | 0 | - |
| 4 | 0 | 0 | - |
| 5 | 0 | 0 | 0 |
| 8 | 0 | 0 | - |
| 16 | 0 | 0 | - |
| 31 | 0 | 0 | - |
| 61 | 0 | 0 | 5 |
| 63 | 0 | 5 | 10 |
| 64 | 0 | - | - |
| 65 | 0 | 0 | 0 |
| 77 | 0 | 0 | - |
| 78 | 0 | 0 | - |
| 99 | 0 | 0 | - |

Beispiel 5

Mit Bohnenspinnmilben (Tetranychus urticae, Vollpopulation) stark befallene Bohnenpflanzen (Phaseolus v.) wurden mit der wäßrigen Verdünnung eines Spritzpulverkonzentrates, das 250 ppm des jeweiligen Wirkstoffes enthielt, gespritzt. Die Mortalität der Milben wurden nach 7 Tagen kontrolliert. 100% Mortalität wurde mit den Verbindungen gemäß Beispiel 4, 5, 14, 23, 42, 43, 46, 47, 49, 50, 51, 65, 69, 73, 74, 75, 82, 90, 92, 95, 100, 102 und 840 erreicht.

Beispiel 6

Mit schwarzer Bohnenblattlaus (Aphis fabae) stark besetzte Ackerbohnen (Vicia faba) wurden mit wäßrigen Verdünnungen von Spritzpulverkonzentraten mit 250 ppm Wirkstoffgehalt bis zum Stadium des beginnenden Abtropfens besprüht. Die Mortalität der Blattläuse wurde nach 3 Tagen bestimmt. Eine 100%ige Mortalität konnte mit den Verbindungen gemäß Beispiel 4, 5, 42, 46, 49, 65, 66, 73, 74, 75, 77, 87 und 102 erzielt werden.

Beispiel 7

Auf die Innenseite des Deckels und des Bodens einer Petrischale wurden jeweils 1 ml der zu testenden Formulierung emulgiert, in Wasser gleichmäßig aufgetragen und nach dem Antrocknen des Belages jeweils 10 Imagines der Hausfliege (Musca domestica) eingegeben. Nach dem Verschließen der Schalen wurden diese bei Raumtemperatur aufbewahrt und nach 3 Stunden die Mortalität der Versuchstiere bestimmt. Bei 250 ppm (bezogen auf den Gehalt an Wirkstoff) zeigen die Präparate 4, 15, 16, 46, 49, 59, 63, 72, 73, 77, 78, 79, 98, 99 und 841 eine gute Wirkung (100% Mortalität) gegenüber der Stubenfliege.

Beispiel 8

Filterpapierscheiben mit aufliegenden Eiern von Baumwollwanzen (Oncopeltus fasciatus) wurden mit jeweils 0,5 ml wäßriger Verdünnung der zu testenden Formulierung behandelt. Nach Antrocknen des Belages wurde die Petrischale verschlossen und der Innenraum auf max. Luftfeuchtigkeit gehalten. Nach Aufbewahrung bei Raumtemperatur wurde nach 7 Tagen die ovizide Wirkung ermittelt. Mit 250 ppm Wirkstoffgehalt wurde für die Verbindungen gemäß Beispiel 1, 2, 4, 5, 14, 15, 16, 30, 33, 34, 35, 40, 41, 43, 45 bis 50, 53, 58, 59, 61, 64, 65, 66, 72, 73, 74, 76, 77, 83, 84, 87, 89, 90, 95, 102, 839 und 841 100% Mortalität erzielt.

## Patentansprüche

1. Substituierte 4-Alkoxypyrimidine der allgemeinen Formel I

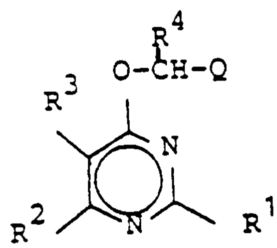

worin

| | |
|---|---|
| $R^1$ | Wasserstoff, Halogen, $(C_1$-$C_4)$Alkyl oder $(C_3$-$C_6)$Cycloalkyl bedeutet, |
| $R^2$ | Wasserstoff, $(C_1$-$C_4)$Alkyl, Halogen, $(C_1$-$C_4)$Halogenalkyl, $(C_1$-$C_{10})$Alkoxy, Phenyl-$(C_1$-$C_4)$alkoxy, $(C_1$-$C_{10})$Alkoxy-$(C_1$-$C_{10})$alkoxy, Benzyloxy-$(C_1$-$C_{10})$alkoxy, $(C_1$-$C_{10})$-Halogenalkoxy, $(C_1$-$C_{10})$Alkoxy-$(C_1$-$C_{10})$alkyl, $(C_1$-$C_{10})$Alkylthio, $(C_1$-$C_{10})$Alkylthio-$(C_1$-$C_{10})$alkyl, $(C_1$-$C_{10})$Alkylamino oder Di-$(C_1$-$C_{10})$alkylamino bedeutet, wobei in den genannten Resten Phenyl gegebenenfalls mit $(C_1$-$C_6)$Alkyl, $(C_1$-$C_6)$Alkoxy oder Halogen monosubstituiert ist, |
| $R^3$ | Wasserstoff, $(C_1$-$C_4)$Alkyl, $(C_1$-$C_4)$Alkoxy, $(C_1$-$C_4)$Halogenalkoxy, Halogen, $(C_1$-$C_4)$-Alkylthio, Amino, $(C_1$-$C_4)$Alkylamino, $(C_1$-$C_4)$Dialkylamino bedeutet oder |
| $R^2$ und $R^3$ | zusammen mit dem Kohlenstoffatom, an das sie gebunden sind, einen ungesättigten 5-gliedrigen Ring bilden, der ein Sauerstoff oder Schwefelatom enthält und der gegebenenfalls durch Alkyl oder Halogen substituiert ist, oder |
| $R^2$ und $R^3$ | zusammen mit dem Kohlenstoffatom, an das sie gebunden sind, einen gesättigten 5-, 6- oder 7-gliedrigen Ring bilden, der ein Sauerstoff- oder Schwefelatom enthalten kann und der gegebenenfalls durch Alkyl substituiert ist, |
| $R^4$ | Wasserstoff, $(C_1$-$C_4)$Alkyl, $(C_3$-$C_6)$Cycloalkyl oder $(C_1$-$C_4)$-Halogenalkyl, wie $CF_3$, bedeutet, |
| Q | die Bedeutung $Q^1$ hat und |
| $Q^1$ | $(C_1$-$C_{15})$Alkyl bedeutet, welches bis zu dreifach ungesättigt sein kann und welches, gegebenenfalls substituiert ist mit einem, zwei oder drei Halogenatomen, einer $(C_3$-$C_8)$Cycloalkylgruppe, einer $(C_1$-$C_{15})$Alkoxygruppe, einer $(C_1$-$C_{15})$Alkoxy-$(C_1$-$C_{15})$-Alkoxygruppe, einer $(C_1$-$C_{15})$Alkylthiogruppe, einer $(C_1$-$C_{15})$Alkylsulfinylgruppe, einer $(C_1$-$C_{15})$Alkylsulfonylgruppe, einer $(C_1$-$C_{15})$-Alkanoylgruppe, einer $(C_4$-$C_8)$-Cycloalkylalkoxygruppe, einer $(C_4$-$C_8)$Cycloalkylalkylthiogruppe, einer Benzyloxy- oder einer Phenoxybenzyloxygruppe, oder |
| Q | die Bedeutung $Q^2$ hat und |
| $Q^2$ | eine Gruppe der allgemeinen Formel IIa-IIj bedeutet |

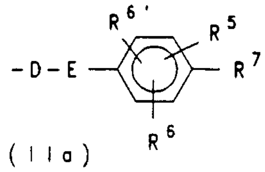
(IIa)

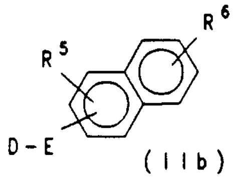
(IIb)

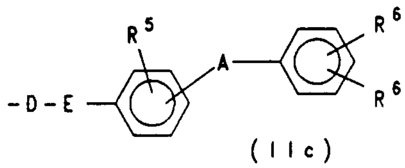
(IIc)

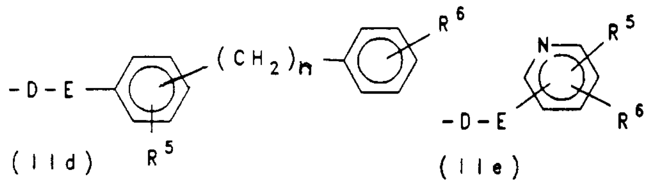
(IId) (IIe)

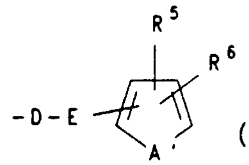
(IIf)

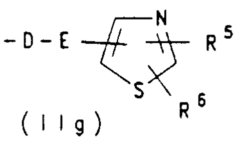
(IIg)

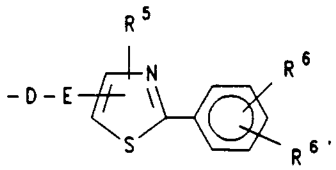
(IIh)

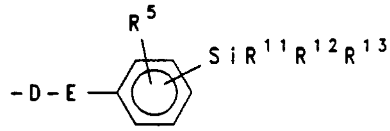
(IIi)

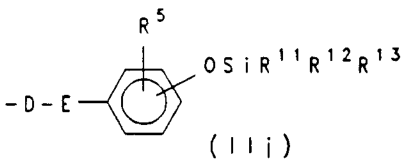
(IIj)

worin

| | |
|---|---|
| n | 0, 1 oder 2 ist |
| A | Sauerstoff, $-O-CH_2-$, Schwefel, Sulfinyl oder Sulfonyl bedeutet, |
| A' | Sauerstoff oder Schwefel bedeutet, |
| D | eine direkte Bindung oder eine $(C_1-C_6)$Alkylengruppe bedeutet, |
| E | eine direkte Bindung, sowie für den Fall, daß D eine Alkylengruppe ist, Sauerstoff oder Imino bedeutet, |
| $R^5$, $R^6$ und $R^{6'}$ | gleich oder verschieden sind und jeweils Wasserstoff, Halogen, $(C_1-C_8)$Alkyl, $(C_2-C_8)$-Alkenyl, $(C_2-C_8)$-Alkinyl, $(C_3-C_6)$Cycloalkyl, $(C_1-C_8)$-Halogenalkyl, $(C_1-C_8)$Alkoxy, $(C_1-C_8)$Alkylthio, $(C_1-C_8)$Alkylsulfinyl, $(C_1-C_8)$Alkylsulfonyl, $(C_1-C_4)$Dialkylamino oder Nitro bedeuten, wobei für den Fall, daß $R^5$, $R^6$, $R^{6'}$ Alkylreste bedeuten, diese auch cyclisch verknüpft sein können, |
| $R^7$ die für $R^5$, $R^6$ und $R^{6'}$ | angegebene Bedeutung hat, sowie für den Fall, daß E eine direkte Bindung bedeutet, $(C_1-C_4)$Alkoxy-$(C_1-C_4)$alkyl, $(C_1-C_4)$Alkoxycarbonyl, $(C_1-C_8)$Alkanoyl oder eine Gruppe der allgemeinen Formel III bedeutet, |

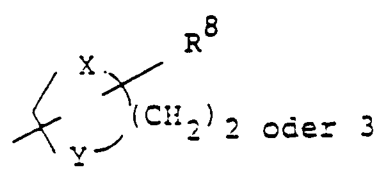
(III),

worin X und Y gleich oder verschieden sind und jeweils Schwefel oder Sauerstoff bedeuten und

| | |
|---|---|
| $R^8$ | Wasserstoff oder $(C_1-C_4)$Alkyl bedeutet, oder |
| Q | die Bedeutung $Q^3$ hat und |
| $Q^3$ | falls von der vorstehenden Formel IId nicht umfaßt, eine Gruppe der allgemeinen Formel IV bedeutet, |

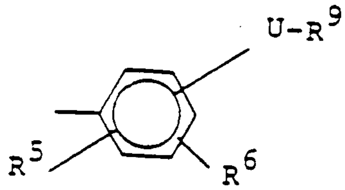

(IV),

worin $R^5$ und $R^6$ die oben angegebenen Bedeutungen haben,

U eine direkte Bindung, Sauerstoff, Schwefel, Sulfinyl, Sulfonyl oder Methylen bedeutet,

$R^9$ Phenyl oder einen Heterocyclen-Rest bedeutet, wobei jeder der beiden vorgenannten Reste unsubstituiert oder mit einem oder zwei Substituenten versehen sein kann und diese Substituenten gleich oder verschieden sind und jeweils Halogen, $(C_1$-$C_4)$Alkyl, Trifluormethyl, Nitro, $(C_1$-$C_4)$Alkoxy, $(C_1$-$C_4)$Alkylthio, $(C_1$-$C_4)$-Alkylsulfinyl, $(C_1$-$C_4)$-Alkylsulfonyl, $(C_1$-$C_4)$Alkoxy-$(C_1$-$C_4)$alkyl, Phenyl, Phenoxy, $(C_1$-$C_4)$-Alkoxyphenoxy, Halogenphenoxy oder $(C_1$-$C_4)$Alkylphenoxy bedeuten,

$R^9$ weiterhin, für den Fall, daß U Sauerstoff bedeutet, $(C_1$-$C_4)$Halogenalkyl oder eine Gruppe der allgemeinen Formel V bedeutet

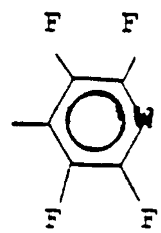

(V),

worin

W Stickstoff oder eine Gruppe $CR^{10}$ bedeutet, worin

$R^{10}$ Wasserstoff, Fluor, Cyano, Formyl, Acetyl, Nitro, Methyl, Methoxy oder 1,3-Dioxolan-2-yl bedeutet,

$R^{11}$, $R^{12}$ und $R^{13}$ gleich oder verschieden sind und $(C_1$-$C_{15})$Alkyl, das gegebenenfalls ein- oder mehrfach halogensubstituiert ist,

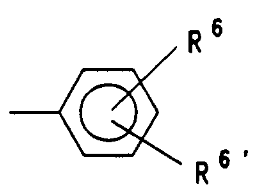

oder

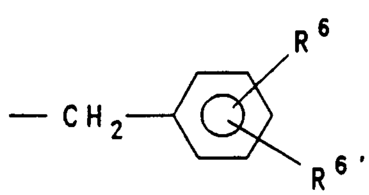

bedeutet, worin $R^6$ und $R^{6'}$ wie oben definiert sind, sowie ihre Salze und ihre Stereosiomeren.

**2.** Verbindungen gemäß Anspruch 1, dadurch gekennzeichnet, daß in Formel I
$R^1$ Wasserstoff, Chlor oder Methyl bedeutet,
$R^2$ $(C_1-C_4)$Alkyl, Chlor, Trifluormethyl, Methoxy, Ethoxy oder Methoxymethyl bedeutet,
$R^3$ Wasserstoff, $(C_1-C_3)$Alkyl, Methoxy, Ethoxy oder Halogen bedeutet oder
$R^2$ und $R^3$ zusammen mit dem Kohlenstoffatom, an das sie gebunden sind, einen ungesättigten 5-gliedrigen Ring bilden, der ein Sauerstoff- oder Schwefelatom enthält oder $R^2$ und $R^3$ zusammen mit dem Kohlenstoffatom, an das sie gebunden sind, einen gesättigten 5- oder 6-gliedrigen Ring bilden, der ein Schwefelatom enthalten kann,
$R^4$ Wasserstoff, Methyl, Ethyl, Trifluormethyl oder Cyclopropyl bedeutet,
Q die Bedeutung $Q^1$, $Q^2$ oder $Q^3$ hat.

**3.** Verbindungen gemäß Anspruch 1, dadurch gekennzeichnet, daß in Formel I
$R^1$ Wasserstoff oder Methyl bedeutet,
$R^2$ Methyl, Ethyl, Trifluormethyl, Methoxy, Ethoxy oder Methoxymethyl bedeutet,
$R^3$ Methyl, Ethyl, Methoxy, Chlor oder Brom bedeutet oder
$R^2$ und $R^3$ zusammen mit dem Kohlenstoffatom, an das sie gebunden sind, einen gesättigten 5- oder 6-gliedrigen Ring bilden,
$R^4$ Wasserstoff, Methyl, Ethyl, Trifluormethyl oder Cyclopropyl bedeutet,
Q die Bedeutung $Q^1$, $Q^2$ oder $Q^3$ hat.

**4.** Verbindungen gemäß Anspruch 1, dadurch gekennzeichnet, daß in Formel I
$R^1$ Wasserstoff bedeutet,
$R^2$ Methyl, Ethyl oder Methoxymethyl bedeutet,
$R^3$ Chlor, Brom oder Methoxy bedeutet oder
$R^2$ und $R^3$ zusammen mit dem Kohlenstoffatom, an das sie gebunden sind, einen gesättigten 6-gliedrigen Ring bilden,
$R^4$ Wasserstoff, Methyl oder Ethyl bedeutet,
Q die Bedeutung $Q^1$, $Q^2$ oder $Q^3$ hat.

**5.** Verbindungen gemäß Anspruch 1, dadurch gekennzeichnet, daß in Formel I
$R^1$ Wasserstoff bedeutet,
$R^2$ Methoxymethyl und $R^3$ Methoxy oder
$R^2$ Methyl oder Ethyl und $R^3$ Chlor oder Brom bedeutet, oder
$R^2$ und $R^3$ zusammen mit dem Kohlenstoffatom, an das sie gebunden sind, einen gesättigten 6-gliedrigen Ring bilden,
$R^4$ Wasserstoff, Methyl oder Ethyl bedeutet,
Q die Bedeutung $Q^1$, $Q^2$ oder $Q^3$ hat.

**6.** Verbindungen gemäß Anspruch 1, dadurch gekennzeichnet, daß in Formel I
$R^1$ Wasserstoff bedeutet,
$R^2$ Methoxymethyl und $R^3$ Methoxy oder
$R^2$ Ethyl und $R^3$ Chlor bedeutet, oder
$R^2$ und $R^3$ zusammen mit dem Kohlenstoffatom, an das sie gebunden sind, einen gesättigten 6-gliedrigen Ring bilden,
$R^4$ Wasserstoff oder Methyl bedeutet,
$Q^1$ eine $(C_4-C_{10})$Alkylgruppe oder eine $(C_1-C_3)$Alkylgruppe, die mit einer Cyclohexylgruppe, $(C_4-C_8)$Cycloalkylalkoxygruppe oder einer $(C_4-C_8)$-Cycloalkylalkylthiogruppe substituiert ist, bedeutet.

**7.** Verbindungen gemäß Anspruch 1, dadurch gekennzeichnet, daß in Formel I
$R^1$ Wasserstoff bedeutet,
$R^2$ Methoxymethyl und $R^3$ Methoxy oder
$R^2$ Ethyl und $R^3$ Chlor bedeutet, oder
$R^2$ und $R^3$ zusammen mit den Kohlenstoffatom, an das sie gebunden sind, einen gesättigten 6-gliedrigen Ring bilden,
$R^4$ Wasserstoff oder Methyl bedeutet,
$Q^2$ eine Gruppe der allgemeinen Formeln IIa-IIj bedeutet, worin D eine Methylengruppe und E eine direkte Bindung bedeutet, $R^5$, $R^6$ und $R^{6'}$ Wasserstoff, $(C_1-C_8)$Alkyl, $(C_1$-

C4)Alkoxy, Cyclohexyl oder Trifluormethyl bedeuten,
$R^7$ Methoxy- oder Ethoxyethyl bedeutet.

**8.** Verbindungen gemäß Anspruch 1, dadurch gekennzeichnet, daß in Formel I

| | |
|---|---|
| $R^1$ | Wasserstoff bedeutet, |
| $R^2$ | Methoxymethyl und $R^3$ Methoxy oder |
| $R^2$ | Ethyl und $R^3$ Chlor bedeutet, oder |
| $R^2$ und $R^3$ | zusammen mit dem Kohlenstoffatom, an das sie gebunden sind, einen gesättigten 5- oder 6-gliedrigen Ring bilden, |
| $R^4$ | Methyl, Ethyl oder Cyclopropyl bedeutet, |
| Q | die Bedeutung $Q^3$ hat, |
| $R^5$ und $R^6$ | Wasserstoff bedeuten, |
| U | Sauerstoff bedeutet, |
| $R^9$ | Phenyl oder einen Heterocyclus bedeutet wobei jeder der beiden vorgenannten Reste unsubstituiert oder mit einem oder zwei Substituenten versehen sein kann und diese Substituenten gleich oder verschieden sind und jeweils Halogen, $(C_1$-$C_4)$Alkyl, Trifluormethyl, Nitro, $(C_1$-$C_4)$Alkoxy, $(C_1$-$C_4)$Alkylthio oder $(C_1$-$C_4)$Alkoxy-$(C_1$-$C_4)$alkyl bedeuten. |

**9.** Verfahren zur Herstellung von Verbindungen der Formel I gemäß Anspruch 1, dadurch gekennzeichnet, daß man eine Verbindung der allgemeinen Formel VI

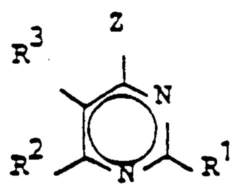

(VI),

worin $R^1$, $R^2$ und $R^3$ die unter Formel I angegebenen Bedeutungen haben, und Z eine Abgangsgruppe bedeutet, mit einem Alkohol der allgemeinen Formel VII

$$\begin{array}{c} R^4 \\ | \\ HO - CH - Q \end{array} \qquad (VII),$$

worin $R^4$ und Q die unter Formel I nach Anspruch 1 angegebenen Bedeutungen haben, umsetzt und die so erhaltenen Verbindungen der Formel I gegebenenfalls am Schwefel einer Thioether-Seitenkette oxidiert oder am $C_5$-Atom des Pyrimidins chloriert oder bromiert, und die so erhaltenen Verbindungen gegebenenfalls in ihre Salze überführt.

**10.** Verfahren zur Herstellung von Verbindungen der allgemeinen Formel I',

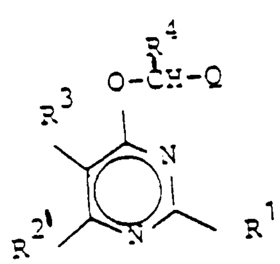

(I'),

worin $R^1$, $R^3$, $R^4$ und Q die unter Formel I nach Anspruch 1 angegebenen Bedeutungen haben und $R^{2'}$ -($C_1$-$C_4$)Alkoxy, ($C_1$-$C_4$)Halogenalkoxy, ($C_1$-$C_4$)Alkylthio, ($C_1$-$C_4$)Alkylamino oder ($C_1$-$C_4$)Dialkylamino bedeutet, dadurch gekennzeichnet, daß man eine Verbindung der Formel VIII

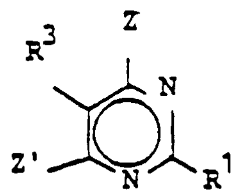

(VIII),

worin Z und Z' gleich oder verschieden sein können, und eine Abgangsgruppe bedeuten, und $R^1$ und $R^3$ die unter Formel I' angegebenen Bedeutungen haben, mit einer Verbindung der Formel VII

$$\begin{array}{c} R^4 \\ | \\ HO - CH - Q \end{array} \qquad (VII),$$

worin $R^4$ und Q die unter Formel I' angegebene Bedeutung hat, zu einer Verbindung der Formel (IX)

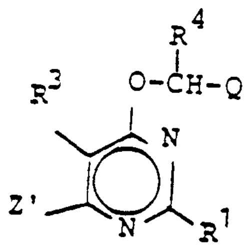

(IX),

worin $R^1$, $R^3$, $R^4$, Q und Z' die oben angegebenen Bedeutungen haben, umsetzt und in einem zweiten Reaktionsschritt die Verbindung der Formel VIII mit einer Verbindung der Formel X umsetzt

$HR^{2'}$ (X),

worin $R^{2'}$ die oben angegebene Bedeutung hat, und die so erhaltene Verbindung der Formel I' gegebenenfalls am Schwefel einer Thioether-Seitenkette oxidiert oder gegebenenfalls, falls $R^3$ Wasserstoff bedeutet, chloriert oder bromiert, und die so erhaltenen Verbindungen gegebenenfalls in ihre Salze überführt.

**11.** Verfahren zur Herstellung einer Verbindung der Formel I' gemäß Anspruch 10, dadurch gekennzeichnet, daß man eine Verbindung der Formel VIII mit einer Verbindung der Formel X zu einer Verbindung der Formel XI

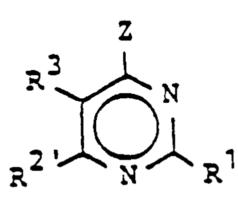

(XI),

worin $R^1$ und $R^3$ die unter Formel I' angegebenen Bedeutungen haben, $R^{2'}$ ($C_1$-$C_4$)Alkoxy, ($C_1$-$C_4$)-Halogenalkoxy, ($C_1$-$C_4$)Alkylthio, ($C_1$-$C_4$)Alkylamino oder ($C_1$-$C_4$)Dialkylamino bedeutet und Z die unter

Formel VI angegebene Bedeutung hat, umsetzt, die Verbindung der Formel XI mit einem Alkohol der obengenannten Formel VII umsetzt und die so erhaltene Verbindung der Formel I' gegebenenfalls am Schwefel einer Thioether-Seitenkette oxidiert, oder, falls $R^3$ Wasserstoff bedeutet, chloriert oder bromiert, und die so erhaltenen Verbindungen in ihre Salze überführt.

**12.** Insektizide oder akarizide Mittel, dadurch gekennzeichnet, daß sie eine wirksame Menge einer Verbindung der Formel I nach Anspruch 1 enthalten.

**13.** Fungizide Mittel, dadurch gekennzeichnet, daß sie eine wirksame Menge einer Verbindung der Formel I nach Anspruch 1 enthalten.

**14.** Nematozide Mittel, dadurch gekennzeichnet, daß sie eine wirksame Menge einer Verbindung der Formel I nach Anspruch 1 enthalten.

**15.** Verwendung der Verbindungen der Formel I nach Anspruch 1 zur Bekämpfung von Schadinsekten und Akariden.

**16.** Verwendung der Verbindungen der Formel I nach Anspruch 1 zur Bekämpfung von Schadpilzen.

**17.** Verwendung der Verbindung der Formel I nach Anspruch 1 zur Bekämpfung von Nematoden.

**18.** Verfahren zur Bekämpfung von Schadinsekten und Akariden, dadurch gekennzeichnet, daß man auf diese oder die von ihnen befallenen Pflanzen, Flächen oder Substrate eine wirksame Menge einer Verbindung der Formel I nach Anspruch 1 appliziert.

**19.** Verfahren zur Bekämpfung von Schadpilzen, dadurch gekennzeichnet, daß man auf diese oder die von ihnen befallenen Pflanzen, Flächen oder Substrate eine wirksame Menge einer Verbindung der Formel I nach Anspruch 1 appliziert.

**20.** Verfahren zur Bekämpfung von Nematoden, dadurch gekennzeichnet, daß man auf diese oder die von ihnen befallenen Pflanzen, Flächen oder Substrate eine wirksame Menge einer Verbindung der Formel I nach Anspruch 1 appliziert.

Europäisches Patentamt

# EUROPÄISCHER RECHERCHENBERICHT

Nummer der Anmeldung

EP 92 11 6108

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,X | GB-A-2 140 010 (SUMITOMO)<br>* Seiten 13,18,19,28,29; Ansprüche *<br>--- | 1,9-20 | C 07 D 239/60<br>C 07 D 239/52<br>C 07 D 401/12<br>C 07 D 239/34<br>C 07 D 239/70<br>C 07 D 239/90<br>C 07 D 405/12<br>C 07 D 409/12<br>A 01 N 43/54 |
| D,X | EP-A-0 326 329 (LILLY)<br>* Seiten 10-16; Ansprüche *<br>--- | 1,9-20 | |
| X | FR-A-2 360 581 (ICI)<br>* Seiten 1-16; Ansprüche *<br>--- | 1,9-20 | |
| X | EP-A-0 331 529 (UBE)<br>* Seiten 2-4 (B2-B4); Seiten 6-12; Seite 13, Beispiel 4; Seiten 16-22; Ansprüche *<br>--- | 1,9-20 | |
| X | CHEMICAL ABSTRACTS, Band 106, 1987, Seiten 668-669, Zusammenfassung Nr. 213982r, Columbus, Ohio, US; & JP-A-62 51 672 (S. ITO et al.) 06-03-1987<br>* Zusammenfassung *<br>--- | 1 | |
| | | | **RECHERCHIERTE SACHGEBIETE (Int. Cl.5)** |
| X | EP-A-0 257 850 (NISSIN FOOD PRODUCTS)<br>* Seite 22; Ansprüche *<br>--- | 1 | C 07 D 239/00<br>C 07 D 401/00 |
| X | DE-A-2 806 661 (MERCK)<br>* Seiten 38,39 *<br>--- | 1 | |
| P,X | CHEMICAL ABSTRACTS, Band 117, 1992, Seite 297, Zusammenfassung Nr. 2834h, Columbus, Ohio, US; & JP-A-04 26 680 (UBE) 29-01-1992<br>* Zusammenfassung *<br>--- -/- | 1,12-20 | |

Der vorliegende Recherchenbericht wurde für alle Patentansprüche erstellt

| Recherchenort | Abschlußdatum der Recherche | Prüfer |
|---|---|---|
| DEN HAAG | 19-12-1992 | FRANCOIS J.C.L. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

Europäisches Patentamt

# EUROPÄISCHER RECHERCHENBERICHT

Nummer der Anmeldung

EP 92 11 6108

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | CHEMICAL ABSTRACTS, Band 105, 1986, Seite 724, Zusammenfassung Nr. 42654h, Columbus, Ohio, US; & JP-A-60 215 671 (SUMITOMO) 29-10-1985 * Zusammenfassung * --- | 1,12-20 | |
| X | JOURNAL OF THE CHEMICAL SOCIETY, PERKIN TRANS. I, 1987, Seiten 2585-2592, London, GB; T.R. JONES et al.: "Azafluorenes containing two bridgehead nitrogen atoms" * Seite 2588 * --- | 1 | |
| X | JOURNAL OF HETEROCYCLIC CHEMISTRY, Band 24, 1987, Seiten 1243-1247, New York, US; H. GERSHON et al.: "Pyrimidines. 9. Chlorination of 6-trifluoromethyluracil with phosphorus oxychloride in the presence of trialkylamines" * Seiten 1243,1245-1246 * --- | 1,9 | RECHERCHIERTE SACHGEBIETE (Int. Cl.5) |
| X | CHEMICAL AND PHARMACEUTICAL BULLETIN, Band 27, Nr. 6, 1979, Seiten 1468-1472, Tokyo, JP; H. ASAKAWA et al.: "Chemistry of salicylic acid and anthranilic acid. III. Hypoglycemic screening tests for salicylic and anthranilic acid derivatives" * Seiten 1469-1471 * ----- | 1 | |

Der vorliegende Recherchenbericht wurde für alle Patentansprüche erstellt

| Recherchenort | Abschlußdatum der Recherche | Prüfer |
|---|---|---|
| DEN HAAG | 19-12-1992 | FRANCOIS J.C.L. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)